# EUROPEAN PATENT APPLICATION

(11) **EP 4 471 063 A1**
(43) Date of publication of application: **04.12.2024**
(21) Application number: 23746145.4
(22) Date of filing: 17.01.2023
(51) Int. Cl.: C07K 16/28, C12N 15/13, C12N 15/63, C12N 5/00, A61P 35/00, A61K 39/395

(54) **ANTIBODY AGAINST HER3, CONJUGATE AND USE THEREOF**

(30) Priority: 25.01.2022 CN 202210089838
(71) Applicant: Medilink Therapeutics (Suzhou) Co., Ltd., Suzhou, Jiangsu 215000 (CN)
(72) Inventor: CAI, Jiaqiang, Suzhou, Jiangsu 215000 (CN)
(74) Representative: Maiwald GmbH
(86) International application number: PCT/CN2023/072657
(87) International publication number: WO 2023/143263

(57) **Abstract**

The present invention relates to an anti-Her3 antibody, a method for preparing same, a conjugate thereof and use thereof, and particularly to an antibody that binds to Her3 to exhibit an anti-tumor activity and a conjugate thereof. The present invention further includes a composition of the antibody and an ADC, and a method for using the same.

## Description

### Technical field

The present disclosure belongs to the field of therapeutic biopharmaceuticals, and more specifically, the present disclosure relates to an antibody against Her3, a conjugate, and further relates to the use of the antibody or conjugate for the treatment of diseases.

### Background technology

Her3 (ErbB3) is a transmembrane receptor tyrosine kinase molecule of the ERBB family, which includes four members: EGFR, Her2, Her3, and Her4. The activation of ERBB receptors requires the formation of homologous or heterologous dimers, and their family members can form dimers with each other. When the ligand is deficient, Her3 is locked in a fixed conformation by interacting with the cysteine-rich region; mutations of non-conserved amino acids in the intracellular protein kinase domain make Her3 to only retain weak kinase activity, the formation of heterodimers with other EGFR family molecules is then needed to mediate signal transduction. Her3 plays an important role in embryonic development, and the absence of Her3 in mice can result in severe hypoplasia of the atrioventricular valve, causing embryonic death of mice. This effect is mainly determined by its NRG1 ligand. In addition, Her3 plays an important role in the differentiation of neural crest (such as Schwann cells) and the development of sympathetic nervous system. Her3 is mutated or overexpressed in a variety of cancers, such as several types of adenocarcinomas (breast cancer, urethral cancer, gastric cancer, colorectal cancer, biliary cancer, bladder cancer, endometrial cancer, ovarian cancer, uterine cancer, prostate cancer, lung cancer), and melanoma, glioma, etc.

For antibody-drug conjugates (ADC), biologically active small molecule compounds are conjugated to monoclonal antibodies or antibody fragments by chemical methods, so as to fully utilize antibodies' binding specificity to normal cell and to tumor cell surface antigens, and small molecule's high anti-tumor biological activity, while avoiding defects such as the low specific efficacy of the former as well as toxic side effects of the latter. Compared with traditional chemotherapeutic or targeted drugs, antibody-drug conjugates can more accurately bind to tumor cells and reduce their effects on normal cells.

There are currently patent reports on Her3 antibodies and ADC drugs, such as U3-1402 developed by Daiichi Sankyo and MP-Her3-ADC developed by MediaPharma.

However, although antibody-drug conjugates (ADC) combine the advantages of the target specificity and targeting property of antibodies to target cells with the tumor killing ability of loaded drugs, but due to the structural complexity ofADC drug molecules, there is still a problem of insufficient safety windows in practical applications. The current Her3 ADCs are still in the early development stage, and therefore, there is still an urgent need for safer and more effective Her3 antibodies or antibody-drug conjugates to meet the unmet clinical needs.

### Summary of the invention

In the present disclosure, a Her3 molecule binding domain comprising a light chain variable region (VL) and/or a heavy chain variable region (VH) has been first developed, and by using above light chain variable region and/or heavy chain variable region, a human antibody has been further developed.

The antibodies involved in the present disclosure can be fully human antibodies, which can be safely administered to human subjects without triggering immunogenic reactions. It has a high ability to bind to human and non-human Her3 molecules, particularly involves molecules with cross reactivity with Her3 from non-human primates (such as monkeys) and mammals (such as rats). The present disclosure also relates to an antibody-drug conjugate (ADC) comprising Her3 antibody. The antibody or the antibody-drug conjugate (ADC) of the present disclosure has significant clinical values.

### Antibodies of the Present Disclosure

In a first aspect, the present disclosure provides an antibody or antigen-binding fragment thereof binding to Her3, said antibody or antigen-binding fragment thereof comprises the Complementarity Determining Regions (CDRs) as shown below:
HCDR1 or a variant of its sequence, HCDR2 or a variant of its sequence, and HCDR3 or a variant of its sequence as comprised in the heavy chain variable region (VH) set forth in SEQ ID NO: 23, 25, or 27; and/or
LCDR1 or a variant of its sequence, LCDR2 or a variant of its sequence, and LCDR3 or a variant of its sequence as comprised in the light chain variable region (VL) set forth in SEQ ID NO: 24, 26, or 28.

In some embodiments, the antibody or antigen-binding fragment thereof comprises HCDR1 or a variant of its sequence, HCDR2 or a variant of its sequence, and HCDR3 or a variant of its sequence comprised in the VH set forth in SEQ ID NO: 23; and/or LCDR1 or a variant of its sequence, LCDR2 or a variant of its sequence, and LCDR3 or a variant of its sequence comprised in the VL set forth in SEQ ID NO: 24.

In some embodiments, the antibody or antigen-binding fragment thereof comprises HCDR1 or a variant of its sequence, HCDR2 or a variant of its sequence, and HCDR3 or a variant of its sequence comprised in the VH set forth in SEQ ID NO: 25; and/or LCDR1 or a variant of its sequence, LCDR2 or a variant of its sequence, and LCDR3 or a variant of its sequence comprised in the VL set forth in SEQ ID NO: 26.

In some embodiments, the antibody or antigen-binding fragment thereof comprises HCDR1 or a variant of its sequence, HCDR2 or a variant of its sequence, and HCDR3 or a variant of its sequence comprised in the VH set forth in SEQ ID NO: 27; and/or LCDR1 or a variant of its sequence, LCDR2 or a variant of its sequence, and LCDR3 or a variant of its sequence comprised in the VL set forth in SEQ ID NO: 28.

In certain preferred embodiments, the variant of sequence is a CDR which has one or several amino acid substitutions, deletions or additions (e.g., 1, 2 or 3 amino acid substitutions, deletions or additions), compared with the CDR from which it is derived.

In certain preferred embodiments, the substitution(s) are conservative substitution(s).

Preferably, the CDRs are defined by the AbM, Chothia, Kabat, or IMGT numbering systems.

In one aspect, the present disclosure provides an antibody or antigen-binding fragment thereof which is able to bind Her3, said antibody or antigen-binding fragment thereof comprises a heavy chain variable region (VH) and/or a light chain variable region (VL).

In some embodiments, the antibody or antigen-binding fragment thereof of the present disclosure comprises the heavy chain variable region (VH) and/or the light chain variable region (VL) as shown below, wherein the CDRs are defined by the Kabat numbering system:
(a) A heavy chain variable region (VH) comprising the following 3 CDRs: HCDR1 consisting of the sequence of SEQ ID NO: 1 or a sequence having one or several amino acid substitutions, deletions or additions (e.g., 1, 2, or 3 amino acid substitutions, deletions or additions) compared with SEQ ID NO: 1; HCDR2 consisting of the sequence of SEQ ID NO: 2 or a sequence having one or several amino acid substitutions, deletions or additions (e.g. 1, 2, or 3 amino acid substitutions, deletions or additions) compared with SEQ ID NO: 2; HCDR3 consisting of the sequence of SEQ ID NO: 3 or a sequence having one or several amino acid substitutions, deletions or additions (e.g., 1, 2, or 3 amino acid substitutions, deletions or additions) compared with SEQ ID NO: 3; and/or,
   a light chain variable region (VL) comprising the following 3 CDRs: LCDR1 consisting of the sequence of SEQ ID NO: 7 or a sequence having one or several amino acid substitutions, deletions or additions (e.g. 1, 2, or 3 amino acid substitutions, deletions or additions) compared with SEQ ID NO: 7; LCDR2 consisting of the sequence of SEQ ID NO: 8 or a sequence having one or several amino acid substitutions, deletions or additions (e.g., 1, 2, or 3 amino acid substitutions, deletions or additions) compared with SEQ ID NO: 8; LCDR3 consisting of the sequence of SEQ ID NO: 9 or a sequence having one or several amino acid substitutions, deletions or additions (e.g., 1, 2, or 3 amino acid substitutions, deletions or additions) compared with SEQ ID NO: 9;
(b) A heavy chain variable region (VH) comprising the following 3 CDRs: HCDR1 consisting of the sequence of SEQ ID NO: 12 or a sequence having one or several amino acid substitutions, deletions or additions (e.g., 1, 2, or 3 amino acid substitutions, deletions or additions) compared with SEQ ID NO: 12; HCDR2 consisting of the sequence of SEQ ID NO: 13 or a sequence having one or several amino acid substitutions, deletions or additions (e.g., 1, 2, or 3 amino acid substitutions, deletions or additions) compared with SEQ ID NO: 13; HCDR3 consisting of the sequence of SEQ ID NO: 14 or a sequence having one or several amino acid substitutions, deletions or additions (e.g., 1, 2, or 3 amino acid substitutions, deletions or additions) compared with SEQ ID NO: 14; and/or,
   a light chain variable region (VL) comprising the following 3 CDRs: LCDR1 consisting of the sequence of SEQ ID NO: 18 or a sequence having one or several amino acid substitutions, deletions or additions (e.g., 1, 2, or 3 amino acid substitutions, deletions or additions) compared with SEQ ID NO: 18; LCDR2 consisting of the sequence of SEQ ID NO: 19 or a sequence having one or several amino acid substitutions, deletions or additions (e.g., 1, 2, or 3 amino acid substitutions, deletions or additions) compared with SEQ ID NO: 19; LCDR3 consisting of the sequence of SEQ ID NO: 20 or a sequence having one or several amino acid substitutions, deletions or additions (e.g., 1, 2, or 3 amino acid substitutions, deletions or additions) compared with SEQ ID NO: 20.

In some embodiments, the antibody or antigen-binding fragment thereof of the present disclosure comprises the heavy chain variable region (VH) and/or the light chain variable region (VL) as shown below, wherein the CDRs are defined by the Kabat numbering system:
(a) A heavy chain variable region (VH) comprising the following 3 CDRs: HCDR1 consisting of the sequence of SEQ ID NO: 1, HCDR2 consisting of the sequence of SEQ ID NO:2, HCDR3 consisting of the sequence of SEQ ID NO:3; and/or,
   a light chain variable region (VL) comprising the following 3 CDRs: LCDR1 consisting of the sequence of SEQ ID NO:7, LCDR2 consisting of the sequence of SEQ ID NO:8, and LCDR3 consisting of the sequence of SEQ ID NO:9; or
(b) A heavy chain variable region (VH) comprising the following 3 CDRs: HCDR1 consisting of the sequence of SEQ ID NO: 12, HCDR2 consisting of the sequence of SEQ ID NO: 13, HCDR3 consisting of the sequence of SEQ ID NO: 14; and/or,
   a light chain variable region (VL) comprising the following 3 CDRs: LCDR1 consisting of the sequence of SEQ ID NO: 18, LCDR2 consisting of the sequence of SEQ ID NO: 19, and LCDR3 consisting of the sequence of SEQ ID NO:20.

In some embodiments, the antibody or antigen-binding fragment thereof of the present disclosure comprises the heavy chain variable region (VH) and/or the light chain variable region (VL) as shown below, wherein the CDRs are defined by the IMGT numbering system:
(a) A heavy chain variable region (VH) comprising the following 3 CDRs: HCDR1 consisting of the sequence of SEQ ID NO: 4 or a sequence having one or several amino acid substitutions, deletions or additions (e.g., 1, 2, or 3 amino acid substitutions, deletions or additions) compared with SEQ ID NO: 4; HCDR2 consisting of the sequence of SEQ ID NO: 5 or a sequence having one or several amino acid substitutions, deletions or additions (e.g., 1, 2, or 3 amino acid substitutions, deletions or additions) compared with SEQ ID NO: 5; HCDR3 consisting of the sequence of SEQ ID NO: 6 or a sequence having one or several amino acid substitutions, deletions or additions (e.g., 1, 2, or 3 amino acid substitutions, deletions or additions) compared with SEQ ID NO: 6; and/or,
   a light chain variable region (VL) comprising the following 3 CDRs: LCDR1 consisting of the sequence of SEQ ID NO: 10 or a sequence having one or several amino acid substitutions, deletions or additions (e.g., 1, 2, or 3 amino acid substitutions, deletions or additions) compared with SEQ ID NO: 10; LCDR2 consisting of a sequence of AAS or a sequence having one or several amino acid substitutions, deletions or additions (e.g., 1, 2, or 3 amino acid substitutions, deletions or additions) compared with AAS; LCDR3 consisting of the sequence of SEQ ID NO: 9 or a sequence having one or several amino acid substitutions, deletions or additions (e.g., 1, 2, or 3 amino acid substitutions, deletions or additions) compared with SEQ ID NO: 9; or
(b) A heavy chain variable region (VH) comprising the following 3 CDRs: HCDR1 consisting of the sequence of SEQ ID NO: 15 or a sequence having one or several amino acid substitutions, deletions or additions (e.g., 1, 2, or 3 amino acid substitutions, deletions or additions) compared with SEQ ID NO: 15; HCDR2 consisting of the sequence of SEQ ID NO: 16 or a sequence having one or several amino acid substitutions, deletions or additions (e.g., 1, 2, or 3 amino acid substitutions, deletions or additions) compared with SEQ ID NO: 16; HCDR3 consisting of the sequence of SEQ ID NO: 17 or a sequence having one or several amino acid substitutions, deletions or additions (e.g., 1, 2, or 3 amino acid substitutions, deletions or additions) compared with SEQ ID NO: 17; and/or,
   a light chain variable region (VL) comprising the following 3 CDRs: LCDR1 consisting of the sequence of SEQ ID NO: 21 or a sequence having one or several amino acid substitutions, deletions or additions (e.g., 1, 2, or 3 amino acid substitutions, deletions or additions) compared with SEQ ID NO: 21; LCDR2 consisting of a sequence of AAS or a sequence having one or several amino acid substitutions, deletions or additions (e.g., 1, 2, or 3 amino acid substitutions, deletions or additions) compared with AAS; LCDR3 consisting of the sequence of SEQ ID NO: 20 or a sequence having one or several amino acid substitutions, deletions or additions (e.g., 1, 2, or 3 amino acid substitutions, deletions or additions) compared with SEQ ID NO: 20.

In some embodiments, the antibody or antigen-binding fragment thereof of the present disclosure comprises the heavy chain variable region (VH) and/or the light chain variable region (VL) as shown below, wherein the CDRs are defined by the IMGT numbering system:
(a) A heavy chain variable region (VH) comprising the following 3 CDRs: HCDR1 consisting of the sequence of SEQ ID NO:4, HCDR2 consisting of the sequence of SEQ ID NO:5, HCDR3 consisting of the sequence of SEQ ID NO:6; and/or,
   a light chain variable region (VL) comprising the following 3 CDRs: LCDR1 consisting of the sequence of SEQ ID NO: 10, LCDR2 consisting of the sequence of AAS, and LCDR3 consisting of the sequence of SEQ ID NO:9;
(b) A heavy chain variable region (VH) comprising the following 3 CDRs: HCDR1 consisting of the sequence of SEQ ID NO: 15, HCDR2 consisting of the sequence of SEQ ID NO: 16, HCDR3 consisting of the sequence of SEQ ID NO: 17; and/or,
   a light chain variable region (VL) comprising the following 3 CDRs: LCDR1 consisting of the sequence of SEQ ID NO:21, LCDR2 consisting of the sequence of AAS, and LCDR3 consisting of the sequence of SEQ ID NO:20.

In certain embodiments, the antibody or antigen binding fragment thereof of the present disclosure comprises a heavy chain variable region (VH) and/or a light chain variable region (VL) as defined below, wherein at least one CDR of the heavy chain variable region (VH) and/or light chain variable region (VL) contains a mutation, compared with the CDRs defined by the aforementioned Kabat or IMGT numbering system, wherein said mutation(s) are one or several amino acid substitutions, deletions or additions or any combination thereof (1, 2 or 3 amino acid substitutions, deletions or additions or any combination thereof), and still has Her3 binding activity.

Preferably, the substitution mentioned in the present disclosure is a conservative substitution.

In certain embodiments, the antibody or antigen binding fragment thereof binds human Her3, monkey Her3, and/or rat Her3.

In certain embodiments, the VH of the antibody or antigen-binding fragment thereof of the present disclosure comprises a framework region (FR) derived from the heavy chain variable region (VH) of human immunoglobulin, and/or the VL of the antibody or antigen-binding fragment thereof comprises a framework region (FR) derived from the light chain variable region (VL) of human immunoglobulin. Thus, in certain embodiments, the antibodies or antigen-binding fragments thereof of the present disclosure are fully humanized. In certain embodiments, the antibodies or an antigen-binding fragment thereof of the present disclosure are humanized.

In certain embodiments, the antibody or antigen-binding fragment thereof of the present disclosure comprises:
(a) the heavy chain framework region of human immunoglobulins or variants thereof, the variants have up to conservative substitutions of 20 amino acids (e.g., conservative substitutions of up to 20, up to 15, up to 10 or up to 5 amino acids; e.g., conservative substitutions of 1, 2, 3, 4, 5, 6, 7, 8, 9 or 10 amino acids), compared with the amino acid sequence encoded by the germline antibody gene from which it is derived; and/or
(b) the light chain framework region of human immunoglobulins or variants thereof, the variants have up to conservative substitutions of 20 amino acid (e.g., conservative substitutions of up to 20, up to 15, up to 10 or up to 5 amino acids; e.g., conservative substitutions of 1, 2, 3, 4, 5, 6, 7, 8, 9 or 10 amino acid), compared with the amino acid sequence encoded by the germline antibody gene from which it is derived.

In certain embodiments, the humanized degree of the antibody or antigen-binding fragment thereof of the present disclosure is at least 75%, at least 80%, at least 85%, at least 90%, at least 91%, at least 92%, at least 93%, at least 94%, at least 95%, at least 96%, at least 97%, at least 98% or at least 99%.

In certain embodiments, the antibody or antigen-binding fragment thereof of the present disclosure comprises:
(a) the heavy chain variable region (VH), which comprises an amino acid sequence selected from the following:
   (i) a sequence set forth in SEQ ID NO:23, 25, or 27;
   (ii) a sequence having one or several substitutions, deletions or additions or any combination thereof (e.g., 1, 2, 3, 4, 5, 6, 7, 8, 9 or 10 amino acid substitutions, deletions or additions or any combination thereof) compared with the sequence set forth in SEQ ID NO: 23, 25, or 27; or
   (iii) a sequence having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity compared with the sequence set forth in SEQ ID NO: 23, 25, or 27;
      and/or
(b) the light chain variable region (VL), which comprises an amino acid sequence selected from the following:
   (iv) a sequence set forth in SEQ ID NO:24, 26 or 28;
   (v) a sequence having one or several substitutions, deletions or additions or any combination thereof (e.g., 1, 2, 3, 4, 5, 6, 7, 8, 9 or 10 amino acid substitutions, deletions or additions or any combination thereof) compared with the sequence set forth in SEQ ID NO: 24, 26 or 28; or
   (vi) a sequence having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity compared with the sequence set forth in SEQ ID NO: 24, 26 or 28.

In certain embodiments, the antibody or antigen-binding fragment thereof of the present disclosure comprises the VH set forth in SEQ ID NO: 23 and/or the VL set forth in SEQ ID NO: 24.

In certain embodiments, the antibody or antigen-binding fragment thereof of the present disclosure comprises the VH set forth in SEQ ID NO: 25 and/or the VL set forth in SEQ ID NO: 26.

In certain embodiments, the antibody or antigen-binding fragment thereof of the present disclosure comprises the VH set forth in SEQ ID NO: 27 and/or the VL set forth in SEQ ID NO: 28.

In certain embodiments, the antibody or antigen-binding fragment thereof of the present disclosure comprises:
(a) the VH consisting of the sequence set forth in SEQ ID NO: 23 and the VL consisting of the sequence set forth in SEQ ID NO: 24;
(b) the VH consisting of the sequence set forth in SEQ ID NO: 25 and the VL consisting of the sequence set forth in SEQ ID NO: 26;
(c) the VH consisting of the sequence set forth in SEQ ID NO: 27 and the VL consisting of the sequence set forth in SEQ ID NO: 28;
(d) a heavy chain variable region (VH) and a light chain variable region (VL), wherein the heavy chain variable region (VH) and the light chain variable region (VL) have at least 70%, at least 80%, at least 85%, at least 90%, at least 91%, at least 92%, at least 93%, at least 94%, at least 95%, at least 96%, at least 97%, at least 98% or at least 99% sequence identity respectively, independently as compared with the VH and VL in any one of (a) to (c); or
(e) a heavy chain variable region (VH) and a light chain variable region (VL), wherein the heavy chain variable region (VH) and the light chain variable region (VL) independently have one or several amino acid substitutions, deletions or additions or any combination thereof (e.g., 1, 2, 3, 4, 5, 6, 7, 8, 9 or 10 amino acid substitutions, deletions or additions or any combination thereof), as compared with the VH and VL in any one of (a) to (c), respectively. Preferably, the substitution(s) are conservative substitution(s).

In certain embodiments, the present disclosure also provides an anti-Her3 antibody or antigen-binding fragment thereof that competes with the antibody or antigen-binding fragment thereof defined above such as antibody 202-2-1 or antigen-binding fragment thereof for binding, which is characterized in that the antibody or antigen-binding fragment thereof is different from the antibody or antigen-binding fragment thereof that competes with it. For example, it is not antibody 202-2-1 or antigen-binding fragment thereof.

In some embodiments, the anti-Her3 antibody or antigen-binding fragment thereof competes with antibody 202-2-1 for binding, characterized in that it is not antibody 202-2-1 or antigen-binding fragment thereof, and has different CDR(s) from antibody 202-2-1 or antigen-binding fragment thereof.

In certain embodiments, the antibody of the present disclosure binds to domain 3 of human Her3, such as one or more amino acids in domain 3. Therefore, in certain embodiments, the present disclosure provides an antibody or antigen-binding fragment thereof that binds to HER3, wherein the antibody or antigen-binding fragment thereof binds to domain 3 of human Her3, preferably to amino acids at positions 328-499 of human Her3 set forth in SEQ ID No.31.

In some embodiments, the present disclosure provides an antibody or antigen-binding fragment thereof that binds HER3, wherein the antibody or antigen-binding fragment thereof binds to the following spatial epitopes: Histidine at position 466, Tryptophan at position 470, Threonine at position 471, Threonine at position 478, (Aspartic acid-Isoleucine-lysine-Histidine-Asparagine) at positions 483 to 487, Isoleucine at position 484, Arginine at position 490 and Arginine at position 491 of human Her3 set forth in SEQ ID No. 31.

The anti-Her3 antibody or antigen-binding fragment thereof that competes with antibody 202-2-1 for binding binds to the following spatial epitopes: Histidine at position 466, Tryptophan at position 470, Threonine at position 471, Threonine at position 478, (Aspartic acid-Isoleucine-lysine-Histidine-Asparagine) at positions 483 to 487, Isoleucine at position 484, Arginine at position 490 and Arginine at position 491 of Her3 set forth in SEQ ID No. 31.

In certain embodiments, the antibody or antigen-binding fragment thereof disclosed herein blocks Her3 ligand-dependent and/or -independent signaling.

In certain embodiments, the antibody or antigen-binding fragment thereof disclosed herein inhibits Her3 ligand-dependent and/or -independent AKT phosphorylation. In certain embodiments, the antibody or antigen-binding fragment thereof disclosed herein inhibits the formation of Her3/Her2 heterodimers that are Her3 ligand-dependent and/or -independent.

In certain embodiments, the antibody of the present disclosure is a chimeric antibody, a humanized antibody, or a fully human antibody. In certain embodiments, the antibody or antigen-binding fragment thereof of the present disclosure is selected from Fab, Fab', (Fab')₂, Fv fragments such as scFv or disulfide-linked Fv (dsFv), double antibodies (diabody), and multi-specific antibodies (such as bispecific antibodies). In certain embodiments, the antibody of the present disclosure is scFv.

In certain embodiments, the antibody or an antigen-binding fragment thereof of the present disclosure comprises a heavy chain constant region (CH) of a human immunoglobulin or a variant thereof, and the variant has up to conservative substitutions of 50 amino acids (e.g., conservative substitutions of up to 45, up to 40, up to 35, up to 30, up to 25, up to 20, up to 15, up to 10 or up to 5 amino acids; e.g., conservative substitutions of 1, 2, 3, 4, 5, 6, 7, 8, 9 or 10 amino acids), compared with the wild type sequence from which it is derived. In certain embodiments, the antibody or an antigen-binding fragment thereof of the present disclosure comprises a light chain constant region (CL) of a human immunoglobulin or a variant thereof, and the variant has up to conservative substitutions of 50 amino acids (e.g., conservative substitutions of up to 45, up to 40, up to 35, up to 30, up to 25, up to 20, up to 15, up to 10 or up to 5 amino acids; e.g., conservative substitutions of 1, 2, 3, 4, 5, 6, 7, 8, 9 or 10 amino acids), compared with the wild type sequence from which it is derived.

In some embodiments, the constant region is altered, e.g., mutated, to modify the properties of the anti-Her3 antibody molecule (e.g., to alter one or more of the following properties: Fc receptor binding, antibody glycosylation, number of cysteine residues, effector cell function, or complement function). Functional changes can be carried out by substituting at least one amino acid residue in the constant region of the antibody with a different residue, for example, changing the affinity of the antibody for effector ligands (e.g., FcR or complement C1q), thereby changing effector function (e.g., decreasing).

The Fc region of the antibody mediates several important effector functions, such as ADCC, phagocytosis (ADCP), CDC, etc.

In certain embodiments, the antibody or antigen-binding fragment thereof of the present disclosure has a heavy chain constant region (CH), which is selected from the heavy chain constant regions of e.g., IgG1, IgG2, IgG3, IgG4, IgM, IgA1, IgA2, IgD, and IgE; particularly from the heavy chain constant regions of e.g., IgG1, IgG2, IgG3, and IgG4; and more particularly from the heavy chain constant region of IgG1 (e.g., human IgG1). In some embodiments, the heavy chain constant region of human IgG1 has a sequence set forth in SEQ ID NO:29. In some embodiments, the antibody or antigen-binding fragment thereof of the present disclosure has a light chain constant region, which is selected from, for example, κ or λ light chain constant region, preferably κ light chain constant region (e.g., human κ light chain constant region). In some embodiments, the light chain constant region has a sequence set forth in SEQ ID NO:30.

In some embodiments, the antibody or antigen-binding fragment thereof has a CH set forth in SEQ ID NO: 29 or a variant thereof, said variant has conservative substitutions of up to 20 amino acids (such as conservative substitutions of up to 20, up to 15, up to 10 or up to 5 amino acids, such as conservative substitutions of 1, 2, 3, 4, 5, 6, 7, 8, 9 or 10 amino acids), compared with SEQ ID NO: 29; or at least 70%, at least 80%, at least 85%, at least 90%, at least 91%, at least 92%, at least 93%, at least 94%, at least 95%, at least 96%, at least 97%, at least 98%, or at least 99% sequence identity compared with SEQ ID NO: 29.

In some embodiments, the antibody or antigen-binding fragment thereof has a light chain constant region or a variant thereof. In some embodiments, the light chain constant region comprises κ light chain constant region. In some embodiments, the light chain constant region comprises a light chain constant region (CL) set forth in SEQ ID NO:30 or a variant thereof, said variant has conservative substitutions of up to 20 amino acids (such as conservative substitutions of up to 20, up to 15, up to 10 or up to 5 amino acids, such as conservative substitutions of 1, 2, 3, 4, 5, 6, 7, 8, 9 or 10 amino acids), compared with SEQ ID NO: 30; or at least 70%, at least 80%, at least 85%, at least 90%, at least 91%, at least 92%, at least 93%, at least 94%, at least 95%, at least 96%, at least 97%, at least 98%, or at least 99% sequence identity compared with SEQ ID NO: 30.

In some embodiments, the antibody or antigen-binding fragment thereof comprises the heavy chain constant region (CH) set forth in SEQ ID NO: 29 and the light chain constant region (CL) set forth in SEQ ID NO: 30.

In some embodiments, the antibody or antigen-binding fragment thereof of the present disclosure comprises:
(a) a heavy chain, which comprises an amino acid sequence selected from the following:
   (i) a sequence comprising the VH sequence set forth in SEQ ID NO: 23 and the CH sequence set forth in SEQ ID NO: 29;
   (ii) a sequence having one or several amino acid substitutions, deletions or additions or any combination thereof (e.g., up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (i); and
(b) a light chain, which comprises an amino acid sequence selected from the following:
   (iv) a sequence comprising the VL sequence set forth in SEQ ID NO: 24 and the CL sequence set forth in SEQ ID NO: 30;
   (v) a sequence having one or several amino acid substitutions, deletions or additions or any combination thereof (e.g., up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (iv).

In certain embodiments, the substitution(s) described in (ii) or (v) are conservative substitution(s).

In certain embodiments, the antibody or antigen-binding fragment thereof of the present disclosure comprises:
(a) a heavy chain, which comprises an amino acid sequence selected from the following:
   (i) a sequence comprising the VH sequence set forth in SEQ ID NO: 25 and the CH sequence set forth in SEQ ID NO: 29;
   (ii) a sequence having one or several amino acid substitutions, deletions or additions or any combination thereof (e.g., up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (i); and
(b) a light chain, which comprises an amino acid sequence selected from the following:
   (iv) a sequence comprising the VL sequence set forth in SEQ ID NO: 26 and the CL sequence set forth in SEQ ID NO: 30;
   (v) a sequence having one or several amino acid substitutions, deletions or additions or any combination thereof (e.g., up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (iv).

In certain embodiments, the substitution(s) described in (ii) or (v) are conservative substitution(s).

In certain embodiments, the antibody or antigen-binding fragment thereof of the present disclosure comprises:
(a) a heavy chain, which comprises an amino acid sequence selected from the following:
   (i) a sequence comprising the VH sequence set forth in SEQ ID NO: 27 and the CH sequence set forth in SEQ ID NO: 29;
   (ii) a sequence having one or several amino acid substitutions, deletions or additions or any combination thereof (e.g., up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (i); and
(b) a light chain, which comprises an amino acid sequence selected from the following:
   (iv) a sequence comprising the VL sequence set forth in SEQ ID NO: 28 and the CL sequence set forth in SEQ ID NO: 30;
   (v) a sequence having one or several amino acid substitutions, deletions or additions or any combination thereof (e.g., up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (iv).

In certain embodiments, the substitution(s) described in (ii) or (v) are conservative substitution(s).

In certain embodiments, the antibody or antigen-binding fragment thereof of the present disclosure comprises a heavy chain and a light chain,
the heavy chain comprises:
   (i) a sequence set forth in SEQ ID NO: 11;
   (ii) a sequence having one or several amino acid substitutions, deletions or additions or any combination thereof (e.g., up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (i); or
   (iii) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (i); and
the light chain comprises:
   (iv) a sequence set forth in SEQ ID NO: 22;
   (v) a sequence having one or several amino acid substitutions, deletions or additions or any combination thereof (e.g., up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof), compared with the sequence shown in (iv); or
   (vi) a sequence having at least 80%, at least 85%, at least 90%, at least 91%, at least 92%, at least 93%, at least 94%, at least 95%, at least 96%, at least 97%, at least 98% or at least 99% sequence identity compared with the sequence shown in (iv); preferably, the substitution(s) described in (ii) or (v) are conservative substitution(s).

In certain embodiments, the antibody or antigen-binding fragment thereof, wherein the antibody is selected from the following groups comprising:
(a) a heavy chain, which comprises the VH sequence set forth in SEQ ID NO: 23 and the CH sequence set forth in SEQ ID NO: 29, and a light chain, which comprises the VL sequence set forth in SEQ ID NO: 24 and the CL sequence set forth in SEQ ID NO: 30; preferably, the heavy chain set forth in SEQ ID NO: 11 and the light chain set forth in SEQ ID NO: 22;
(b) a heavy chain, which comprises the VH sequence set forth in SEQ ID NO: 25 and the CH sequence set forth in SEQ ID NO: 29, and a light chain, which comprises the VL sequence set forth in SEQ ID NO: 26 and the CL sequence set forth in SEQ ID NO: 30;
(c) a heavy chain, which comprises the VH sequence set forth in SEQ ID NO: 27 and the CH sequence set forth in SEQ ID NO: 29, and a light chain, which comprises the VL sequence set forth in SEQ ID NO: 28 and the CL sequence set forth in SEQ ID NO: 30.

In a second aspect, the antibody of the present disclosure is a multi-specific antibody that binds to Her3 and one or more other antigens. Therefore, this disclosure provides a multi-specific antibody.

Preferably, the multi-specific antibody is a bispecific antibody or a tri-specific antibody or a tetra-specific antibody.

In some embodiments, the multi-specific antibody of the present disclosure comprises an antigen-binding fragment of the present disclosure that binds to Her3, and other antibodies or antigen-binding fragments or antibody mimics thereof.

### Antibody Derivatives of the Present Disclosure

An antibody or antigen-binding fragment thereof of the present disclosure may be derivatized, for example, linked to another molecule (e.g., another polypeptide or protein). In general, derivatization (e.g., labeling) of an antibody or antigen-binding fragment thereof does not adversely affect its binding to Her3 (particularly human Her3). Thus, the antibody or antigen-binding fragment thereof of the present disclosure is also intended to include such derivatized forms. For example, an antibody or antigen-binding fragment thereof of the present disclosure may be linked (by chemical coupling, gene fusion, noncovalent linking, or otherwise) to one or more other molecular groups, such as another antibody (e.g., to form a bispecific antibody), a detection agent, a pharmaceutical agent and/or a protein or polypeptide capable of mediating the binding of an antibody or antigen-binding fragment thereof to another molecule (e.g., avidin or polyhistidine tag).

One type of derivatized antibody (e.g., bispecific antibody) is produced by cross-linking 2 or more antibodies (belonging to the same or different types). Methods for obtaining bispecific antibodies are well known in the art, examples of which include, but are not limited to, chemical crosslinking method, cell engineering method (hybridoma method), or genetic engineering method.

Another type of derivatized antibody is the labeled antibody. For example, an antibody or antigen-binding fragment thereof of the present disclosure may be attached to a detectable marker. The detectable marker described in the present disclosure may be any substance detectable by fluorescence, spectroscopic, photochemical, biochemical, immunological, electrical, optical, or chemical means. Such markers are well known in the art and examples include, but are not limited to, enzymes (e.g., horseradish peroxidase, alkaline phosphatase, β-galactosidase, urease, glucose oxidase, etc.), radionuclides (e.g., 3H, 125I, 35S, 14C, or 32P), fluorescent dyes (e.g., fluorescein isothiocyanate (FITC), fluorescein, tetramethylrhodamine isothiocyanate (TRITC), phycoerythrin (PE), Texas red, rhodamine, quantum dots or cyanine dye derivatives (e.g., Cy7, Alexa 750)), acridines esters, magnetic beads (e.g., Dynabeads^{®}), calorimetric markers such as colloidal gold or colored glass or plastic (e.g., polystyrene, polypropylene, latex, etc.) beads, and biotin for binding avidin (e.g., streptavidin) modified by the above marker(s). Patents that teach the use of the marker include, but are not limited to, U.S. patent No.3,817,837; 3,850,752; 3,939,350; 3,996,345; 4,277,437; 4,275,149; and 4,366,241 (which are all hereby incorporated by reference in their entirety). Detectable markers as described above can be detected by methods known in the art. For example, radioactive markers can be detected using photographic film or a scintillation counter, while fluorescent markers can be detected using a photodetector to detect emitted light. Enzyme markers are generally detected by supplying the substrate to the enzyme and detecting the reaction products produced by the enzyme's action on the substrate, while calorimetric markers are detected by simple visualization of colored markers. In certain embodiments, such labeling can be suitable for immunological detection (e.g., enzyme-linked immunoassay, radioimmunoassay, fluorescence immunoassay, chemiluminescent immunoassay, etc.). In certain embodiments, the detectable marker as described above can be attached to the antibody or antigen-binding fragment thereof of the present disclosure by linkers having different lengths, to reduce potential steric hindrance.

In addition, the antibody or antigen-binding fragment thereof of the present disclosure can also be derivatized with chemical groups, such as polyethylene glycol (PEG), methyl or ethyl, or glycosyl. These groups can be used to improve the biological properties of antibodies, such as increasing serum half-life.

### Preparation of the Antibody

The antibody of the present disclosure can be prepared by various methods known in the art, such as obtaining them by genetic engineering recombination technology. For example, DNA molecules encoding the heavy and light chain genes of the antibody according to the present disclosure are obtained by chemical synthesis or PCR amplification. The resulting DNA molecules are inserted into the expression vectors and then transfected into the host cells. Then, the transfected host cells are cultured under specific conditions to express the antibodies of the present disclosure.

The antigen-binding fragment of the present disclosure can be obtained by hydrolyzing intact antibody molecules (c.f., Morimoto et al., J. Biochem. Biophys. Methods 24: 107-117 (1992) and Brennan et al., Science 229: 81 (1985)). In addition, these antigen-binding fragments can also be produced directly by recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells; Fab' fragments can be chemically coupled to form F(ab')₂ fragments (Carter et al., Bio/Technology, 10: 163-167 (1992)). In addition, Fv, Fab, or F(ab')₂ fragments can also be directly isolated from recombinant host cell cultures. Other techniques for preparing these antigen-binding fragments are fully known to those of ordinary skill in the art.

Thus, in a third aspect, the present disclosure provides an isolated nucleic acid molecule which comprises a nucleotide sequence encoding an antibody or antigen-binding fragment thereof, or a heavy and/or light chain variable region thereof, or one or more CDRs thereof according to the present disclosure; or a nucleotide sequence encoding the multi-specific antibodies of the present disclosure. In certain embodiments, the nucleotide sequence may be substituted according to codon degeneracy. In certain embodiments, the nucleotide sequence is codon-optimized.

In certain embodiments, the isolated nucleic acid molecule of the present disclosure comprises: (i) a first nucleic acid and a second nucleic acid encoding a heavy chain variable region and a light chain variable region, respectively, of an antibody or antigen binding fragment thereof of the present disclosure, or (ii) a first nucleic acid and a second nucleic acid encoding a heavy chain variable region and a heavy chain constant region, as well as a light chain variable region and a light chain constant region respectively, of an antibody or antigen binding fragment thereof of the present disclosure, or (iii) a first nuclei acid and a second nucleic acid encoding the heavy and light chains, respectively, of the antibody or antigen-binding fragment thereof of the present disclosure. In certain embodiments, the first nucleic acid and the second nucleic acid comprises a nucleic acid that is the degenerate sequence of or substantially identical to the sequence of the first and second nucleic acids in any of items (i)-(iii) above. In certain embodiments, the degenerate sequence or substantially identical sequence refers to a sequence having at least about 85%, 90%, 95%, or greater sequence identity or having one or more nucleotide substitutions, or having a difference in no more than 3, 6, 15, 30 or 45 nucleotides, compared with the nucleic acid molecules set forth in items (i)-(iii).

In a fourth aspect, a vector (e.g., a cloning vector or an expression vector) comprising an isolated nucleic acid molecule of the present disclosure is provided. In certain embodiments, the vectors of the present disclosure are cloning vectors or expression vectors. In certain embodiments, the vectors of the present disclosure are, for example, plasmids, Cosmids, bacteriophages, lentiviruses, etc. In certain embodiments, the vector is capable of expressing an antibody or antigen-binding fragment thereof of the present disclosure *in vivo* in a subject (e.g., a mammal, such as a human).

In a fifth aspect, there is provided a host cell comprising an isolated nucleic acid molecule of the present disclosure or a vector of the present disclosure. The host cell may be a eukaryotic cell (e.g., mammalian cell, insect cell, yeast cell) or a prokaryotic cell (e.g., *Escherichia coli*). Suitable eukaryotic cells include, but are not limited to, NS0 cells, Vero cells, Hela cells, COS cells, CHO cells, HEK293 cells, BHK cells, and MDCKII cells. Suitable insect cells include, but are not limited to, Sf9 cells. In certain embodiments, the host cell of the present disclosure is a mammalian cell, such as CHO (e.g., CHO-K1, CHO-S, CHO DXB 11, CHO DG44).

In a sixth aspect, there is provided a method of preparing an antibody or antigen-binding fragment thereof according to the present disclosure, or a multi-specific antibody according to the present disclosure, comprising culturing host cells of the present disclosure under conditions that allow expression of the antibody or antigen-binding fragment thereof, or the multi-specific antibody, and recovering the antibody or antigen-binding fragment thereof from the cultured host cell culture.

### Antibody-drug Conjugate

In a seventh aspect, an antibody-drug conjugate, a stereoisomer thereof, a prodrug thereof, a pharmaceutically acceptable salt thereof or a pharmaceutically acceptable solvate thereof is provided, wherein the antibody is the above-mentioned antibody or antigen-binding fragment thereof that binds to Her3 and is linked to a coupling moiety via a connector.

The coupling moiety is selected from: a detectable marker, a radioisotope, a fluorescent agent, a luminescent agent, a colored agent, an enzyme, polyethylene glycol (PEG), a nuclide, a nucleic acid, a small molecule toxin, a polypeptide having binding activity, a protein, a receptor, a ligand, and other active agent that inhibits tumor cell growth, promotes tumor cell apoptosis or necrosis.

In certain examples, an antibody-drug conjugate comprises the anti-Her3 antibody drug conjugate (Her3-ADC) of the following formula:

Tb-(L-D) q,

wherein:
Tb is the above-mentioned antibody or antigen-binding fragment thereof that binds to Her3 and;
D is a small molecule toxin drug moiety;
L is a bond or linker which covalently links Tb and D;
q is an integer between 1 and 16, and represents the number of L-D covalently linked to Tb;

In some embodiments, the antibody-drug conjugate has the structure represented by formula I: 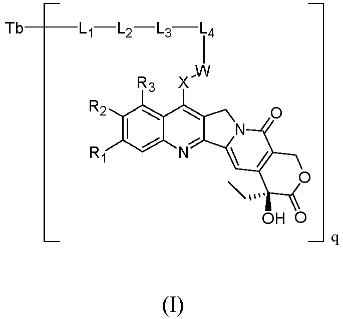 wherein,
L₁ is selected from 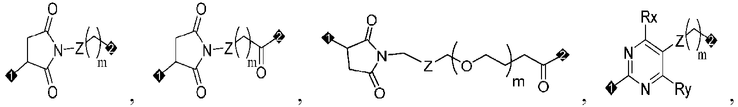 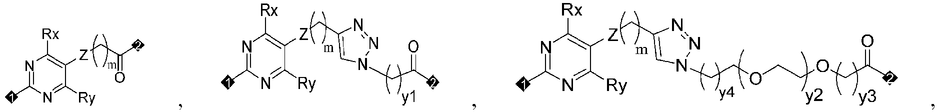 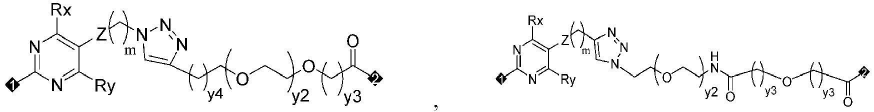 and 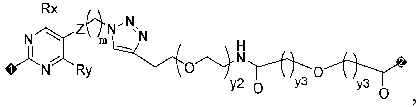 each Z is independently selected from a direct bond, a carbon-carbon triple bond, a carbon-carbon double bond, and amido (preferably selected from a direct bond, a carbon-carbon triple bond, and a carbon-carbon double bond); Rx and Ry are each independently selected from H and C1-4 allcyl; each m is independently selected from 0, 1, 2, 3, 4, 5 and 6; y1 is selected from any integer between 1 and 6 (such as 4, 5, and 6); each y2 is independently selected from any integer between 0 and 15 (such as 6-15); each y3 is independently selected from 1, 2, and 3; each y4 is independently selected from 0 and 1; position 1 is attached to Tb via an S atom, and position 2 is attached to L₂ or L₃;
L₂ is absent or present, and when L₂ is present, L₂ is selected from: 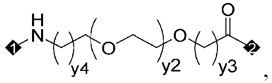 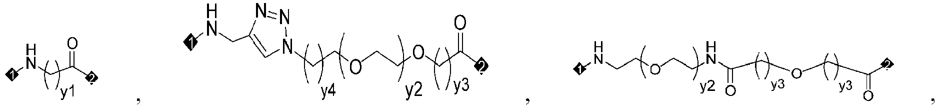 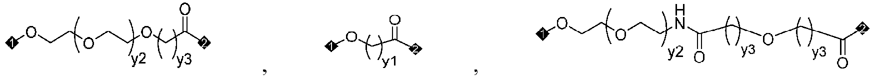 and 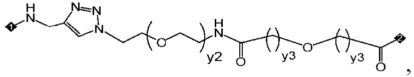 y1 is selected from any integer between 1 and 6 (such as 4, 5, and 6); each y2 is independently selected from any integer between 0 and 10 (such as 6-10); each y3 is independently selected from 1 or 2; each y4 is independently selected from 0 and 1, position 1 is attached to L₁, and position 2 is attached to L₃;
L₃ is selected from an amino acid residue or a short peptide consisting of 2-10 amino acid residues; the amino acid residue is selected from natural amino acid residues, non-natural amino acid residues, or selected from amino acid residue represented by AA¹ or stereoisomer thereof; 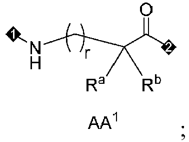 in the amino acid residue represented by AA¹, any one of R^{a} and R^{b} is H, and the other is H, 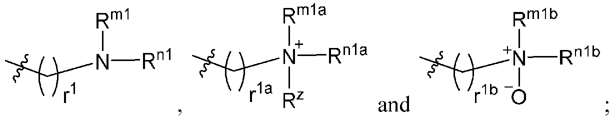 or, R^{a} and R^{b}, together with
the carbon atom to which they are both attached, form a 5-6 membered heterocyclic ring, and said 5-6 membered heterocyclic ring is piperidine ring or piperazine ring.
r, r¹, r^{1a} and r^{1b} are each independently 0, 1, 2, 3, 4 or 5;
R^{m1}, Rⁿ¹, R^{m1a}, R^{n1a}, R^{m1b} and R^{n1b} are each independently H, C₁₋₆ alkyl or -COOR^{x1}, wherein, R^{x1} is C₁₋₆ alkyl;
or, R^{m1} and Rⁿ¹, R^{m1a} and R^{n1a}, and R^{m1b} and R^{n1b}, together with the nitrogen atom to which they are both attached, form a 5-6 membered heterocyclic ring, and of said 5-6 membered heterocyclic ring, the heteroatom is selected from 1 or 2 N atoms; the said 5-6 membered heterocyclic ring is optionally substituted with one or more R^{0'};
R^{z} is selected from C₁₋₆ alkyl;
R⁰ and R^{0'} are each independently selected from C₁₋₆ alkyl, -NR^{m2}Rⁿ² or 5-6 membered heterocyclyl optionally substituted with C₁₋₆ alkyl; of said 5-6 membered heterocyclyl, the heteroatom is selected from 1 or 2 N atoms;
R^{m2} and Rⁿ² are each independently selected from H and C₁₋₆ alkyl;
L₄ is absent or present, when L₄ is present, L₄ is selected from 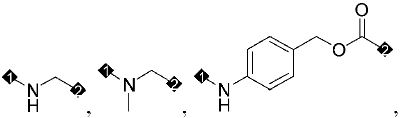 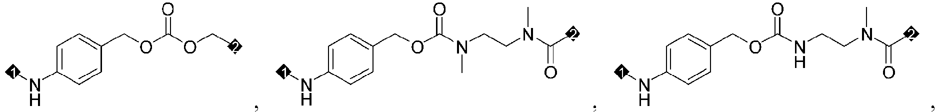 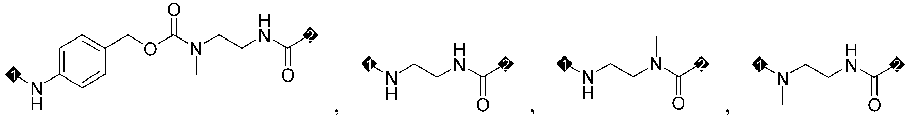 and 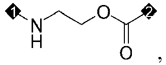 position 1 is attached to L₃, and position 2 is attached to D;
R₁ and R₂ are each independently selected from H, halogens and C1-4 alkyl; or, R₁ and R₂, together with the carbon atom to which they are both attached form a 5-6 membered heterocyclic ring, the heterocyclic ring contains 1, 2, or 3 of O, S, or N or any combination thereof;
R₃ is selected from H and C₁₋₄ alkyl; or R₃ and X, together with the carbon atom to which they are both attached, form a 5-6 membered carbon ring;
W is absent or present, when W is present, W is selected from -O-, -S-, -NR₄-, 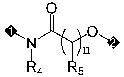 and 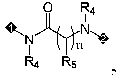 position 1 is attached to X, and position 2 is attached to L₄ or L₃;
X is selected from optionally substituted -(CH₂)ₙ₁-, 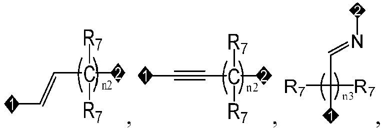 position 1 is attached to the parent ring and position 2 is attached to W or L₄; the substituent is selected from one or two C1-4 alkyls;
R₄, R₅, and R₇ are each independently selected from H and C 1-4 alkyl;
n, n1, n2, n3 are each independently selected from any integer between 0 and 6.

In addition, it should also be noted that, for "position 1 of L₁ is attached to Tb via S atom", it can be understood by those skilled in the art that position 1 of L₁ is connected to the sulfhydryl group comprised in Tb (such as antibody) after disulfide bonds are opened (for example, reduction of the disulfide bonds by the reducing agent TCEP can break the disulfide bond to generate sulfhydryl -SH groups). That is, -S-between L₁ and Tb is not an additional extraneous sulfur atom. For example, in 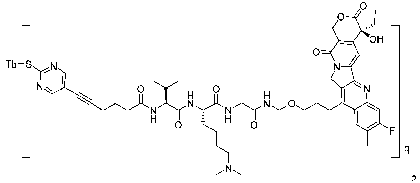 -S- is not an additional extraneous sulfur atom, but is -S-formed by linking the sulfhydryl group comprised in Tb after opening the disulfide bond to position 1 of L₁ such as

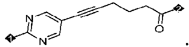

In some embodiments, L₁ is selected from 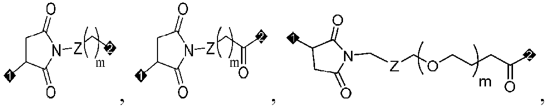 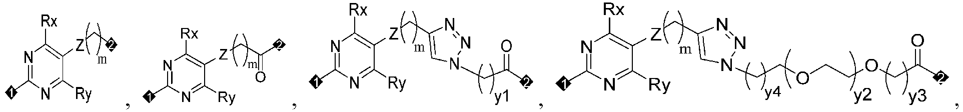 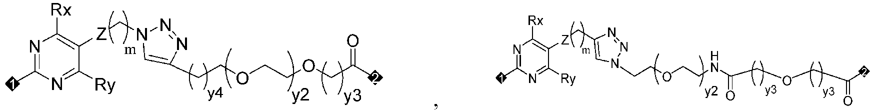 and 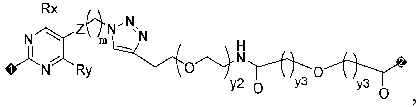 each Z is independently selected from a direct bond, a carbon-carbon triple bond, a carbon-carbon double bond, C6 aryl, and amido (preferably selected from a direct bond, a carbon-carbon triple bond, and a carbon-carbon double bond); Rx and Ry are each independently selected from H and C1-4 alkyl;; each m is independently selected from 0, 1, 2, 3, 4, 5 and 6;y1 is selected from any integer between 1 and 6 (such as 4, 5, and 6); each y2 is independently selected from any integer between 0 and 15 (such as 6 to15); each y3 is independently selected from 1, 2, and 3; each y4 is independently selected from 0 and 1; position 1 is attached to Tb via an S atom, and position 2 is attached to L₂ or L₃.

In some embodiments, L₁ is selected from 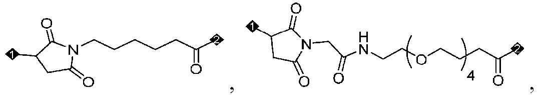 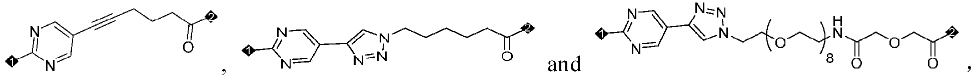 position 1 is attached to Tb via an S atom, and position 2 is attached to L₂ or L₃.

In some embodiments, L₁ is selected from 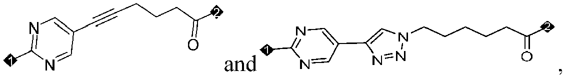 position 1 is attached to Tb via an S atom, and position 2 is attached to L₂ or L₃.

In some embodiments, L₂ is absent or present, and when L₂ is present, L₂ is selected from 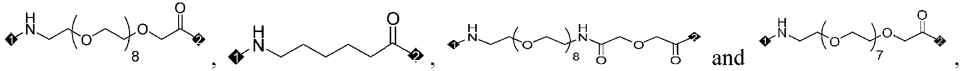 position 1 is attached to L₁, and position 2 is attached to L₃.

In some embodiments, L₂ is absent.

In some embodiments, L₃ is selected from AA¹, AA'-Gly, Val-Cit, Val-Ala, Val-AA¹, Val-AA¹-Gly, AA¹-Ala-Asn, Ala-Ala-Ala, Ala-Ala-Asn and Gly-Gly-Phe-Gly.

In some embodiments, L₃ is selected from AA¹, AA¹-Gly, Val-Cit, Val-AA¹-Gly, AA¹-Ala-Asn and Gly-Gly-Phe-Gly.

In some embodiments, L₃ is selected from Val-AA'-Gly.

In some embodiments, r, r¹, r^{1a} and r^{1b} are each independently selected from 0 or 4; preferably, r is 0, and r¹, r^{1a} and r^{1b} are 4; or r is 4, r¹, r^{1a} and r^{1b} are 0.

In some embodiments, r, r¹, r^{1a} and r^{1b} are not all 0.

In some embodiments, r is 0, and r¹ is 4.

In some embodiments, R^{m1}, Rⁿ¹, R^{m1a}, R^{n1a}, R^{m1b} and R^{n1b} are each independently H, methyl, ethyl, n-propyl, n-butyl, -COOCH₃, -COOCH₂CH₃, -COOCH₂CH₂CH₃, -COOCH(CH₃)₂, -COOC(CH₃)₃ or - COOCH₂CH₂CH₂CH₃.

In some embodiments, R^{m1}, Rⁿ¹, R^{m1a}, R^{n1a}, R^{m1b}, and R^{n1b} are each independently H, methyl, ethyl, n-propyl, -COOCH₃, -COOCH₂CH₃, -COOCH₂CH₂CH₃, -COOCH(CH₃)₂, -COOC(CH₃)₃, or - COOCH₂CH₂CH₂CH₃. In some embodiments, R^{m1} and Rⁿ¹, R^{m1a} and R^{n1a}, and R^{m1b} and R^{n1b}, together with the nitrogen atom to which they are both attached, form a piperidine ring or a piperazine ring; and further preferably 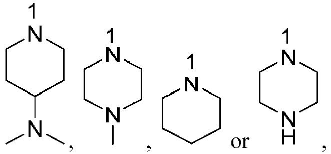 and the nitrogen atom marked with number 1 is the nitrogen atom to which R^{m1} and Rⁿ¹ are both attached.

In some embodiments, R^{m1} and Rⁿ¹, R^{m1a} and R^{n1a}, and R^{m1b} and R^{n1b}, together with the nitrogen atom to which they are both attached, form a piperidine ring; and further preferably 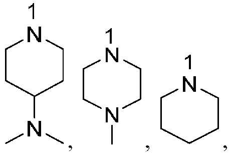 or 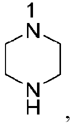 and the nitrogen atom marked with number 1 is the nitrogen atom to which R^{m1} and Rⁿ¹ are both attached.

In some embodiments, for r and r¹, when r is 4, and r¹ is 0, R^{m1} and Rⁿ¹ are each independently selected from H, C1-6 alkyl (e.g., H, methyl); when r is 0, and r¹ is 4, R^{m1} and Rⁿ¹ are each independently selected from C1-6 alkyl (e.g., methyl, ethyl, n-propyl), and preferably selected from C2-6 alkyl (e.g., ethyl, n-propyl).

In some embodiments, when r is 0, and r¹ is 4, R^{m1} and Rⁿ¹ are each independently selected from C₁₋₆ alkyl (e.g., methyl, ethyl, and n-propyl).

In some embodiments, R^{z} is methyl.

In some embodiments, R⁰ is C₁₋₆ alkyl, or piperidinyl or piperazinyl substituted with C₁₋₆ alkyl; and preferably is methyl, ethyl or piperidinyl substituted with methyl; such as methyl or

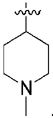

In some embodiments, R^{01'} is each independently C₁₋₆ alkyl or -NR^{m2}Rⁿ²; more preferably is methyl or - NR^{m2}Rⁿ², wherein, R^{m2} and Rⁿ² are each independently preferably H or C₁₋₆ alkyl, and more preferably, are methyl.

In some embodiments, in the amino acid residue represented by AA¹, R^{a1} and R^{b}, together with the carbon atom to which they are both attached, form a piperidine ring, e.g. 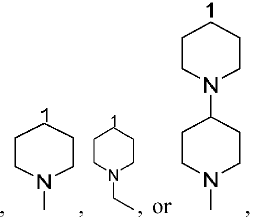 and the carbon atom marked with number 1 is the carbon atom to which R^{a} and R^{b} are both attached; such as

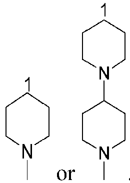

In some embodiments, the amino acid residue represented by AA¹ is selected from

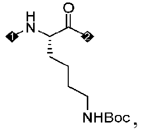

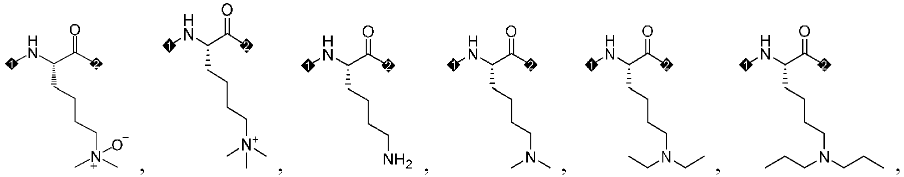

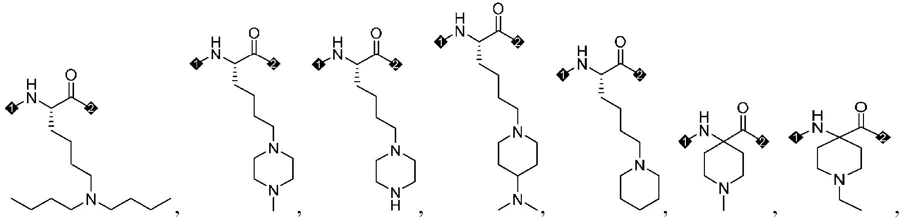

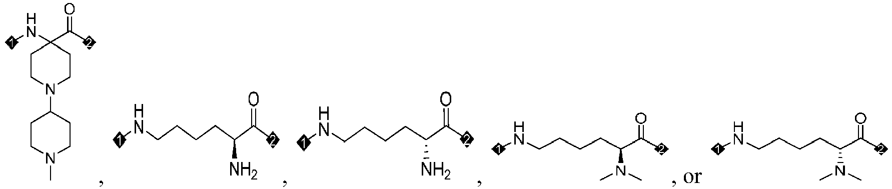

In some embodiments, the amino acid residue represented by AA¹ is selected from

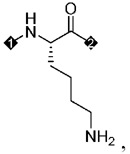

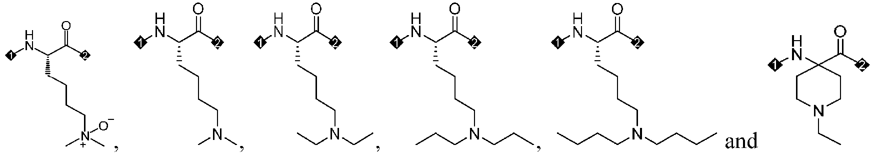

In some embodiments, the amino acid residue represented by AA¹ is selected from 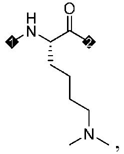 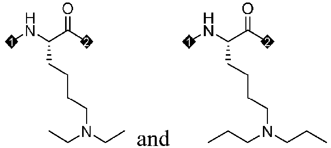 In some embodiments, L₃ is selected from 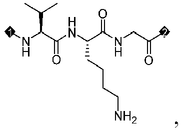 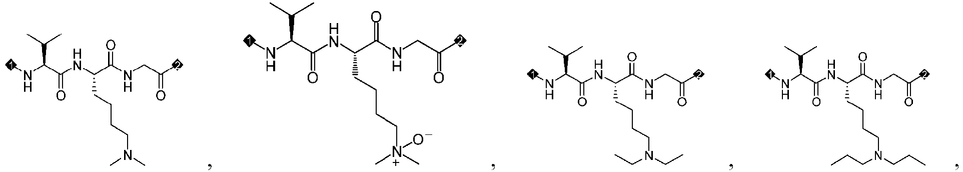 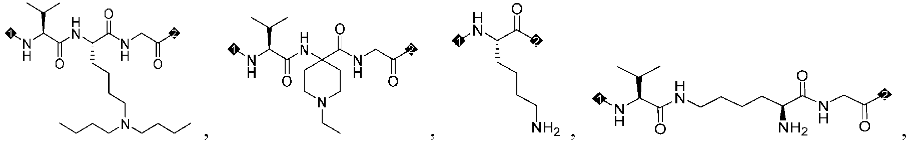 and 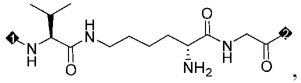 position 1 is attached to L₁ or L₂, and position 2 is attached to L₄ or D.

In some embodiments, L₃ is selected from 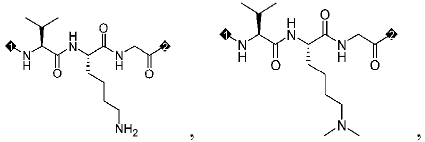 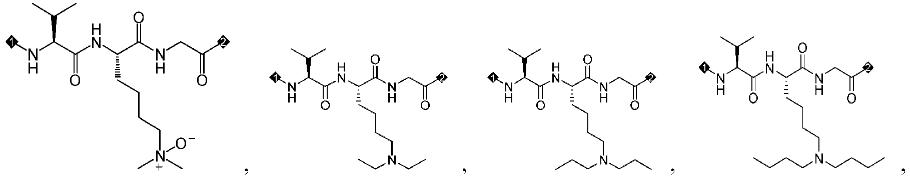 and 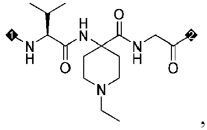 position 1 is attached to L₁ or L₂, and position 2 is attached to L₄ or D.

In some embodiments, L₃ is selected from 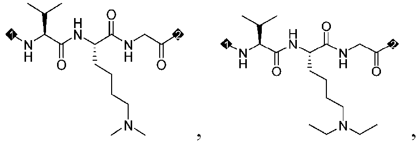 and 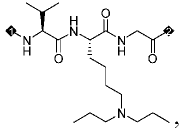 position 1 is attached to L₁ or L₂, and position 2 is attached to L₄ or D. In some embodiments, L₄ is absent or present, when L₄ is present, L₄ is selected from 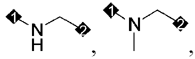 and 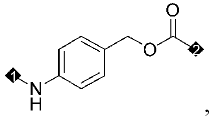 position 1 is attached to L₃, and position 2 is attached to D.

In some embodiments, L₄ is absent.

In some embodiments, L₄ is selected from 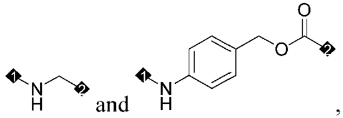 position 1 is attached to L₃, and position 2 is attached to D.

In some embodiments, L₄ is selected from 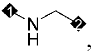 position 1 is attached to L₃, and position 2 is attached to D.

In some embodiments, R₁ is selected from H and halogen, and R₂ is selected from H and C1-4 alkyl.

In some embodiments, R₁ and R₂, together with the carbon to which they are both attached, form 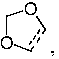 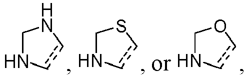 the "dotted line" indicates where the heterocyclic ring is fused to the benzene ring.

In some embodiments, R₁ is H or F, and R₂ is H or methyl.

In some embodiments, R₁ is F, R₂ is methyl, or R₁ and R₂ together with the carbon atoms to which they are attached form

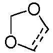

In some embodiments, R₁ is F, and R₂ is methyl.

In some embodiments, R₁ and R₂ together with the carbon atoms to which they are attached form

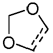

In some embodiments, R₃ is H; or, R₃ and X together with the carbon atoms to which they are attached form 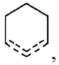 the dotted line indicates where the carbocyclic ring is fused to the benzene ring and pyridine ring.

In some embodiments, R₃ is H.

In some embodiments, each R₄ is independently selected from H and C1-4 alkyl, and R₅ is H.

In some embodiments, each R₄ is independently selected from H, methyl, ethyl, n-propyl, isopropyl and tert-butyl, and R₅ is H.

In some embodiments, each R₇ is independently selected from H and C1-4 alkyl.

In some embodiments, R₇ is H.

In some embodiments, n is selected from 1, 2, and 3.

In some embodiments, n is 1.

In some embodiments, n1 is selected from 1, 2, 3, and 4.

In some embodiments, n2 is 1.

In some embodiments, n3 is 0.

In some embodiments, W is selected from -O-, -NR₄- and 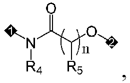 position 1 is attached to X, and position 2 is attached to L₄ or L₃.

In some embodiments, W is selected from -O- and -NR₄-, position 1 is attached to X, and position 2 is attached to L₄ or L₃.

In some embodiments, X is selected from optionally substituted 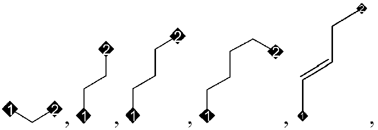 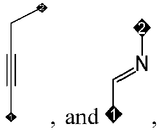 position 1 is attached to the parent ring, and position 2 is attached to W or L₄; the substituent is selected from 1 or 2 C1-4 alkyl groups (e.g., methyl), or 2 C1-4 alkyl groups (e.g., methyl).

In some embodiments, X is selected from 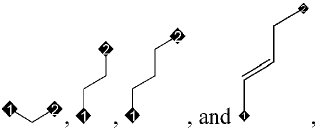 position 1 is attached to the parent ring, and position 2 is attached to W.

In some embodiments, W is selected from -O-, -NR₄-, and 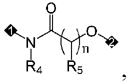 position 1 is attached to X, and position 2 is attached to L₄ or L₃; X is selected from 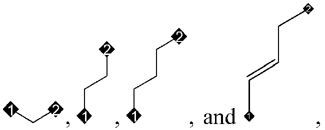 position 1 is attached to the parent ring, and position 2 is attached to W.

W is absent or present, and so, when W is absent, position 1 of L₄ is attached to L₃, and position 2 is attached to X; when W is present, position 1 of L₄ is attached to L₃, and position 2 is attached to W. The following connection relationship for L₄ can be understood with reference to the foregoing.

In some embodiments, the structure 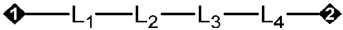 is selected from the following structural fragments: 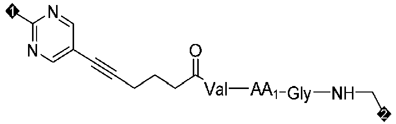 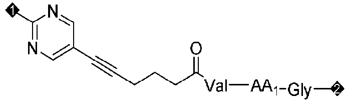 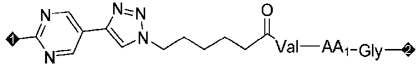 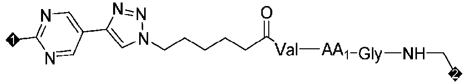 wherein, position 1 is attached to Tb, and position 2 is attached to D; AA¹ is defined above.

In some embodiments, the structure 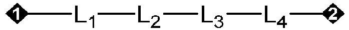 is selected from the following structural fragments:

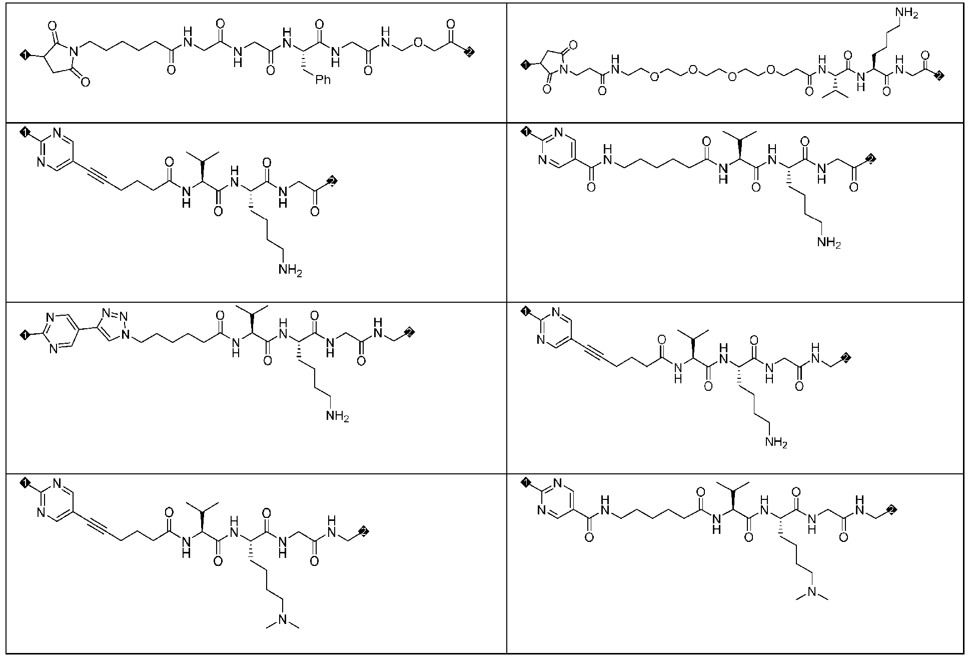

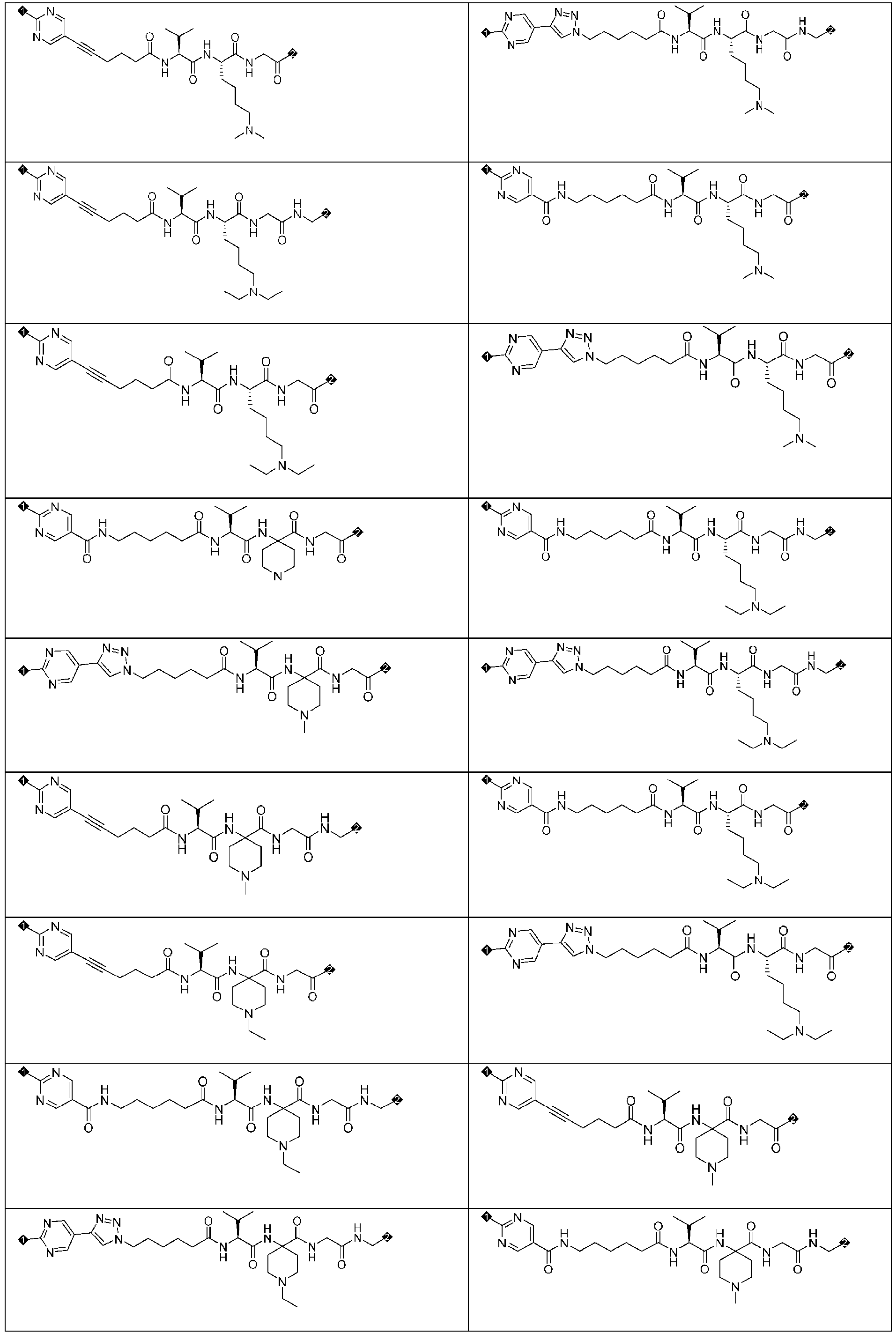

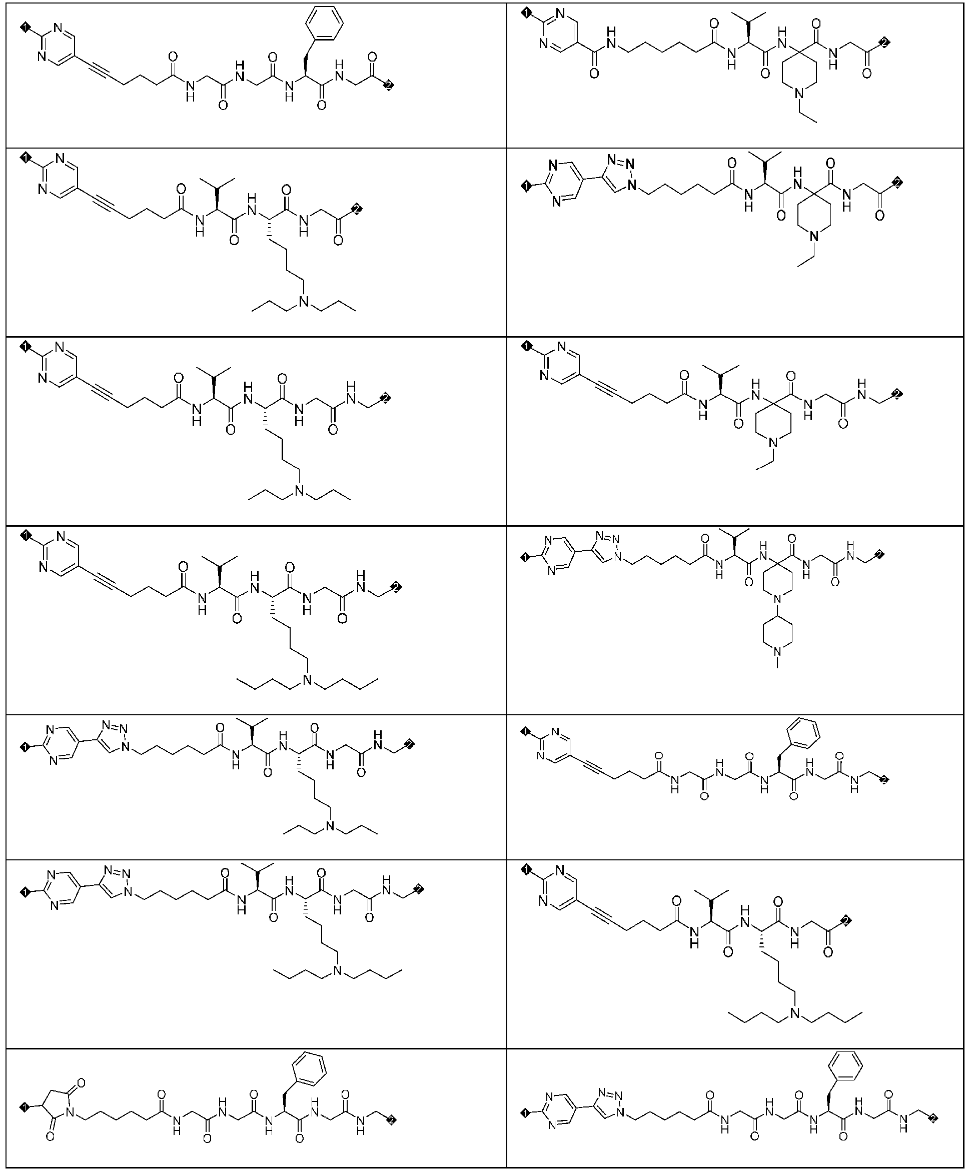

wherein, position 1 is attached to Tb, and position 2 is attached to W.

In some embodiments, the structure 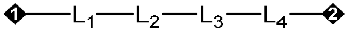 is selected from the following structural fragments:

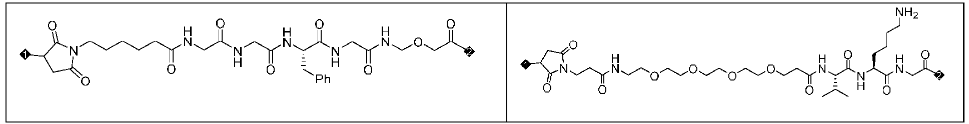

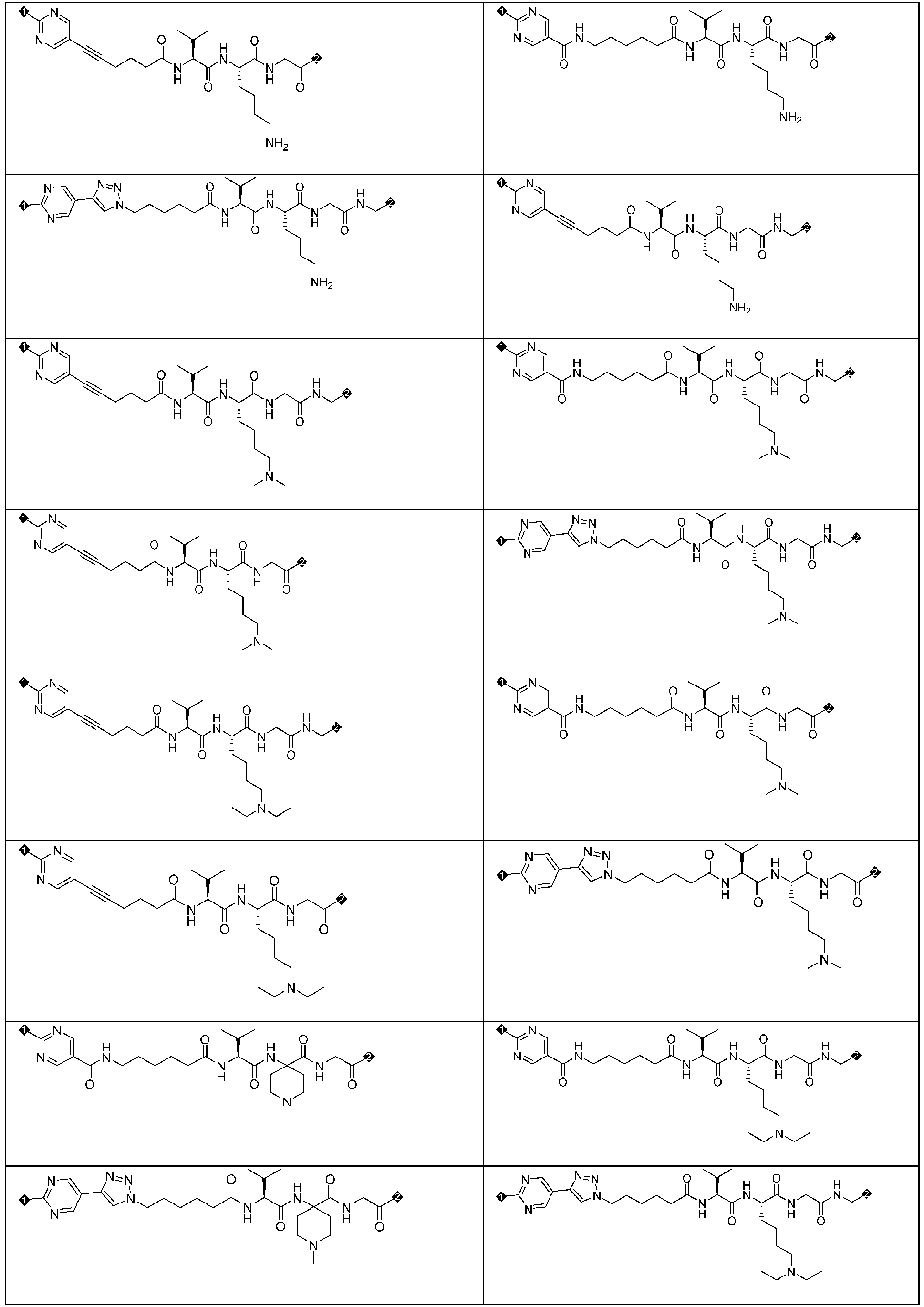

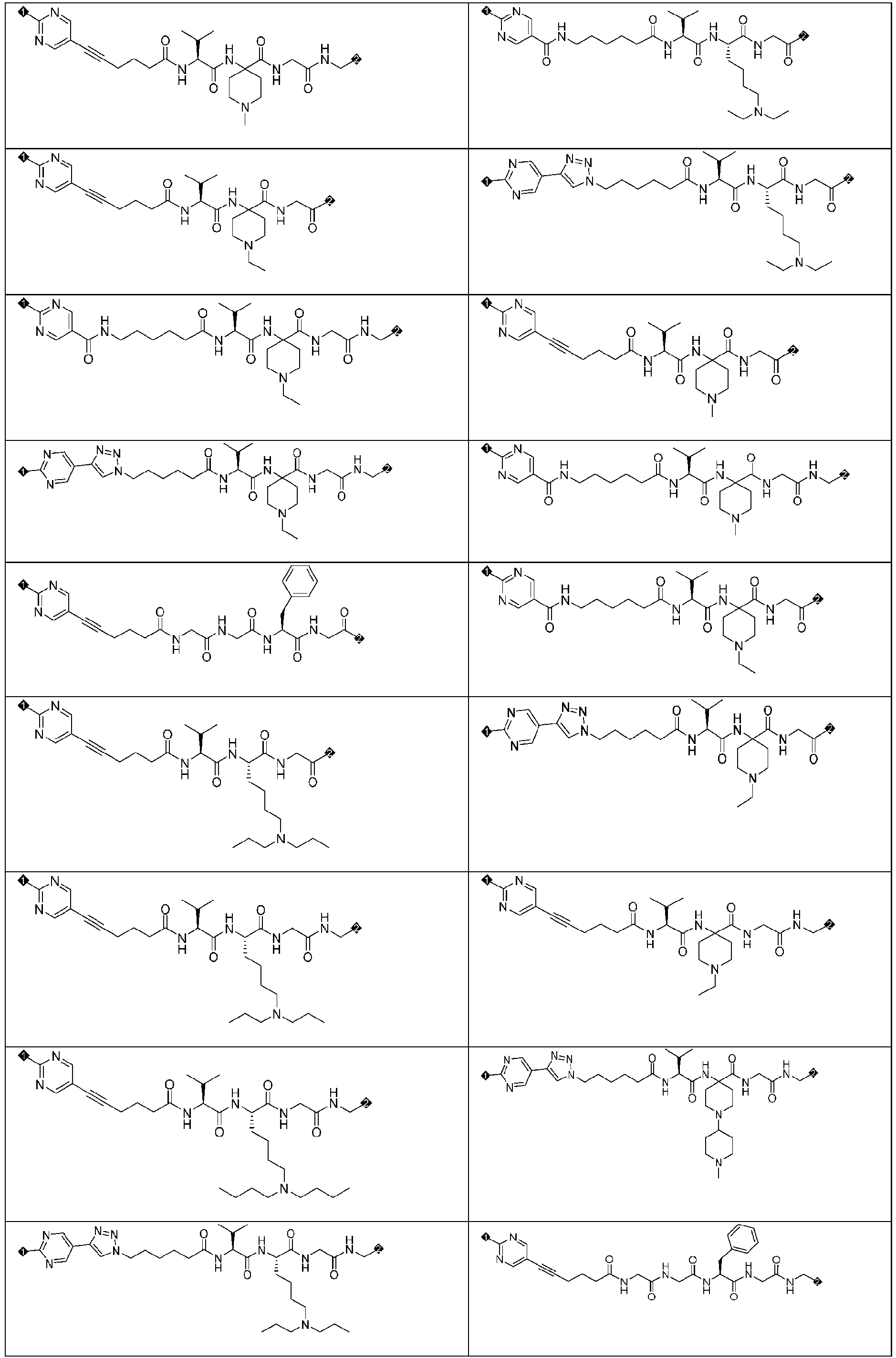

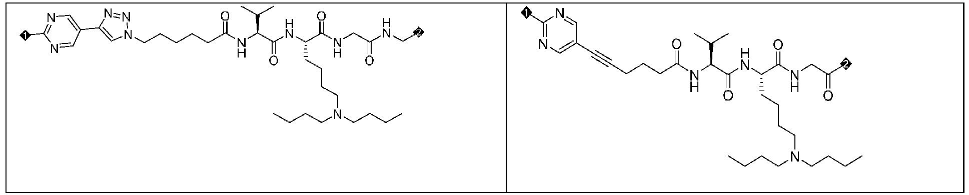

wherein, position 1 is attached to Tb, and position 2 is attached to W.

In some embodiments, the structure 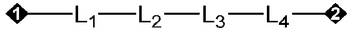 is selected from the following structural fragments: 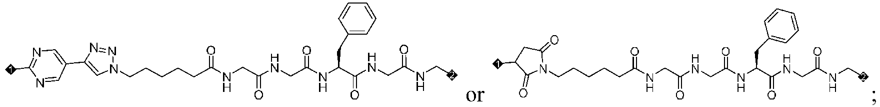 wherein, position 1 is attached to Tb via an S atom, and position 2 is attached to W;

In some embodiments, the structural fragment represented by 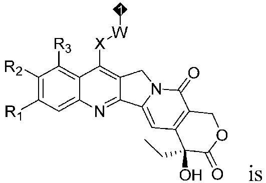 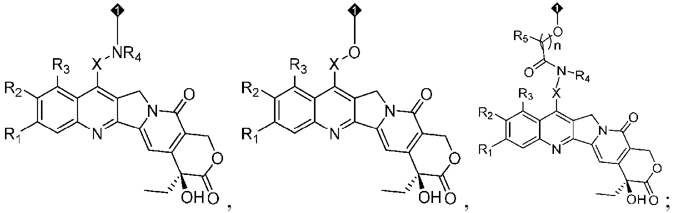 position 1 is attached to L₄.

In some embodiments, the structure 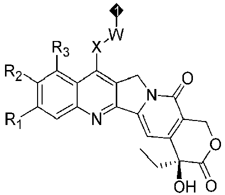 is selected from the following structural fragments:

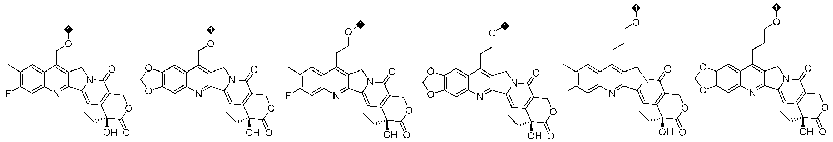

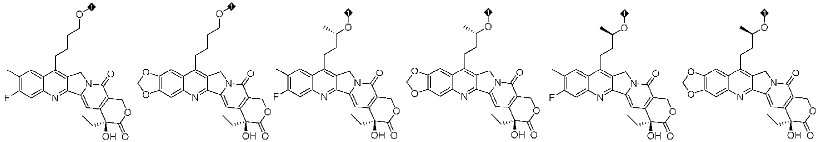

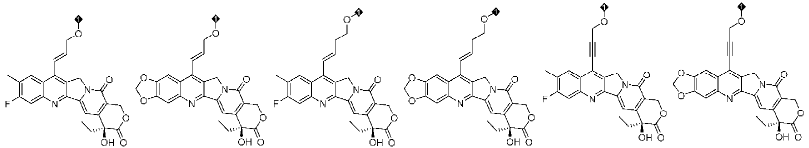

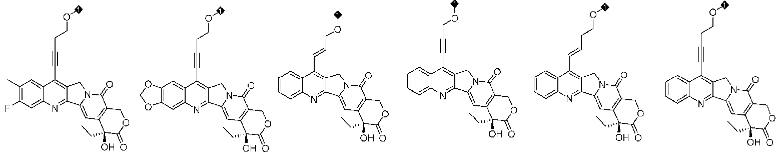

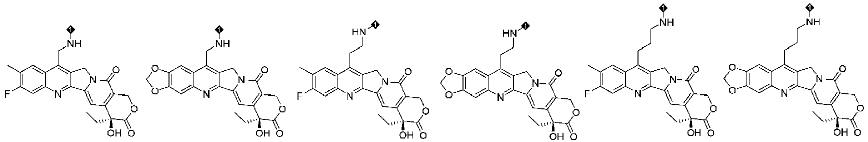

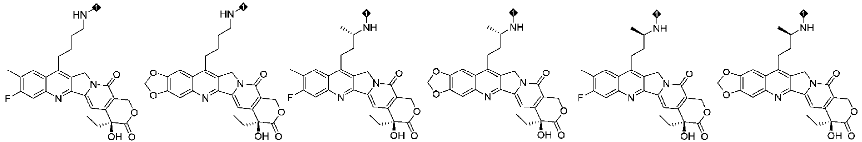

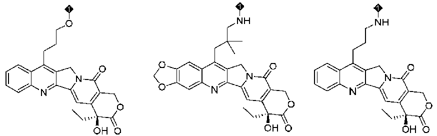

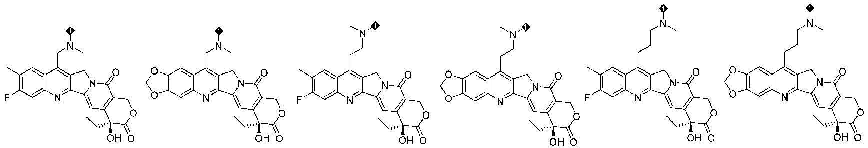

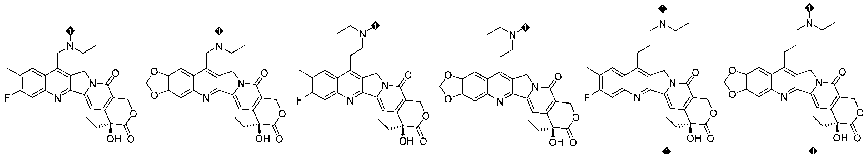

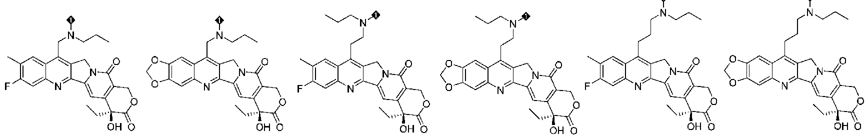

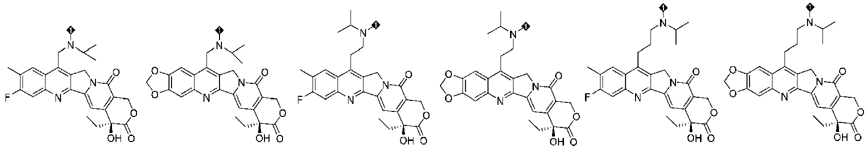

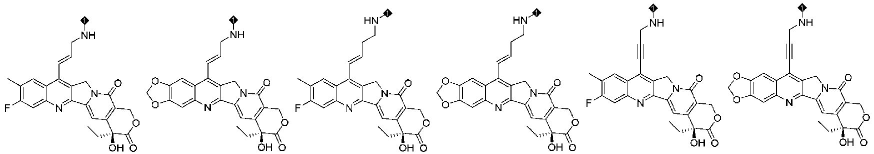

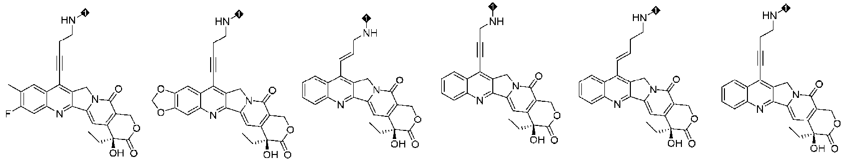

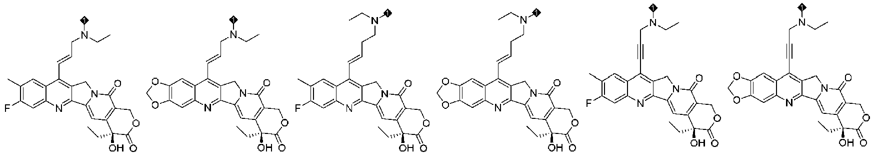

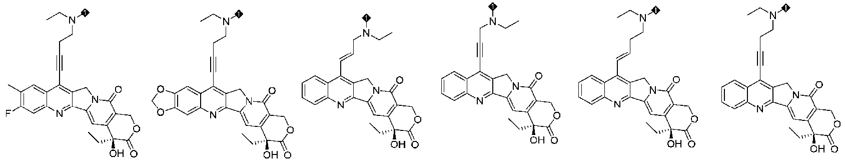

In some embodiments, the structure 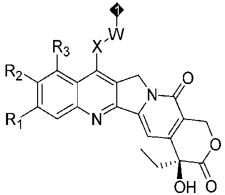 is selected from the following structural fragments:

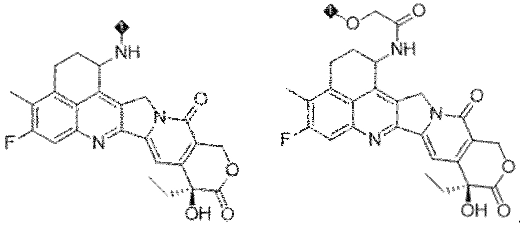

In some embodiments, the ligand-drug conjugate has the structure represented by formula I-1: 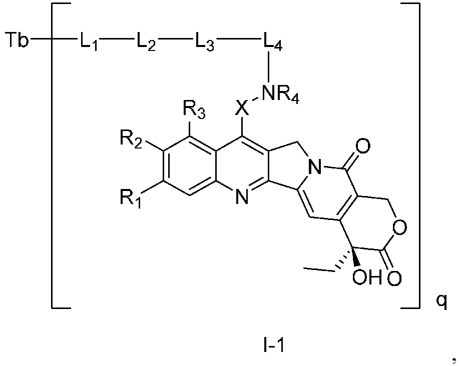 wherein, Tb, L₁, L₂, L₃, L₄, X, R₁, R₂, R₃, R₄, and q are as defined in any embodiment specifically described above and herein.

In some embodiments, the ligand-drug conjugate has the structure represented by formula I-1A or I-1B: 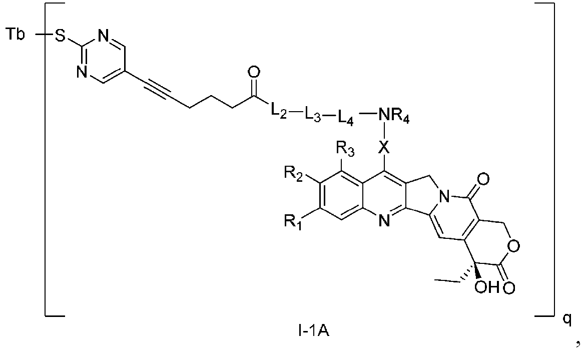 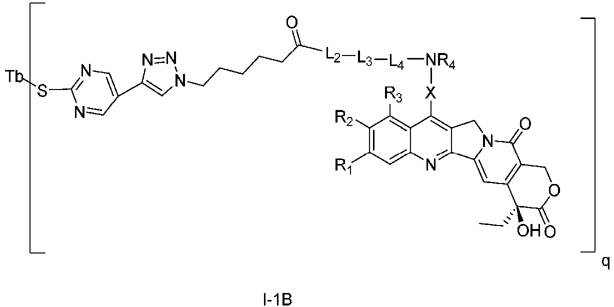 wherein, Tb, L₂, L₃, L₄, X, R₁, R₂, R₃, R₄, and q are as defined in any embodiment specifically described above and herein.

In some embodiments, the ligand-drug conjugate has the structure represented by formula 1-2: 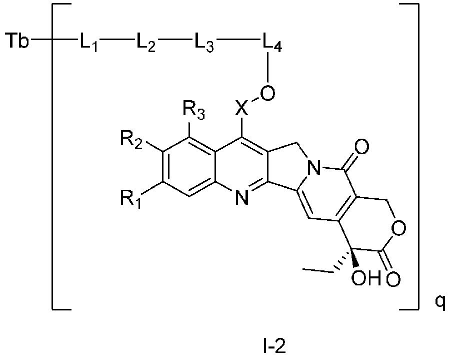 wherein, Tb, L₁, L₂, L₃, L₄, X, R₁, R₂, R₃, and q are as defined in any embodiment specifically described above and herein.

In some embodiments, the ligand-drug conjugate has the structure represented by formula I-2A or I-2B: 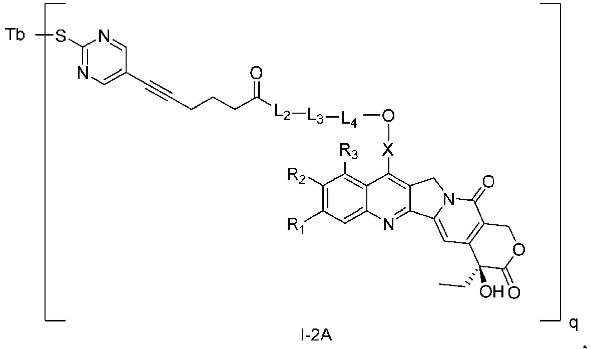 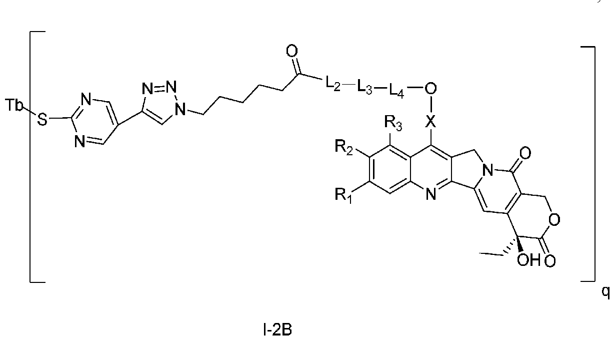 wherein, Tb, L₂, L₃, L₄, X, R₁, R₂, R₃, and q are as defined in any embodiment specifically described above and herein.

In some embodiments, the ligand-drug conjugate has the structure represented by formula 1-3: 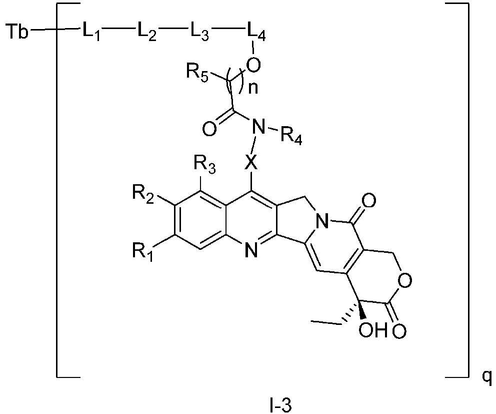 wherein, Tb, L₁, L₂, L₃, L₄, X, R₁, R₂, R₃, R₄, R₅, n, and q are as defined in any embodiment specifically described above and herein.

In some embodiments, the ligand-drug conjugate has the structure represented by formula I-3A or I-3B: 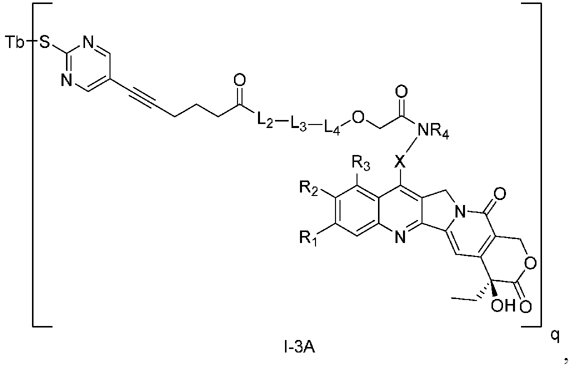 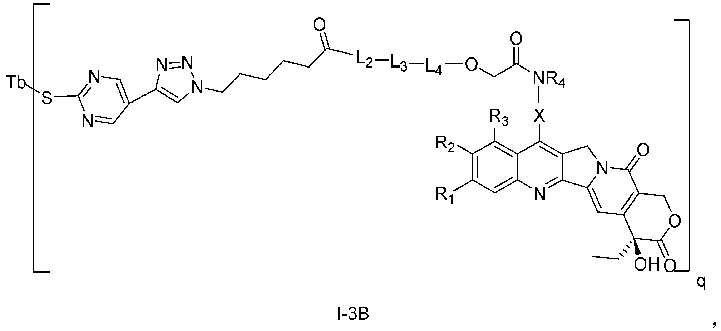 wherein, Tb, L₂, L₃, L₄, X, R₁, R₂, R₃, R₄, and q are as defined in any embodiment specifically described above and herein.

In some embodiments, the ligand-drug conjugate has the structure represented by formula I-A: 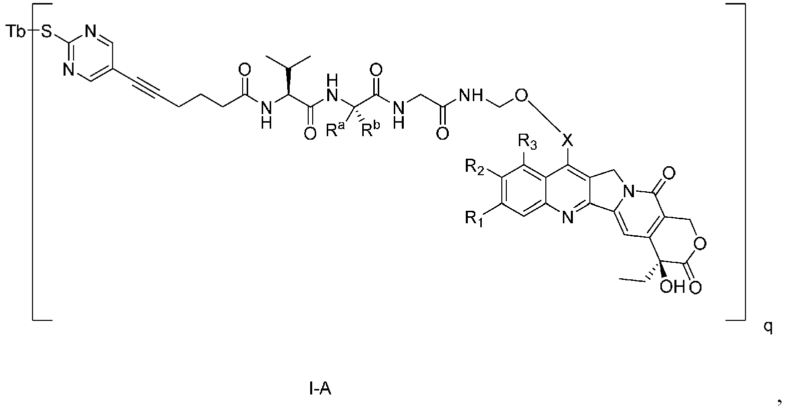 wherein, Tb, X, R₁, R₂, R₃, R^{a}, R^{b}, and q are as defined in any embodiment specifically described above and herein.

In some embodiments, the ligand-drug conjugate has the structure represented by formula I-B: 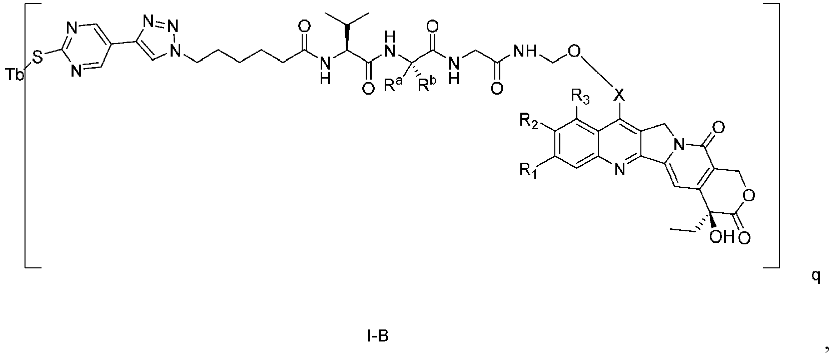 wherein, Tb, X, R₁, R₂, R₃, R^{a}, R^{b}, and q are as defined in any embodiment specifically described above and herein.

In some preferred embodiments, the ligand-drug conjugate is selected from:

| The structures of the ligand-drug conjugates |
|---|
| 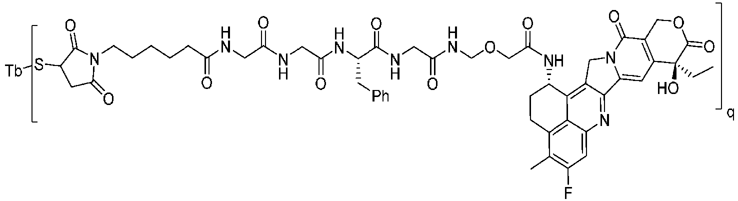 |
| 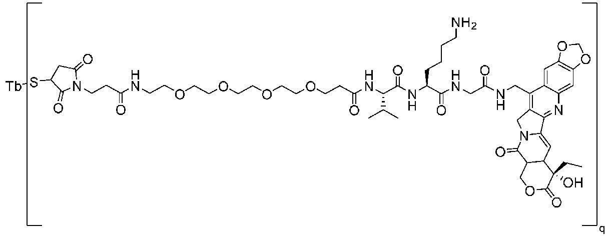 |
| 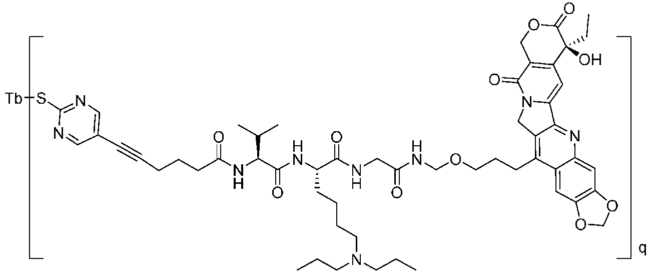 |
| 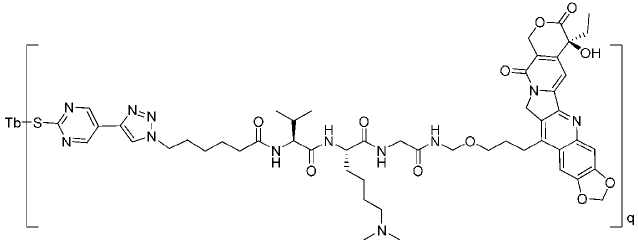 |
| 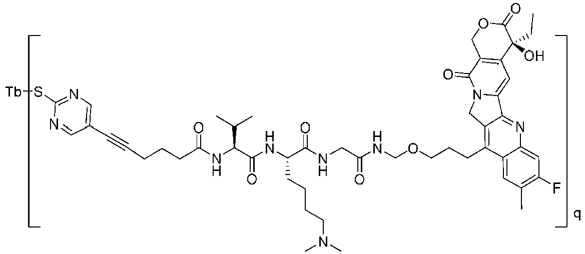 |
| 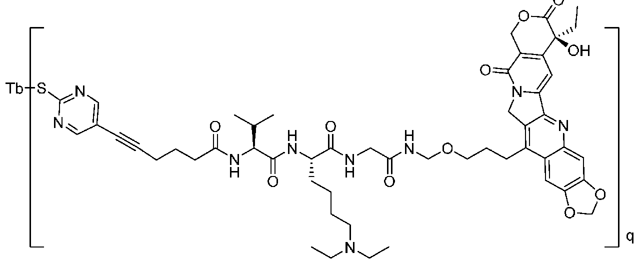 |
| 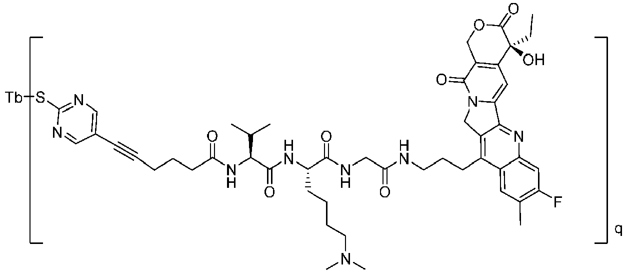 |
| 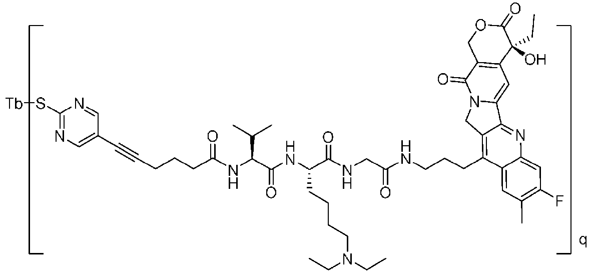 |
| 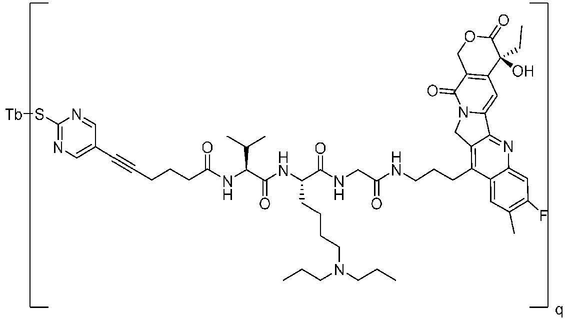 |
| 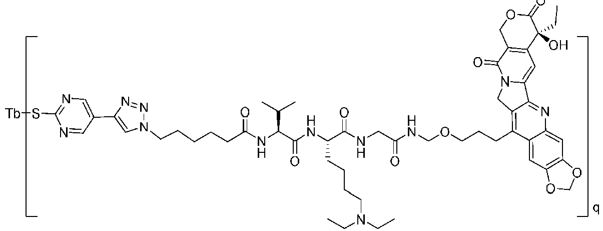 |
| 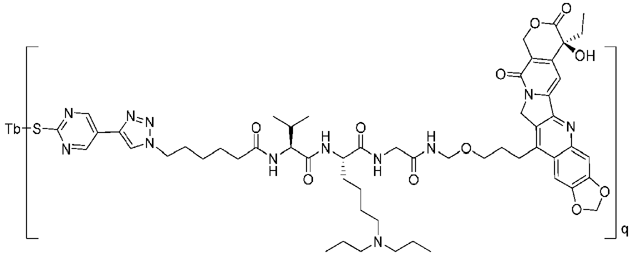 |
| 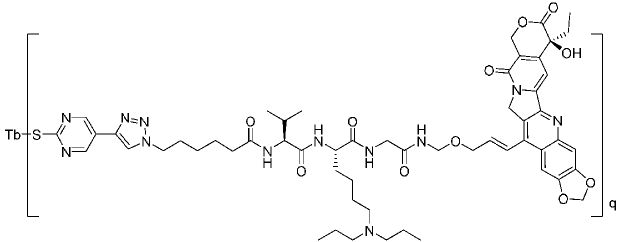 |
| 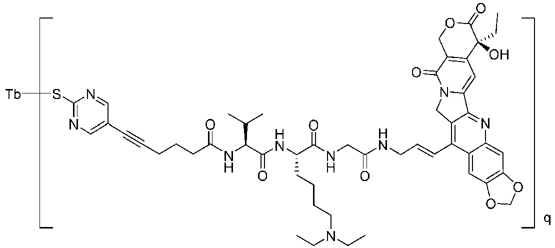 |
| 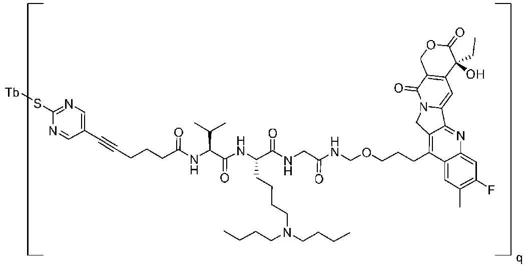 |
| 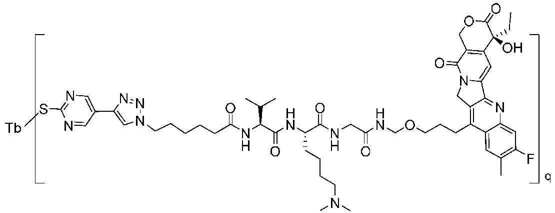 |
| 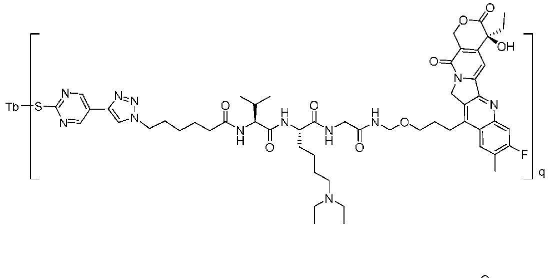 |
| 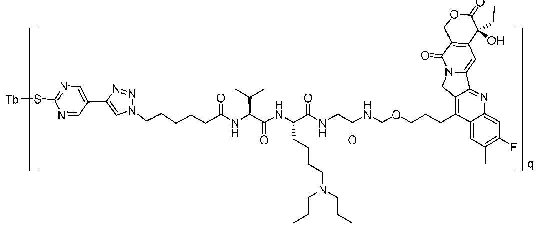 |
| 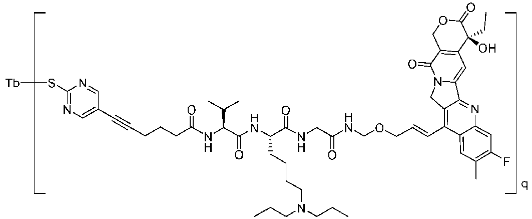 |
| 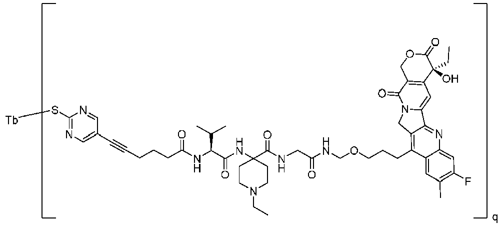 |
| 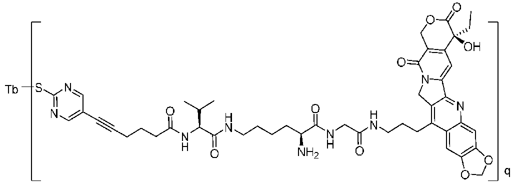 |

wherein, Tb is Her3 antibody as described in any embodiment above; q is 1, 2, 3, 4, 5, 6, 7, 8, 9, 10, 11, 12, 13, 14, 15, 16; and q is preferably 1, 2, 3, 4, 5, 6, 7, 8;
for example, 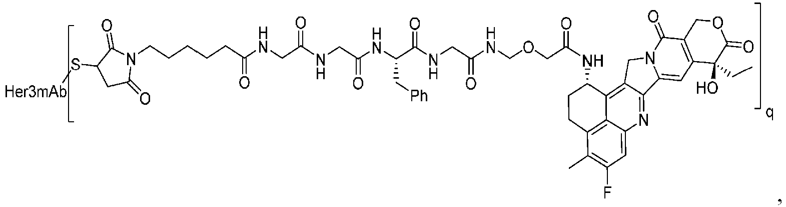 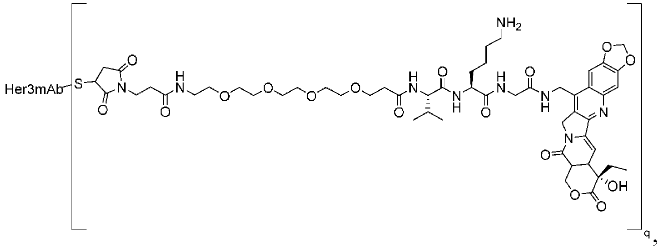 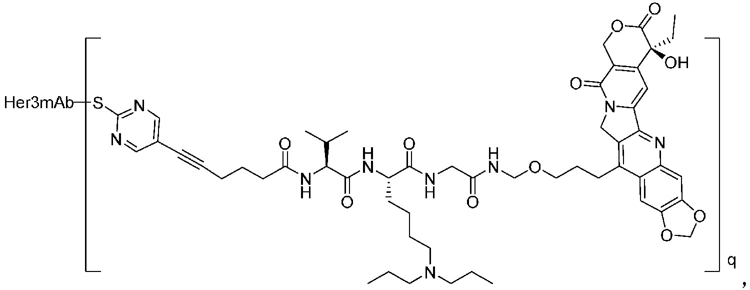 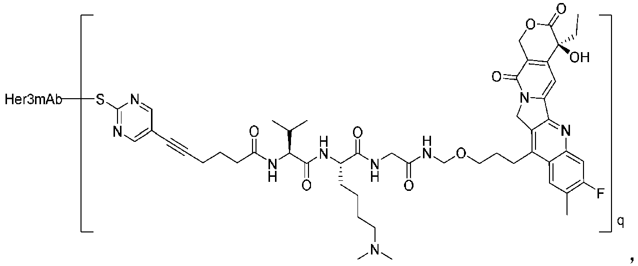 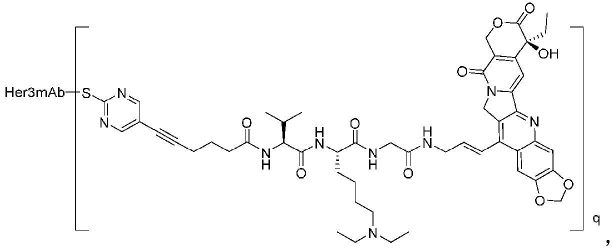 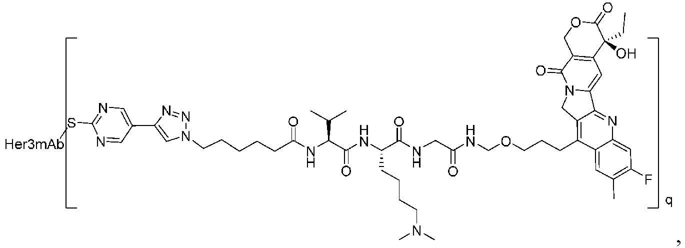
wherein, Her3mAb is antibody 202-2-1.

In some preferred embodiments, the ligand-drug conjugate is selected from:

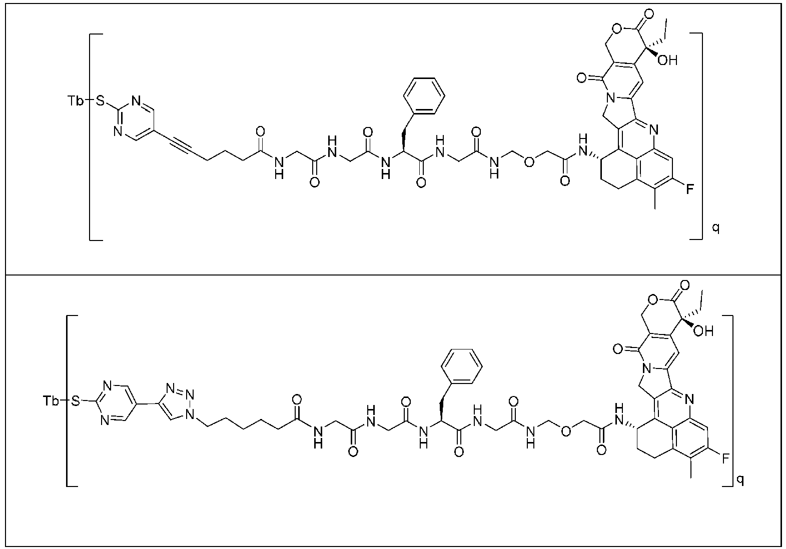

wherein, Tb is antibody Her3 as described in any embodiment above; q is 1, 2, 3, 4, 5, 6, 7, 8, 9, 10, 11, 12, 13, 14, 15, or 16; and q is preferably 1, 2, 3, 4, 5, 6, 7, or 8.

In some preferred embodiments, the ligand-drug conjugate is selected from: 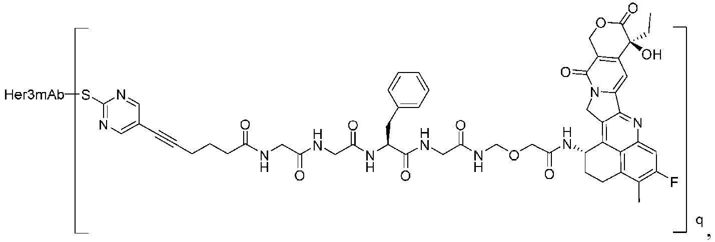 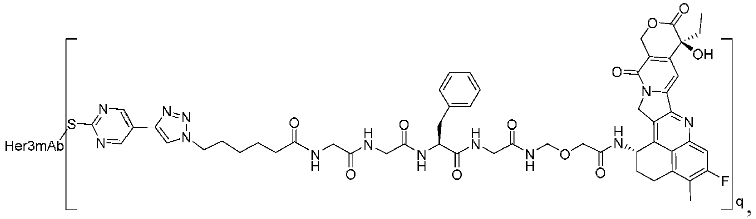 wherein, Her3mAb is antibody 202-2-1.

In some preferred embodiments, q in the ligand-drug conjugate is 1, 2, 3, 4, 5, 6, 7, 8, 9, 10, 11, 12, 13, 14, 15, or 16; q is preferably 1, 2, 3, 4, 5, 6, 7, or 8.

In an eighth aspect of the present disclosure, the present disclosure provides antibody-drug conjugates, which comprises two or more of the antibody-drug conjugates mentioned above, a stereoisomer thereof, prodrugs thereof, pharmaceutically acceptable salts thereof or pharmaceutically acceptable solvates thereof, and the antibody-drug conjugate in the antibody-drug conjugates has one, two or more q values.

In some embodiments, the ratio of drug to antibody (DAR) in the antibody-drug conjugates is selected from an integer or decimal between 1-10.

In some embodiments, the ratio of drug to antibody (DAR) in the antibody-drug conjugates is selected from 2, 2.5, 3, 3.5, 4, 4.5, 5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9 and 9.

In some embodiments, the DAR value of the antibody-drug conjugates is selected from 2 ± 0.5, 4 ± 0.5, 6 ± 0.5, or 8 ± 0.5.

In some embodiments, the DAR value of the antibody-drug conjugates is selected from 2 ± 0.5, 4 ± 0.5, 5 ± 0.5, 6 ± 0.5, 7 ± 0.5, or 8±0.5.

In some embodiments, the DAR value of the antibody-drug conjugates is 8 ± 0.5, 8 ± 0.4, 8 ± 0.3, 8 ± 0.2, 8 ± 0.1, or 8.0.

In some embodiments, the ligand-drug conjugates comprise ADCs having a DAR distribution of 2 to 8, such as 1.5, 2, 4, 6 and 8 (i.e., 1.5, 2, 4, 6 and 8 payload species). It should be noted that degradation products may be generated, such that the ligand drug conjugates may also comprise DAR of 1, 3, 5, and 7. Furthermore, the ADC in the mixture may also have a DAR greater than 8. The ligand drug conjugate is produced by reduction of interchain disulfide followed by coupling. In some embodiments, the ligand drug conjugate comprises both the following: ADC having a DAR of 4 or less (i.e., the payload species are 4 or less) and ADC having a DAR of 6 or more (i.e., the payload species are 6 or more).

### Uses, Treatment Methods, and Pharmaceutical Compositions

In a nineth aspect of the present disclosure, the present disclosure provides a pharmaceutical composition, comprising the antibody or antigen-binding fragment thereof described in the first aspect of the present disclosure; the multi-specific antibody described in the second aspect; the nucleic acid molecule described in the third aspect; the vector described in the fourth aspect; the host cell described in the fifth aspect; the antibody-drug conjugate described in the seventh aspect, a stereoisomer thereof, a prodrug thereof, a pharmaceutically acceptable salt thereof or a pharmaceutically acceptable solvate thereof, and/or the antibody-drug conjugate described in the eighth aspect, as well as a pharmaceutically acceptable carrier and/or excipient.

The antibody or antigen-binding fragment thereof described in the first aspect of the present disclosure; the multi-specific antibody described in the second aspect; the nucleic acid molecule described in the third aspect; the vector described in the fourth aspect, the host cell described in the fifth aspect; the antibody-drug conjugate described in the seventh aspect, a stereoisomer thereof, a prodrug thereof, a pharmaceutically acceptable salt thereof or a pharmaceutically acceptable solvate thereof, and/or the antibody-drug conjugate described in the eighth aspect, which may be in a therapeutically effective amount.

In certain embodiments, the pharmaceutical composition of the present disclosure comprises an antibody or antigen-binding fragment thereof of the present disclosure, and a pharmaceutically acceptable carrier and/or excipient.

In certain embodiments, the pharmaceutical composition of the present disclosure comprises a host cell of the present disclosure, and a pharmaceutically acceptable carrier and/or excipient, wherein the host cell comprises an isolated nucleic acid molecule or vector as previously described.

In certain embodiments, the pharmaceutical composition of the present disclosure comprises the multi-specific antibodies of the present disclosure, and a pharmaceutically acceptable carrier and/or excipient.

In certain embodiments, the pharmaceutical composition of the present disclosure comprises the antibody-drug conjugate of the present disclosure, a stereoisomer thereof, a prodrug thereof, a pharmaceutically acceptable salt thereof or a pharmaceutically acceptable solvate thereof, and/or the antibody-drug conjugate, as well as a pharmaceutically acceptable carrier and/or excipient.

In another aspect of the present disclosure, the present disclosure provides the use of the antibody or antigen-binding fragment thereof, the nucleic acid, the vector, the host cell, the multi-specific antibody, or the antibody drug conjugate, a stereoisomer thereof, a prodrug thereof, a pharmaceutically acceptable salt thereof or a pharmaceutically acceptable solvate thereof, the antibody-drug conjugate, or the pharmaceutical composition according to the present disclosure in the manufacture of a medicament for modulating (inhibiting or blocking) the activity of Her3. The antibody or antigen-binding fragment thereof, the nucleic acid molecule, the vector, the host cell, the multi-specific antibody, or the antibody-drug conjugate, a stereoisomer thereof, a prodrug thereof, a pharmaceutically acceptable salt thereof or a pharmaceutically acceptable solvate thereof may be in a therapeutically effective amount.

In some examples, the present disclosure provides the use of the antibody or antigen-binding fragment thereof, the nucleic acid, the vector, the host cell, the multi-specific antibody, the antibody drug conjugate, a stereoisomer thereof, a prodrug thereof, a pharmaceutically acceptable salt thereof or a pharmaceutically acceptable solvate thereof, the antibody-drug conjugate, or the pharmaceutical composition according to the present disclosure in the manufacture of a medicament for the treatment or prevention of diseases related to Her3 activity.

In some examples, the present disclosure provides the use of the antibody or antigen-binding fragment thereof, the nucleic acid, the vector, the host cell, the multi-specific antibody, the antibody drug conjugate, a stereoisomer thereof, a prodrug thereof, a pharmaceutically acceptable salt thereof or a pharmaceutically acceptable solvate thereof, the antibody-drug conjugate, or the pharmaceutical composition according to the present disclosure in the manufacture of a medicament for the treatment or prevention of tumors related to Her3 activity.

In some embodiments, the cancer disease is selected from esophageal cancer (e.g., esophageal adenocarcinoma and esophageal squamous cell carcinoma), brain tumor, lung cancer (e.g., small cell lung cancer and non-small cell lung cancer), squamous cell carcinoma, bladder cancer, stomach cancer, ovarian cancer, peritoneal cancer, pancreatic cancer, breast cancer, head and neck cancer, cervical cancer, endometrial cancer, colorectal cancer, liver cancer, kidney cancer, urothelial cancer, solid tumor, non-Hodgkin lymphoma, central nervous system tumor (e.g., neuroglioma, glioblastoma multiforme, glioma or sarcoma), prostate cancer or thyroid cancer.

In some embodiments, the cancer is solid tumors.

In some embodiments, the tumor is selected from colorectal cancer (e.g., colon cancer), lung cancer (e.g., non-small cell lung cancer), breast cancer, and prostate cancer.

In some embodiments, the caner is breast cancer or lung cancer (preferably non-small cell lung cancer).

In another aspect, the present disclosure provides the use of the antibody or antigen-binding fragment thereof or the multi-specific antibody of the present disclosure in the manufacture of a kit for detecting the presence of Her3 or its level in a sample. In another aspect, the present disclosure provides diagnostic or therapeutic kits comprising one or more of the following agents: the antibody or antigen binding fragment thereof, the nucleic acid, the vector, the host cell, the multi-specific antibody, the antibody-drug conjugate, or the pharmaceutical composition of the present disclosure. Optionally, the diagnostic or therapeutic kit further includes instructions for use.

### Definitions

In the present disclosure, unless otherwise stated, the scientific and technical terms used herein have the meanings that are generally understood by those skilled in the art. Moreover, the cell culture, biological chemistry, nucleic acid chemistry, and immunology laboratory procedures etc. used herein are all routine procedures widely used in the corresponding field. Meanwhile, for a better understanding of the present disclosure, definitions and explanations of relevant terms are provided below.

The term "HER3 ligand" used herein refers to a polypeptide that binds and activates HER3. Examples of HER3 ligands include, but are not limited to, neuregulin 1 (NRG) and neuregulin 2, betacellulin, heparin-binding epidermal growth factor (HB-EGF) and Epiregulin.

The present disclosure relates to an isolated antibodies or fragment thereof that recognizes a conformational epitope of HER3 receptors, wherein the conformational epitope comprises amino acid residues in domain 3 of HER3, and wherein the antibody or fragment thereof inhibits the phosphorylation of HER3, as evaluated by HER3 ligand-independent phosphorylation assay. In one embodiment, HER3 ligand-independent phosphorylation assay uses cells with amplified HER2, wherein the cells with amplified HER2 are SK-Br-3 cells.

The term "ligand-dependent signaling" used herein refers to a signaling that relies on ligand activation, such as the activation of Erbb (e.g., HER3) by ligands. The activation of HER3 can be demonstrated by increasing oligomerization (e.g., heterodimerization) and/or HER3 phosphorylation in downstream signaling pathways (e.g., PI3K). The antibody or fragment thereof can statistically significantly reduce the amount of phosphorylated HER3 in stimulated cells exposed to an antigen-binding protein (e.g., an antibody) relative to untreated (control) cells when measured using the assays described in the Examples. A cell expressing HER3 can be a naturally occurring cell line (e.g., MCF7), or can be a recombinant cell produced by introducing nucleic acid encoding HER3 protein into a host cell. Cell stimulation can be performed by exogenous addition of an activating HER3 ligand or by endogenous expression of an activating ligand.

The term "ligand-independent signaling" as used herein refers to signaling that does not require ligand binding, such as HER3 activation independent of ligand binding (e.g., phosphorylation). For example, ligand-independent HER3 activation can be the result of overexpression of HER2 or the result of activation mutations of HER3 heterodimer partners such as EGFR and HER2. The antibody or fragment thereof can statistically significantly reduce the amount of phosphorylated HER3 in cells exposed to an antigen binding protein (e.g., an antibody) relative to untreated (control) cells. A cell expressing HER3 can be a naturally occurring cell line (e.g., SK Br-3), or they can be a recombinant cell produced by introducing nucleic acid encoding HER3 protein into a host cell.

As used herein, examples of the term "pharmaceutically acceptable salt" are organic acid addition salts formed with organic acids that provide pharmaceutically acceptable anions, the organic acid addition salts include but are not limited to formate, acetate, propionate, benzoate, maleate, fumarate, succinate, tartrate, citrate, ascorbate, α-ketoglutarate, α-glycerophosphate, alkylsulfonate or arylsulfonate; preferably, the alkylsulfonate is methylsulfonate or ethylsulfonate; the arylsulfonate is a benzenesulfonate or p-toluenesulfonate. Suitable inorganic salts may also be formed, including but not limited to hydrochloride, hydrobromide, hydroiodide, nitrate, bicarbonate and carbonate, sulfate or phosphate, etc.

As used herein, the term "pharmaceutically acceptable carrier and/or excipient" refers to a carrier and/or excipient that is pharmacologically and/or physiologically compatible with the subject and active ingredients, which is known in the art (seeing such as Remington's Pharmaceutical Sciences. Edited by Gennaro AR, 19th ed. Pennsylvania: Mack Publishing Company, 1995), and includes, but is not limited to: pH adjusters, surfactants, adjuvants, ionic strength enhancers, diluents, agents to maintain osmotic pressure, agents to delay absorption, preservatives.

Pharmaceutically acceptable salts can be obtained using standard procedures well known in the art, e.g., by reacting a sufficient amount of basic compound with a suitable acid providing a pharmaceutically acceptable anion.

In the present disclosure, the pharmaceutically acceptable adjuvant refers to the excipient and additive used in the production of medicaments and the formulation, refers to the substances contained in a pharmaceutical preparation whose safety has been reasonably evaluated, besides the active ingredients. In addition to shaping, acting as a carrier and improving stability, pharmaceutically acceptable adjuvants also have important functions such as solubilization, hydro trope, slow and controlled release, and are important components that may affect the quality, safety and effectiveness of drugs. According to its source, it can be divided into natural, semi-synthetic and fully synthetic. According to its functions and uses, it can be divided into: solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, fragrances, anti-adherents, antioxidants, chelating agents, penetration enhancers, pH adjusters, buffers, plasticizers, surfactants, foaming agents, defoaming agents, thickeners, inclusion agents, humectants, absorbents, diluents, flocculants and deflocculants, filter aids, release blockers, etc.; it can be divided into oral, injectable, mucosal, transdermal or topical administration, nasal or oral inhalation administration and ocular administration according to its routes of administration. The same pharmaceutical acceptable adjuvant can be used in pharmaceutical formulations with different routes of administration, and has different effects and uses.

The pharmaceutical composition can be prepared into various suitable dosage forms according to the route of administration. For example, tablets, capsules, granules, oral solutions, oral suspensions, oral emulsions, powders, tinctures, syrups, injections, suppositories, ointments, creams, pastes, ophthalmic formulation, pills, implants, aerosols, powder aerosols, sprays, etc., in which the pharmaceutical composition or suitable dosage form may contain 0.01 mg to 1000 mg of the antibody, antibody drug conjugate, a stereoisomer thereof, a prodrug thereof, a pharmaceutically acceptable salt thereof, or a pharmaceutically acceptable solvate thereof of the present disclosure, suitably 0.1 mg to 800 mg, preferably 0.5 mg to 500 mg, preferably 0.5 mg to 350 mg, and particularly preferably 1 mg to 250 mg.

The pharmaceutical composition can be administered in the form of injection, including injection solution, sterile powder for injection and concentrated solution for injection. Among them, useful vehicles and solvents include water, Ringer's solution and isotonic sodium chloride solution. In addition, sterile nonvolatile oils can also be employed as a solvent or suspending medium, such as mono- or diglycerides.

The term "treatment" as used herein generally refers to obtaining a desired pharmacological and/or physiological effect. The effect may be prophylactic in terms of complete or partial prevention of the disease or its symptoms; and/or therapeutic in terms of partial or complete stabilization or cure of the disease and/or side effects due to the disease. "Treatment" as used herein encompasses any treatment of a disease in a patient, including: (a) prevention of a diseases or a symptom in a patient susceptible to the disease or symptom but not yet diagnosed to have the disease or symptom; (b) suppression of symptoms of the disease, that is, preventing its development; or (c) alleviating symptoms of the disease, i.e., causing the disease or symptoms to regress.

In the present disclosure, the term "individual" includes human or non-human animals. Exemplary human individuals include human individuals (referred to as patients) with a disease (e.g., a disease described herein) or normal individuals. The term "non-human animal" in the present disclosure includes all vertebrates, such as non-mammals (e.g., birds, amphibians, reptiles) and mammals, such as non-human primates, livestock and/or domesticated animals (e.g., sheep, dogs, cats, cows, pigs, etc.).

In the present disclosure, the term "effective dose" refers to the amount of a compound that, after administered, alleviates one or more symptoms of the disorders being treated to some extent.

In the present disclosure, the term "ligand drug conjugate" refers to a substance in which a bioactive molecule (drug molecule) is linked to a targeted moiety. In some embodiments of the present disclosure, the bioactive molecule is linked to the targeting moiety by a linker. The linker can be broken in a specific environment (e.g., in the presence of hydrolase within the tumor and/or a low pH environment) or under a specific action (e.g., the action of lysosomal proteases), so that the bioactive molecule was separated from the target moiety. In some embodiments of the present disclosure, the linker comprises cleavable or non-cleavable units, such as peptides or disulfide linkage. In some embodiments of the present disclosure, the bioactive molecule is directly linked to the target moiety by a covalent bond that can be broken under a particular environment or action, thereby separating the bioactive molecule from the target moiety. In some embodiments of the present disclosure, the ligand drug conjugate comprises a targeting moiety, a linker, and a compound fragment of formula II of the present disclosure.

In the present disclosure, the term "ligand-drug conjugate" refers to a substance in which a bioactive molecule (drug molecule) is linked to an antibody or antigen-binding fragment thereof. In some embodiments of the present disclosure, the bioactive molecule is linked to an antibody or antigen-binding fragment thereof by a linker. The linker can be broken in a specific environment (e.g., in the presence of hydrolase within the tumor and/or a low pH environment) or under a specific action (e.g., the action of lysosomal proteases), so that the bioactive molecule was separated from the antibody or antigen-binding fragment thereof. In some embodiments of the present disclosure, the linker comprises cleavable or non-cleavable units, such as peptides or disulfide linkage. In some embodiments of the present disclosure, the bioactive molecule is directly linked to the antibody or antigen-binding fragment thereof by a covalent bond that can be broken under a particular environment or action, thereby separating the bioactive molecule from the antibody or antigen-binding fragment thereof. In some embodiments of the present disclosure, the ligand drug conjugate comprises an anti-Her3 antibody or antigen-binding fragment thereof, a linker, and a compound fragment of formula II of the present disclosure.

In the present disclosure, the term "bioactive substance", "bioactive molecule" or "drug molecule" refers to a substance that inhibits or prevents the function of cells and/or causes cell death or destruction. In some embodiments of the present disclosure, the bioactive substance, bioactive molecule or drug molecule in the conjugate is a molecule with anti-tumor biological activity. For example: radioisotopes such as At211, 1131, 1125, Y90, Re186, Re188, Sm153, Bi212, P32, Pb212and Lu; metal complexes, such as metal platinum complexes, metal gold complexes, oxaliplatin, etc.; glycopeptide antibiotics, such as bleomycin, pingyangmycin; DNA topoisomerase inhibitors, such as topoisomerase I inhibitors, camptothecin, hydroxylcamptothecin, 9-aminocamptothecin, SN-38, irinotecan, topotecan, belotecan, rubitecan, topoisomerase II inhibitor, actinomycin D, adriamycin, doxorubicin, duocarmycin, daunorubicin, mitoxantrone, podophyllotoxin, etoposide, etc.; drugs that interfere with DNA synthesis, such as methotrexate, 5-fluorouracil, cytarabine, gemcitabine, mercaptopurine, pentostatin, fludarabine, cladribine, nelarabine, etc.; drugs acting on structural proteins, such as tubulin inhibitors, vinca alkaloids, vincristine, vinblastine, paclitaxel, docetaxel, cabazitaxel, etc.; tumor signaling pathway inhibitors, such as serine/threonine kinase inhibitors, tyrosine kinase inhibitors, aspartate kinase inhibitors or histidine kinase inhibitors, etc.; also including proteasome inhibitors, histone deacetylation enzyme inhibitors, tumor angiogenesis inhibitors, cyclin inhibitors, maytansine derivatives, calicheamicin derivatives, auristatin derivatives, Pyrrolobenzodiazepines (PBD) derivatives, melphalan, mitomycin C, chlorambucil, or other active substances that inhibit tumor cell growth, promote tumor cell apoptosis and necrosis; enzymes and fragments thereof, such as nucleolysin; antibiotics; toxins, such as small molecule toxins or enzymatically active toxins originated from bacteria, fungi, plants or animals, including fragments and/or variants thereof; growth inhibitors; drug modules. The term "toxin" refers to a substance capable of producing deleterious effects on the growth or proliferation of cells.

In the present disclosure, the term "small molecule" refers to a bioactive small molecule drug. The term small molecule toxin has cell-damaging activity, i.e., a state of causing pathological changes of cells in some forms, and cell damage is not limited to direct damage, including all kinds of damage to the structure and function of cells, such as DNA cleavage, base-dimer formation, chromosome cleavage, damage to cell division mechanism and reduction of various enzymatic activities.

In the present disclosure, the term "linker" refers to a fragment that links a bioactive molecule (drug molecule) to a targeting moiety.

In the present disclosure, the term "targeting moiety" refers to a moiety in a conjugate that is capable of specifically binding to a target (or a portion of a target) on the cell surface. Through the interaction of the targeting moiety with the target, the conjugate can be delivered to a specific population of cells.

In the present disclosure, when the targeting moiety in a conjugate is an antibody, the conjugate may be referred to as a "drug-antibody conjugate".

In the present disclosure, the antibody or antigen-binding fragment thereof includes a derivatized antibody or antigen-binding fragment thereof, such as an antibody or antigen-binding fragment thereof having a thiol group, wherein the derivatization allows the antibody to have a group or ability reactive with a drug-linker conjugate. The thiol -SH can be derivatized by breaking the disulfide bond (e.g., by reduction with the reducing agent TCEP).

As used in the present disclosure and the appended claims, singular forms such as "a" and "the" include plural meanings, unless the context clearly indicates otherwise. Therefore, the meaning of the singular word "a" includes "one or more".

As used herein, the terms "cancer" and "tumor" are used with the same meaning.

The term "gene" used herein includes not only DNA but also its mRNA, its cDNA and its cRNA.

The term "polynucleotide" as used herein is used in the same sense as nucleic acid and also includes DNA, RNA, probes, oligonucleotides and primers.

The term "Tb" as used herein is an abbreviation for "target binding" and includes antibodies or any molecule that binds to a target, and the term "Ab" is an abbreviation for "antibody" and is used interchangeably with "antibody".

As used herein, the terms "polypeptide" and "protein" are used interchangeably.

The term "cell" as used herein also includes cells within an individual animal and cultured cells.

"An antibody or antigen-binding fragment thereof binding to Her3", as used in the present disclosure, refers to antibody or antigen-binding fragment thereof specifically binding to Her3, such as human Her3.

"Her3", as used herein, also known as "ErbB3", is a transmembrane receptor tyrosine kinase molecule in the ERBB family. HER3 gene is located on the long arm of Chromosome 12 (12q132) and encodes a 180 kDa protein. The extracellular domain of HER3 is divided into four sub domains (domains 1-4): domain1 and domain3 are leucine-rich beta-helical regions responsible for ligand binding; domain 2 and domain 4 are cysteine-rich regions. In addition, domain 2 also contains a dimer arm necessary for interaction with other receptors. The transmembrane domain is followed by the intracellular domain, including a flexible Juxta membrane region, a kinase structure, and a C-terminal tail. In the absence of ligands, the binding between domain 2 and domain 4 renders HER3 inactive. Once bound to the ligand, the other half of the kinase structure of the heterodimer phosphorylates the tyrosine residue on the C-terminal tail of HER3.

The Her3 to which the present disclosure relates may be conventional Her3 in the art (e.g., human Her3), e.g., soluble Her3, Her3 in membrane form, etc., and also represents Her3 variant 1 and/or Her3 variant 2. In some embodiments, the human Her3 is shown in accession number Uniport ID: P21860-1 or NCBI:NP_001973.2. In some embodiments, the human Her3 comprises an amino acid sequence set forth in SEQ ID NO:31.

Her3(1-643) set forth in SEQ ID No.31 is originated from Uniport ID: P21860-1, wherein SEQ ID No.31 is show as follows:

MRANDALQVLGLLFSLARGSEVGNSQAVCPGTLNGLSVTGDAENQYQTLYKLYERCEVVMGN
LEIVLTGHNADLSFLQWIREVTGYVLVAMNEFSTLPLPNLRVVRGTQVYDGKFAIFVMLNYNT
NSSHALRQLRLTQLTEILSGGVYIEKNDKLCHMDTIDWRDIVRDRDAEIVVKDNGRSCPPCHEV
CKGRCWGPGSEDCQTLTKTICAPQCNGHCFGPNPNQCCHDECAGGCSGPQDTDCFACRHFND
SGACVPRCPQPLVYNKLTFQLEPNPHTKYQYGGVCVASCPHNFVVDQTSCVRACPPDKMEVD
KNGLKMCEPCGGLCPKACEGTGSGSRFQTVDSSNIDGFVNCTKILGNLDFLITGLNGDPWHKIP
ALDPEKLNVFRTVREITGYLNIQSWPPHMHNFSVFSNLTTIGGRSLYNRGFSLLIMKNLNVTSLG

FRSLKEISAGRIYISANRQLCYHHSLNWTKVLRGPTEERLDIKHNRPRRDCVAEGKVCDPLCSS
GGCWGPGPGQCLSCRNYSRGGVCVTHCNFLNGEPREFAHEAECFSCHPECQPMEGTATCNGSG
SDTCAQCAHFRDGPHCVSSCPHGVLGAKGPIYKYPDVQNECRPCHENCTQGCKGPELQDCLG
QTLVLIGKTHLT.

KD refers to the dissociation constant obtained from the ratio (or Kd/Ka, expressed in molar concentration (M)) of Kd (dissociation rate of specific binding molecule-target protein interaction) to Ka (binding rate of specific binding molecule-target protein interaction). KD values can be determined using methods well established in the art. The preferred method for determining the KD of binding molecules is by using surface plasmon resonance, for example a biosensor system, such as the Biacore TM (GE Healthcare Life Sciences) system.

The term "antigen-binding fragment" as used herein refers to a partial fragment of an antibody having antigen-binding activity, wherein the fragment has full or partial functionality of the antibody, including, but not limited to, Fab, Fab', F(ab')2, Fv such as single-chain Fv (scFv), disulfide-linked Fv (sdFv), or di-scFv, etc. The term also includes Fab', which is a monovalent fragment of the variable region of an antibody obtained by treating F(ab')2 under reducing conditions. However, the term is not limited to these molecules as long as the fragment has a binding affinity for the antigen. Furthermore, these functional fragments include not only fragments obtained by treating full-length molecules of antibody proteins with appropriate enzymes, but also proteins produced in appropriate host cells using genetically modified antibody genes.

"Antibody molecule" or "antibody" as used herein refers in its broadest interpretation to immunoglobulin molecules and immunologically active portions of immunoglobulin molecules, i.e., molecules that contain an antigen-binding site allowing to immunospecifically bind to an antigen. Thus, the term antibody broadly encompasses not only a whole antibody molecule, but also fragments of said antibody as well as variants (including derivatives) of said antibody and antibody fragments. When "antibody molecule" or "antibody" is used in the same context as antigen-binding fragment, "antibody molecule" or "antibody" refers to an intact antibody molecule or a full-length antibody.

The term "single chain Fv" or "scFv" refers to a polypeptide comprising the VL domain of an antibody linked to the VH domain of the antibody. For example, antibodies that immunospecifically bind to Her3 can cross-react with other antigens, and preferably, can not cross-react with other antigens. Antibodies that immunospecifically bind to Her3 can be identified, for example, by immunoassays or other methods known to those skilled in the art. "Intact" antibody or "full-length" antibody refers to a protein comprising two heavy chains (H) and two light chains (L) linked to each other by disulfide bonds, and the protein comprises: (1) for the heavy chain, a variable region (abbreviated herein as "VH") and a constant region of the heavy chain comprising three domains CH1, CH2, CH3; and (2) for the light chain, a variable region (abbreviated herein as "VL") of the light chain and a constant region of the light chain comprising a domain CL. Antibodies of the present disclosure include, but are not limited to, monoclonal, multi-specific, human or chimeric antibodies, single chain antibodies, Fab fragments, F(ab') fragments, anti-idiotypic (anti-Id) antibodies (including, for example, anti-Id antibody of an antibody of the present disclosure), and epitope-binding fragments of any of the aforementioned antibodies. The immunoglobulin molecules of the present disclosure can be any type (e.g., IgG, IgE, IgM, IgD, IgA and IgY), any class (e.g., IgGl, IgG2, IgG3, IgG4, IgAl, and IgA2), or any subclass of immunoglobulins. Preferably, the antibodies of the present disclosure comprise or consist of a VH domain, a VH CDR, a VL domain, or a VL CDR having any of the amino acid sequences or fragments or variants thereof described in the Table 1.

The term "Fab" as used herein denotes a monovalent fragment of a variable region of an antibody obtained by treating F(ab')2 under reducing conditions as described above. However, Fab' of the present disclosure also includes Fab' produced using genetically modified antibody genes.

As used herein, the term "scFv" refers to a single polypeptide chain comprising VL and VH domains, wherein the VL and VH are linked by a linker or directly (see, e.g., Bird et al., Science 242: 423-426 (1988); Huston et al., Proc. Natl. Acad. Sci. USA 85: 5879-5883 (1988); and Pluckthun, The Pharmacology of Monoclonal Antibodies, Vol. 113, Eds. Roseburg and Moore, Springer-Verlag, New York, pp. 269-315 (1994)). Such scFv molecules can have the general structure: NH2-VL-linker-VH-COOH or NH2-VH-linker-VL-COOH. Suitable linkers in prior art consist of repeated GGGGS amino acid sequences or variants thereof. For example, a linker with the amino acid sequence (GGGGS)4 can be used, and variants thereof can also be used (Holliger et al. (1993), Proc. Natl. Acad. Sci. USA 90: 6444-6448). Other linkers that may be used in the present disclosure were described in Alfthan et al. (1995), Protein Eng. 8: 725-731, Choi et al. (2001), Eur. J. Immunol. 31: 94-106, Hu et al (1996), Cancer Res. 56: 3055-3061, Kipriyanov et al (1999), J. Mol. Biol. 293: 41-56 and Roovers et al (2001), Cancer Immunol. In some cases, disulfide bonds may also exist between VH and VL of scFv. As used herein, the term "di-scFv" refers to an antibody fragment formed by linking two scFv.

The term "Her3 variant" refers to a polypeptide having similar or identical functions to a HER3 polypeptide, HER3 fragment, anti-HER3 antibody or antibody fragment thereof, but not necessarily comprising the amino acid sequences of similar or identical HER3 polypeptide, HER3 fragment, anti-HER3 antibodies or fragments thereof, or the structures of similar or identical HER3 polypeptides, HER3 fragments, anti-Her3 antibodies or fragments thereof.

As used herein, even if there are variants, as well as amino acid substitutions, deletions, or additions in the antibody or antigen-binding fragment thereof, it still has the activity of binding to Her3.

As used herein, the percentage of homology between two amino acid sequences is equal to the percentage of the identity between the two sequences. The percentage of sequence identity between two sequences is a function of the number of identical positions shared by the sequences (i.e., % homology = number of identical positions/total number of positions X 100), taking into account the number of gaps and the length of each gap, which needs to be introduced for optimal alignment of the two sequences. Sequence comparison and determination of the percentage of identity between sequences can be carried out by methods generally known in the art, and such sequence comparison and determination of the percentage of identity can be realized by mathematical algorithms. For example, the algorithm in Meyers and Miller, 1988, Comput. Appl. Biosci. 4: 11-17 (which has been integrated into the ALIGN program (version 2.0)) can be used to determine the percentage of identity between amino acid sequences and/or between nucleotide sequences. In addition, the GAP program in the GCG package obtained online from Accelrys (using its default parameters) can be used to determine the percentage of identity between amino acid sequences or between nucleotide sequences. In one embodiment, the two sequences are of equal length.

The term "epitope" refers to a portion of Her3 that has antigenic or immunogenic activity in an animal, preferably a mammal. Epitope with immunogenic activity is a portion of Her3, that triggers antibody responses in animals. The epitope with antigenicity activity is a portion of Her3, to which the antibody immunospecifically binds, that can be determined by methods known in the art. The antigenicity epitope is not necessarily immunogenic.

The epitope of the antibody or antigen-binding fragment thereof of the present disclosure can be determined by existing techniques, such as synthetic peptide method, immune informatics prediction, determination of polypeptide activity, epitope peptide scanning method, phage display technology, X-ray diffraction and nuclear magnetic resonance analysis, homologous antibody modeling protein docking prediction method. The antibody or antigen-binding fragment thereof of the present disclosure has an epitope that binds to Her3 domain 3.

The antibody or antigen-binding fragment thereof of the present disclosure includes antibodies that bind to the same epitope. The phrase "antibodies that bind to the same epitope" as used herein refers to different antibodies that bind to a common epitope. If the second antibody binds to a part of the peptide or part of the tertiary structure to which the first antibody binds, it can be determined that the first antibody and the second antibody bind to the same epitope.

"Epitope 202-2-1" or "202-2-1 epitope" is the region in the extracellular domain of HER3 that binds to antibody 202-2-1. This epitope is close to the transmembrane domain of HER3 and within domain 3 of HER3. The "epitope 202-2-1" is composed of Histidine at position 466, Tryptophan at position 470, Threonine at position 471, Threonine at position 478, (Aspartic acid-Isoleucine-lysine-Histidine-Asparagine) at positions 483 to 487, Isoleucine at position 484, Arginine at position 490 and Arginine at position 491 of Her3 set forth in SEQ ID No.31.

Herein, the CDRs comprised by the antibodies or antigen-binding fragments thereof of the present disclosure can be identified by various numbering systems known in the art. In certain embodiments, the CDRs comprised in the antibody or antigen-binding fragment thereof of the present disclosure are preferably determined by the Kabat, Chothia, AbM or IMGT numbering systems.

As used herein, the terms "framework region" or "FR" residues refer to those amino acid residues in the variable region of the antibody, except for the CDR residues defined above.

As used herein, the term "germline antibody gene" is a gene encoding an immunoglobulin expressed by non-lymphocytes, which has not undergone the maturation process leading to the genetic rearrangement and maturation of the specific immunoglobulin expressed. One advantage provided by various embodiments of the present disclosure lies in the recognition that the amino acid sequences encoded by germline antibody genes retain more characteristic important amino acid sequence structures of individuals of animal species than that encoded by mature antibody genes. Thus, when applied therapeutically to the species, it is less likely to be recognized as a foreign substance by the species.

As for amino acid substitutions, conservative amino acid substitutions are preferred in this specification. Conservative amino acid substitutions refer to substitutions that occur within a set of amino acids associated with amino acid side chains. Preferred sets of amino acids are as follows: acidic sets (aspartic acid and glutamic acid); basic sets (lysine, arginine and histidine); non-polar sets (alanine, valine, leucine, isoleucine, proline, phenylalanine, methionine, and tryptophan); and the uncharged polar families (glycine, asparagine, glutamine, cysteine, serine, threonine and tyrosine). More preferred sets of amino acids are as follows: aliphatic hydroxyls (serine and threonine); amide-containing sets (asparagine and glutamine); aliphatic sets (alanine, valine, leucine and isoleucine); and aromatic sets (phenylalanine, tryptophan, and tyrosine). Such amino acid substitutions are preferably made within a set that does not impair the properties of the substance having the original amino acid sequence.

In addition, it is known that the lysine residue at the carboxyl end of the heavy chain of the antibody produced in cultured mammalian cells is deleted (Journal of Chromatography A, 705: 129-134 (1995)), as well as it is also known that the two amino acid residues (glycine and lysine) at the carboxyl end of the heavy chain of the antibody produced in cultured mammalian cells are deleted, and the new proline residue at the carboxyl terminus is amidated (Analytical Biochemistry, 360: 75-83 (2007)). However, such deletions and modifications of the heavy chain sequence do not affect the antigen binding affinity and effector function of the antibody (complement activation, antibody-dependent cell cytotoxicity, etc.).

The IgG isotype control antibodies of the present disclosure are fully known to one of ordinary skill in the art and can be purchased or prepared. For example, the sequence of human anti-Hen Egg Lysozyme IgG (anti-HEL, such as human IgG1, abbreviated as hIgG1) are derived from the variable region of Fab F10.6.6 sequence in "Affinity maturation increases the stability and plasticity of the Fv domain of anti-protein antibodies" published by Acierno et al. (Aciemo et al. J Mol Biol. 2007; 374(1): 130-46). Anti-hel-hIgG I, a common hIgG1 antibody against Hen Egg Lysozyme, can be purchased from Baiyin Biological, Catalog number: B117901. It can also be prepared by oneself. The preparation method is as follows: the amino acid codon optimization and gene synthesis of the heavy and light chain (full sequence or variable region) gene of human IgG antibody are performed by Nanjing GenScript Biotech Corp., according to the standard technology introduced in the "Molecular Cloning, A Laboratory Manual (3rd Edition)". The heavy and light chain genes were respectively subcloned into antibody heavy chain expression vector and antibody light chain expression vector of mammalian expression system by standard molecular cloning techniques such as PCR, enzyme digestion, DNA gel recovering, ligation transformation, colony PCR or enzyme digestion identification, and the heavy and light chain genes of the recombinant expression vector were further sequenced. After the sequence was verified correctly by sequencing, a large number of expression plasmids of endotoxin-free grade were prepared and then the weight and light chain expression plasmids were transiently cotransfected into HEK293 cells for recombinant antibody expression. After 7 days of culture, the cell culture was collected and purified by rProtein A affinity column (GE). The quality of the harvested antibody samples was determined by SDS-PAGE and SEC-HPLC standard analytical techniques.

The twenty conventional amino acids referred to herein have been written following conventional usage. See, e.g., Immunology-A Synthesis (2nd Edition, E. S. Golub and D. R. Gren, Eds., Sinauer Associates, Sunderland, Mass. (1991)), which is incorporated herein by reference. As used herein, the terms "polypeptide" and "protein" have the same meaning and are used interchangeably. And in the present disclosure, amino acids are generally represented by one-letter and three-letter abbreviations well known in the art. For example, alanine can be represented by A or Ala; arginine can be represented by R or Arg; glycine can be represented by G or Gly; glutamine can be represented by Q or Gln.

As used herein, the term "pharmaceutically acceptable carrier and/or excipient" refers to a carrier and/or excipient that is pharmacologically and/or physiologically compatible with the subject and active ingredients, which is known in the art (vide such as Remington's Pharmaceutical Sciences. Edited by Gennaro AR, 19th ed. Pennsylvania: Mack Publishing Company, 1995), and includes, but is not limited to: pH adjusters, surfactants, adjuvants, ionic strength enhancers, diluents, agents to maintain osmotic pressure, agents to delay absorption, preservatives.

As used herein, the term "prevention" refers to a method performed to prevent or delay the occurrence of a disease or disorder or symptom (e.g., tumors and infectious diseases) in a subject. As used herein, the term "treatment" refers to a method performed to obtain a beneficial or desired clinical result. For the purposes of the present disclosure, a beneficial or desired clinical result includes, but is not limited to, alleviation of symptoms, reduction in the extent of the disease, stabilization (i.e., not worsening) of the disease state, delaying or slowing the progression of the disease, amelioration or alleviation of the disease status, and relief of symptoms (whether in part or in full), whether detectable or undetectable. In addition, "treatment" can also mean prolonging survival compared with expected survival (if not receiving treatment).

As used herein, the term "subject" refers to a mammal, e.g., a primate mammal, such as a non-human primate mammal or a human. In certain embodiments, the subject (e.g., human) has tumors and infectious diseases, or is at risk of having such diseases.

As used herein, the term "effective amount" refers to an amount sufficient to achieve, or at least partially achieve, a desired effect. For example, an effective amount to prevent diseases (e.g., tumors and infectious diseases) is an amount sufficient to prevent, arrest, or delay the occurrence of diseases (e.g., tumors and infectious diseases); an effective amount to treat diseases is an amount sufficient to cure or at least partially arrest the diseases and their complications in patients who already have them. The determination of such an effective amount is entirely within the capabilities of those skilled in the art. For example, the amount effective for therapeutic use will depend on the severity of the disease to be treated, the general state of the patient's own immune system, the general conditions of the patient such as age, weight and sex, the way in which the drug is administered, and other treatments administered simultaneously, etc.

As used herein, the term "effector function" refers to those biological activities attributable to an Fc region of an antibody (either a native sequence Fc region or an amino acid sequence variant Fc region), which varies with the antibody isotypes.

The term "pharmaceutically acceptable" means that the molecule itself, molecular fragment, or composition does not produce detrimental, allergic or other adverse reactions when suitably administered to an animal or human. Specific examples of some substances that may be pharmaceutically acceptable carriers or components thereof include sugars (e.g., lactose), starch, cellulose and derivatives thereof, vegetable oils, gelatin, polyols (e.g., propylene glycol), alginic acid, and the like.

The *in vivo* therapeutic effect of antibodies and antibody-drug conjugates on cancers can be determined using experimental animals, e.g., administering the antibody to nude mice implanted with a Her3-expressing tumor cell line, and measuring any changes in cancer cells.

Examples of cancer types include lung cancer, colorectal cancer, etc.

Herein, the term "direct bond" means that the groups on both sides thereof are directly connected, such as in a compound represented by formula II 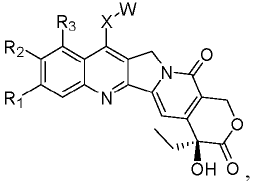 if X is a direct bond, its structural formula is

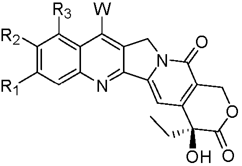

Other direct bonds can be understood with reference to the foregoing content.

As used herein, the term "absent" means that the group is not present, such as in a compound represented by formula II 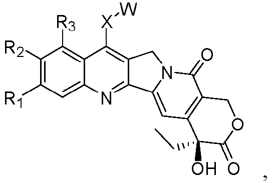 if W is absent, its structural formula is

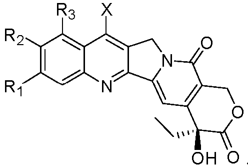

In a compound represented by formula II 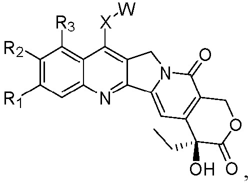 R₁ and R₂ together with the carbons atom to which they are both attached form 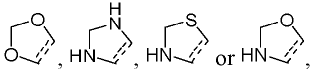 the "dotted line" indicates where the heterocyclic ring is fused to the benzene ring, such as forming

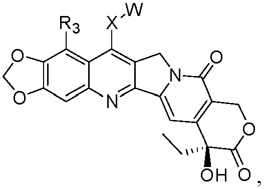

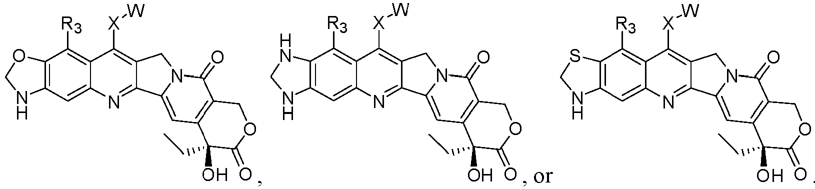

Herein, X is defined as, for example, "X is selected from optionally substituted 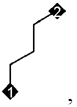 and the substituent is selected from 1 or 2 C1-4 alkyl groups (such as methyl)", then X can be, for example,

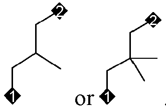

Other similar definitions of X can be understood with reference to the foregoing content.

Herein, X is defined as, for example, "X is selected from optionally substituted 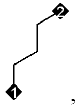 and the substituent is selected from 2 C1-4 alkyl groups (such as methyl) and which together with the carbon atom to which they are both attached form a C3-6 cycloalkyl (e.g., cyclopropyl)", then X can be, for example,

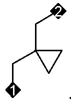

Other similar definitions of X can be understood with reference to the foregoing content.

In the structure of amino acid residue represented by AA¹ 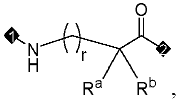 if r is 0, it will be understood by those skilled in the art that the structure of the amino acid residue represented by AA¹ will become

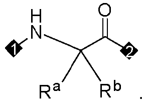

In the structure of amino acid residue represented by AA¹ 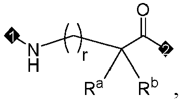 if R^{a} and R^{b} together with the carbon atom to which they are both attached form 4-10-membered heterocycle, and the 4-10-membered heterocycle is optionally substituted with one or more R⁰, wherein the term "the 4-10-membered heterocycle is optionally substituted with one or more R⁰" means that the 4-10-membered heterocycle may be unsubstituted or substituted with one or more R⁰, and in the case of more R⁰, the definition of each R⁰ may be the same or different. Other similar definitions can be understood with reference to the foregoing content.

Herein, for example, when L₃ is selected from Lys, Val-Cit, Ala-Ala-Asn, Ala-Ala-Asp, Gly-Gly-Phe-Gly, Val-Lys-Gly, Val-Ala, Lys-Ala-Asn, "the distal amino group of the lysine (Lys) is optionally substituted with 1, 2 or 3 substituents selected from tert-butoxycarbonyl, C1-6 alkyl (preferably methyl), O" means that the distal amino group of Lys in each option of L₃ is optionally substituted with 1, 2 or 3 subsituents selected from tert-butoxycarbonyl, C1-6 alkyl (preferably methyl), O. Wherein "the distal amino group of Lys" refers to the naked amino group -NH₂ in the lysine residue

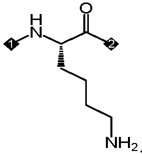

"The distal amino group of the lysine (Lys) is optionally substituted with 1, 2 or 3 substituents selected from tert-butoxycarbonyl, C1-6 alkyl (preferably methyl), O" indicates that the distal amino group of the lysine (Lys) may not be substituted, or may be substituted by 1, 2 or 3 substituents selected from tert-butoxycarbonyl, C1-6 alkyl (preferably methyl), O. For example, it can be substituted by one tert-butoxycarbonyl group, that is, it becomes 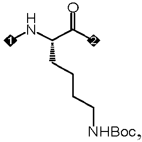 or it can be substituted by two methyls, that is, it becomes 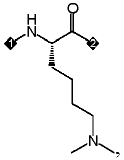 or it can be substituted by two methyls and one O, that is, it becomes

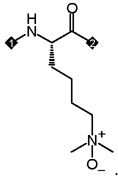

It should be noted that "the distal amino group of the lysine (Lys) is substituted with O" means that the distal amino group of the lysine (Lys) is substituted with oxo, that is, it becomes 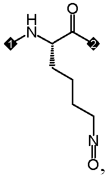 and if the distal amino group is further substituted with two methyl groups, it becomes

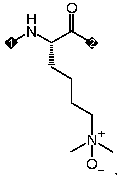

In various parts of this specification, substituents of compounds in the present disclosure are disclosed according to group types or scopes. Specifically, the present disclosure includes each individual subcombination of each member of these group types or scopes. For example, the term "C1-6 alkyl" particularly refers to methyl, ethyl, C3 alkyl, C4 alkyl, C5 alkyl and C6 alkyl as disclosed independently.

As used herein, the term "C1-6 alkyl" refers to a straight or branched chain alkyl group containing 1-6 carbon atoms, including, for example, "C1-3 alkyl" or "C1-4 alkyl", methyl, ethyl, etc., and specific examples include but are not limited to: methyl, ethyl, n-propyl, isopropyl, n-butyl, isobutyl, sec-butyl, tert-butyl, pentyl, hexyl.

As used herein, the term "C₁₋₄ alkyl" refers to straight or branched chain alkyl groups containing 1-4 carbon atoms, including, for example, "C₁₋₃ alkyl", methyl, ethyl, etc. Specific examples include but are not limited to: methyl, ethyl, n-propyl, isopropyl, n-butyl, isobutyl, sec-butyl, tert-butyl.

As used herein, the term "C₂₋₆ alkenyl" refers to a straight-chain, branched-chain or cyclic alkenyl group containing at least one double bond and 2-6 carbon atoms, including, for example, "C₂₋₄ alkenyl" and the like. Examples include, but are not limited to: vinyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, cyclopentenyl, 1,3-cyclopentadienyl, cyclohexenyl, 1,4-cyclohexadienyl and the like.

As used herein, the term "C₂₋₆ alkynyl" refers to a straight or branched alkynyl group containing at least one triple bond and 2-6 carbon atoms, including, for example, "C₂₋₄ alkynyl" and the like. Examples include, but are not limited to: ethynyl, propynyl, 2-butynyl, 2-pentynyl, 3-pentynyl, 4-methyl-2-pentynyl, 2-hexynyl, 3-hexynyl, 5-methyl-2-hexynyl, etc.

As used herein, the term "halogen" includes fluorine, chlorine, bromine, and iodine.

As used herein, the term "3-6-membered cycloalkyl" or "C₃₋₆ cycloalkyl" refers to a saturated cyclic alkyl containing 3-6 carbon atoms, including cyclopropanyl (i.e., cyclopropyl), cyclobutanyl (i.e., cyclobutyl), cyclopentanyl (i.e. cyclopentyl), cyclohexyl.

As used herein, the term "3-7-membered carbocycloalkyl" or "C₃₋₇ cycloalkyl" refers to a saturated cyclic alkyl containing 3-7 carbon atoms, including cyclopropyl, cyclobutyl, cyclopentyl, cyclohexyl, cycloheptyl.

As used herein, the term "C₁₋₆ alkoxy" refers to an alkyl group as defined above which is attached to the parent molecular moiety by an oxygen atom. Specific examples include, but are not limited to, methoxy, ethoxy, propoxy, isopropoxy, n-propoxy, isopropoxy, n-butoxy, isobutoxy, tert-butoxy, pentoxy, hexyloxy, etc.

As used herein, the term "C₁₋₄ alkoxy" refers to an alkyl group as defined above which is attached to the parent molecular moiety by an oxygen atom. Specific examples include, but are not limited to, methoxy, ethoxy, propoxy, isopropoxy, n-propoxy, isopropoxy, n-butoxy, isobutoxy, tert-butoxy, etc.

As used herein, the term "4-10-membered heterocyclic group" refers to a cyclic group containing 4 to 10 ring atoms (wherein at least one ring atom is a heteroatom, such as nitrogen, oxygen, or sulfur atom). The term "4-6 membered heterocyclic group" refers to a cyclic group containing 4-6 ring atoms (wherein at least one ring atom is a heteroatom, such as nitrogen, oxygen, or sulfur atom). Optionally, ring atoms (e.g., carbon, nitrogen, or sulfur atoms) in the ring structure may be substituted by oxygen. "4-8-membered heterocyclic groups" include, for example, "4-8-membered nitrogen-containing heterocyclic group", "4-8-membered oxygen-containing heterocyclic group", "4-7-membered heterocyclic group", "4-7-membered oxygen-containing heterocyclic group", "4-7-membered heterocyclic group", "4-6-membered heterocyclic group", "5-7-membered heterocyclic group", "5-6-membered heterocyclic group", "5-6-membered nitrogen-containing heterocyclic group", including, but not limited to, oxocyclobutanyl, pyrrolidinyl, tetrahydrofuryl, piperidinyl, piperazinyl, tetrahydropyranyl, homopiperazinyl, etc.

As used herein, the term "4-10-membered heterocycle" refers to a ring containing 4-10 ring atoms (wherein at least one ring atom is a heteroatom such as nitrogen, oxygen or sulfur atom). The term "5-6 membered heterocycle" refers to a ring containing 5-6 ring atoms (wherein at least one ring atom is a heteroatom such as nitrogen, oxygen or sulfur atom), including but not limited to pyrrolidine, tetrahydrofuran, piperidine, piperazine, tetrahydropyran, etc.

As used herein, the term "aryl" refers to a monocyclic or polycyclic hydrocarbon group having aromaticity, such as 6-10-membered aryl, 5-8-membered aryl, and the like. Specific examples include, but are not limited to, phenyl, naphthyl, anthracenyl, phenanthryl, and the like. The "6-10-membered aryl" refers to an aryl having 6-10 ring atoms. The "C6-10 aryl" refers to an aryl group containing 6-10 carbon atoms.

As used herein, the term "heteroaryl" refers to a cyclic group having aromaticity, in which at least one ring atom is a heteroatom, such as nitrogen, oxygen or sulfur atom. Optionally, ring atoms (e.g., carbon, nitrogen, or sulfur atoms) in the ring structure may be substituted by oxygen. Specific examples include, but are not limited to, 5-10 membered heteroaryl, 5-6 membered heteroaryl, 5-10 membered nitrogen-containing heteroaryl, 6-10 membered oxygen-containing heteroaryl, 6-8 membered nitrogen-containing heteroaryl, 5-8 membered oxygen-containing heteroaryl, etc., such as furyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, 2-pyridonyl, 4-pyridonyl, pyrimidinyl, 1,4-dioxinyl, 2*H*-1,2-oxazinyl, 4*H*-1,2-oxazinyl, 6*H*-1,2-oxazinyl, 4H-1,3-oxazinyl, 6*H*-1,3-oxazinyl, 4*H*-1,4-oxazinyl, pyridazinyl, pyrazinyl, 1,2,3-triazinyl, 1,3,5-triazinyl, 1,2,4,5-tetrazinyl, azacycloheptatrienyl, 1,3-diazacycloheptatrienyl, azacyclooctatetraenyl, etc.

A bond in a structural formula represented herein by a wavy line "~~" is intended to indicate that the structure represents cis or trans isomer, or a mixture of cis and trans isomers in any ratio.

Herein, "between" includes the end values of the interval, such as between 1 and 16, which can refer to 1 or 16.

The term "the ratio of drug to antibody" or "DAR" refers to the amount of drug, e.g., the average ratio of a small molecule toxin attached to the antibody of the ADC to the antibody. The DAR of an ADC can range from 1 to 16, but depends on the number of attachment sites on the antibody, thus higher loads (e.g., 20) are also possible. The term DAR can be used when referring to the amount of drug loaded onto a single antibody, or alternatively, when referring to an average or mean DAR of a group of ADCs. DAR can also be calculated as the average DAR of a molecular population in the product, which is the overall ratio (molar ratio) of the drug portion (D) coupled to the Ab portion in the product to the Ab portion measured by detection methods (such as conventional methods e.g., mass spectrometry, ELISA, electrophoresis, and/or HPLC).

On the basis of common knowledge in the field, the above preferred conditions can be optionally combined to obtain the preferred examples of the present disclosure. The reagents and raw materials used in the present disclosure are commercially available.

The beneficial effects of the present disclosure are as follows:
Compared with the prior art, the antibodies involved in the present invention are fully human antibodies, which can be safely administered to human subjects without eliciting immunogenic reactions. The antibodies involved in the present invention have extremely high affinity and specificity, which can be beneficial for exerting anti-tumor effects. Unexpectedly, the antibody-drug conjugate of the present disclosure has significant *in vivo* anti-tumor activity.

### Abbreviations

| | | | |
|---|---|---|---|
| CDR | Complementarity determining regions in immunoglobulin variable regions | HRP | Horseradish peroxidase |
| FR | Antibody framework region: amino acid residues other than CDR residues in the variable region of an antibody | hFc | Fc fragment of Human IgG antibody |
| VH | Heavy chain variable region of antibody | KD | Equilibrium dissociation constant |
| VL | Light chain variable region of antibody | HCDR1 | Complementarity determining region 1 in the heavy chain variable region of an immunoglobulin |
| AbM | The definition of AbM CDR is originated from Martin's related research (Martin ACR, Cheetham JC, Rees AR (1989) Modelling antibody hypervariable loops: A combined algorithm. Proc Natl Acad Sci USA 86: 9268-9272), and this definition method integrates some of the definitions of Kabat and Chothia. | HCDR2 | Complementarity determining region 2 in the heavy chain variable region of an immunoglobulin |
| Kabat | Immunoglobulin alignment and numbering system by Elvin A. Kabat (see, such as Kabat et al., Sequences of Proteins of Immunological Interest, 5th Ed. Public Health Service, National Institutes of Health, Bethesda, Md., 1991). | HCDR3 | Complementarity determining region 3 in the heavy chain variable region of an immunoglobulin |
| Chothia | The immunoglobulin numbering system proposed by Chothia et al., which is a classical rule for identifying CDR region boundaries based on the position of structural loop regions (see, such as Chothia & Lesk (1987) J. Mol. Biol. 196:901-917; Chothia et al., (1989) Nature 342:878-883). | LCDR1 | Complementarity determining region 1 in the light chain variable region of an immunoglobulin |
| IMGT | Based on the numbering system of the International ImMunoGeneTics information system ^{®} (IMGT) sponsored by Lefranc et al, see Lefranc et al., Dev. Comparat. Immunol. 27:55-77, 2003. | LCDR2 | Complementarity determining region 2 in the light chain variable region of an immunoglobulin |
| mAb | Monoclonal antibodies | LCDR3 | Complementarity determining region 3 in the light chain variable region of an immunoglobulin |
| EC50 | Concentration at which 50% efficacy or binding is produced | SEC-HPLC | Size exclusion high performance liquid chromatography |
| ELISA | Enzyme linked immunosorbent assay | HC | Heavy chain of immunoglobulin |
| PCR | polymerase chain reaction | LC | Light chain of immunoglobulin |
| OMs | Methanesulfonate | FA | Formic acid |
| OTs | p-toluenesulfonate | ACN | Acetonitrile |
| OTf | Trifluoromethanesulfonate | CCK8 reagent | 2-(2-methoxy-4-nitrophenyl)-3-(4-nitrophenyl)-5-(2,4-disulfophenyl)-2H-tetrazolium monosodium salt |
| TBS | tert-butyldimethylsilyl | FBS | fetal bovine serum |
| MMT | p-methoxyphenyldiphenylmethyl | DMSO | dimethyl sulfoxide |
| PB/PBS | Phosphate buffer | HBTU | O-benzotriazolyl-tetramethyluronium hexafluorophosphate |
| HOBT | 1-Hydroxybenzotriazole | HATU | 2-(7-aza-benzotriazolyl)-N,N,N',N'-tetramethyl uronium hexafluorophosphate |
| NBS | N-Bromosuccinimide | TFA | Trifluoroacetic acid |
| PPTS | pyridinium p-toluenesulfonate | DCM | dichloromethane |
| THF | Tetrahydrofuran | DMF | N,N-Dimethylformamide |
| EDC | 1-(3-Dimethylaminopropyl)-3-ethyl carbodiimide hydrochloride | IgG | Immunoglobulin |

### Description of Figures

Figure 1A. Assay for binding of anti-Her3 antibody to human Her3-his protein by ELISA.
Figure 1B. Assay for binding of anti-Her3 antibody to monkey Her3-his protein by ELISA.
Figure 1C. Assay for binding of anti-Her3 antibody to rat Her3-his protein by ELISA.
Figure 2A. Assay for binding of anti-Her3 antibody to A549-human Her3 cells by flow cytometry.
Figure 2B. Assay for binding of anti-Her3 antibody 202-2-1 to T47D cells by flow cytometry.
Figure 2C. Assay for binding of anti-Her3 antibody 202-2-1 to MCF-7 cells by flow cytometry.
Figure 3. Inhibition of anti-Her3 antibody on the phosphorylation of AKT by NRG1 in tumor cells.
Figure 4. Assay for endocytosis of tumor cells A549-human Her3 cells for anti-Her3 antibody.
Figure 5A. Assay for anti-Her3 antibody competing with the biotinylation control antibody 2 for binding to human Her3 protein.
Figure 5B-1. The complex of Fab of antibody 202-2-1 and Her3 shows binding, a complex has formed.

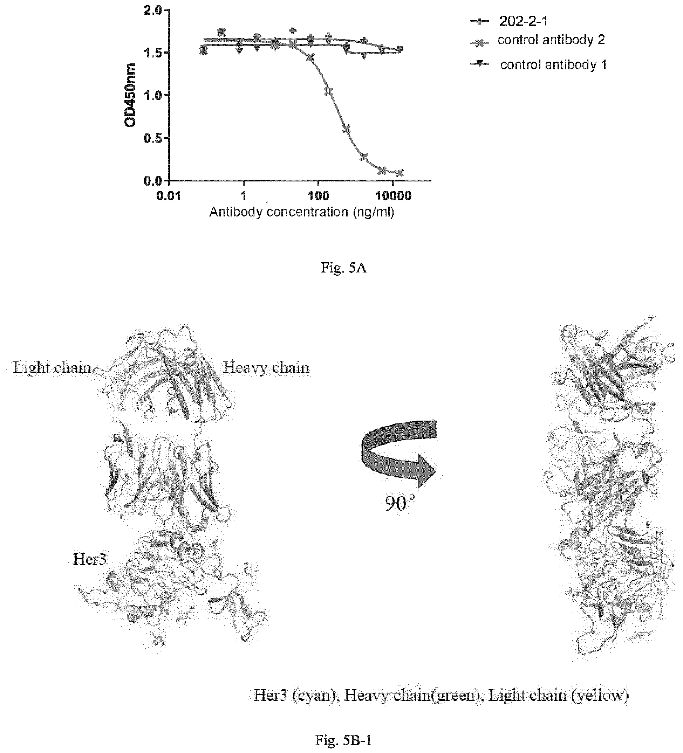

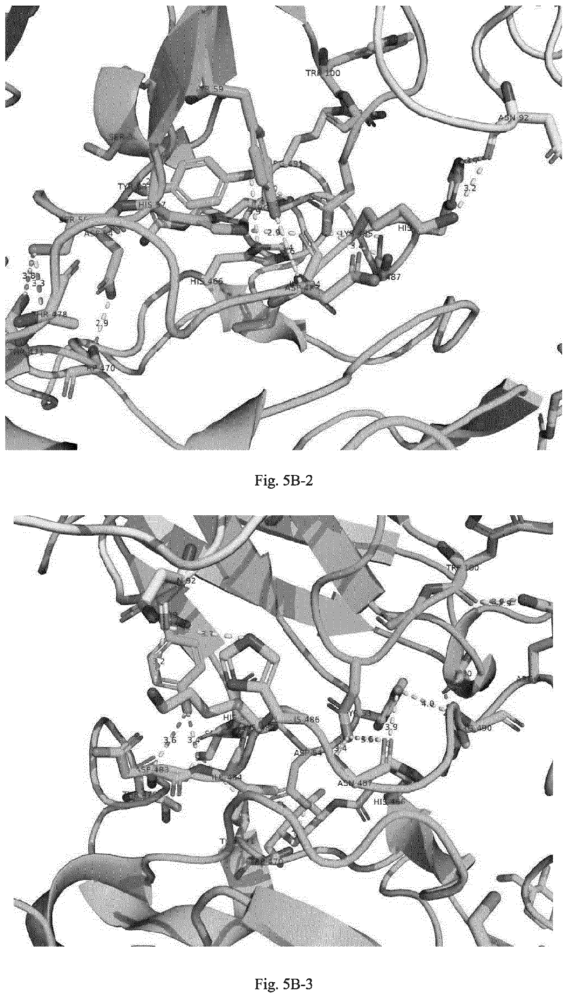

Figure 5B-2. Interface view 1 for the complex of Fab of antibody 202-2-1 and Her3, wherein the dotted line and numbers indicate the amino acid side chains interacting with each other and their distances (Å).
Figure 5B-3. Interface view 2 for the complex of Fab of antibody 202-2-1 and Her3, wherein the dotted line and numbers indicate the amino acid side chains interacting with each other and their distances (Å).

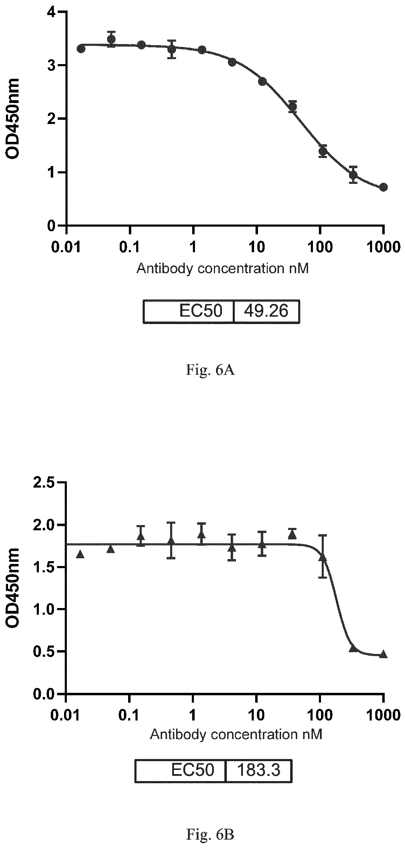

Figure 6A. Assay for the killing of anti-Her3 antibody 202-2-1 ADC drug on PC-9 tumor cells.
Figure 6B. Assay for the killing of anti-Her3 antibody 202-2-1 ADC drug on NCI-H358 tumor cells.

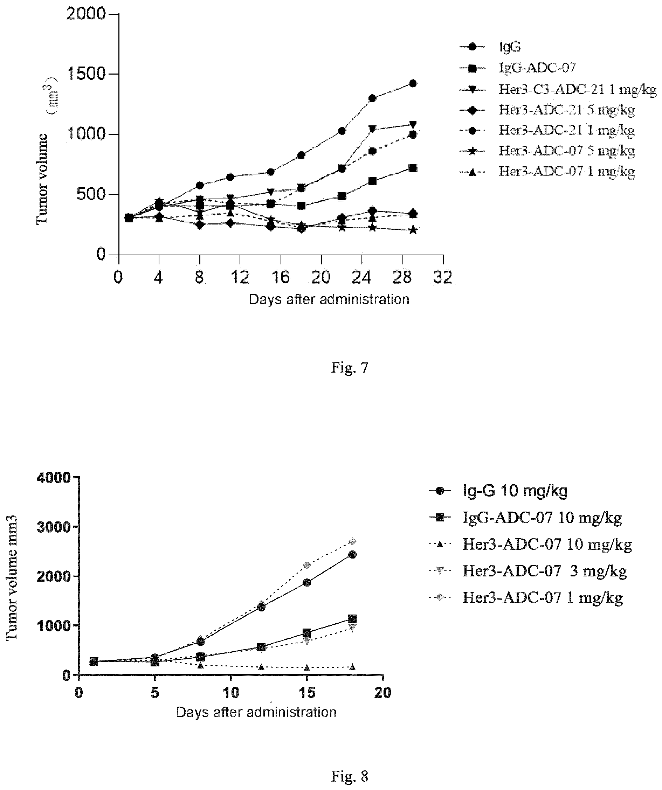

Figure 7. Pharmacodynamic testing of anti-Her3 antibody 202-2-1 ADC drug on NCI-H358 tumor model.
Figure 8. Pharmacodynamic testing of anti-Her3 antibody 202-2-1 ADC drug on one SW480 tumor model.

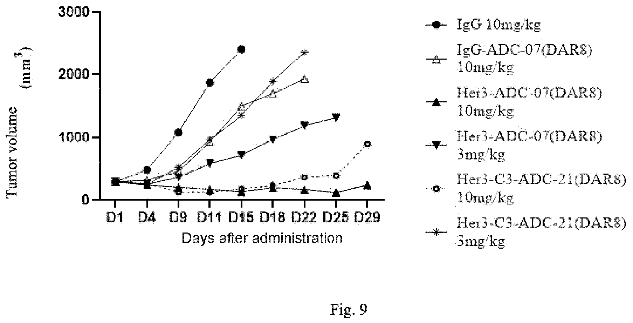

Figure 9. Pharmacodynamic testing of anti-Her3 antibody 202-2-1 ADC drug on another SW480 tumor model.

**Embodiments** By following description of specific examples, the present disclosure is further illustrated, but this is not intended to limit the present disclosure. Based on the teachings of the present disclosure, those skilled in the art may make various modifications or improvements without departing from the basic spirit and scope of the present disclosure. The reagents or instruments used without the manufacturer's indication are conventional products that can be commercially available.

### Example 1. Preparation of Her3 proteins and control antibodies

The nucleic acid sequence for amino acids at positions 1-643 (NCBI: NP_001973.2) of human Her3 was selected, in which a 6×His detection tag was added to the C-terminus and named as human Her3-his; the amino acid sequence at positions 20-641 (Uniport ID: Q61526-1) of rat Her3 was selected, in which a 6×His purification tag was added to the C-terminus and named as rat Her3-his. Control antibody 1 was obtained from MediaPharma's patent CN 103189392 B (heavy chain SEQ ID NO: 10, light chain SEQ ID NO: 14); control antibody 2 was obtained from Daiichi Sankyo Company, see IMGT database, IMGT/mAb DB ID: 964, INN No.: 11093. The genes of the above human Her3 antigens were synthesized, as well as the genes of two control antibodies were synthesized by codon-optimization (GenScript (Nanjing) Co., Ltd.), and then the genes were constructed into PTT5 expression vector, and a large number of plasmids were extracted. The extracted and prepared expression vectors were transiently transfected into HEK293F by PEIMAX and expressed for 7 days, and the expression supernatants were purified and prepared by Ni column or ProA. The amino acid sequence at positions 1-643 (NCBI: XP_001113953.2) of monkey Her3 was selected, in which a 6×His purification tag was added to the C-terminus and named as monkey Her3-his.

### Example 2. Screening of anti- Her3 antibodies from a phage library

Venous blood was collected from healthy individuals, and PBMC cells were extracted by Ficol, and then mRNA was extracted for reverse transcription. The heavy chain variable region (VH) and the light chain variable region (VL) were amplified by designing specific primers. ScFv was constructed on a phage vector through homologous recombination and electro-transformed into TG1, together with an auxiliary vector, and thus a full human ScFv phage library was obtained, with a library capacity of 1.5×10¹⁰. After initial enrichment of human Her3-His, followed by secondary enrichment of monkey Her3-His (purchased from Sino Biological Inc.), 96 phages were selected, and the optimal clones 202-2-1 and 202-3-1 were obtained by ELISA and T47D tumor cell flow cytometry. In order to improve the physicochemical properties of 202-3-1, site-directed mutagenesis was performed on its FR, resulting in a clone named 202-3-2.

### Example 3 Expression of anti-Her3 antibodies

The heavy chain variable regions of clone 202-2-1, clone 202-3-1, and clone 202-3-2 were respectively linked to the IgG1 constant region (SEQ NO: 29), while the light chain variable regions of clone 202-2-1, clone 202-3-1, and clone 202-3-2 were respectively linked to the Kappa constant region (SEQ NO: 30), and then constructed into PTT5 vector by codon optimization and gene synthesis (General Biosystems (Anhui) Co., Ltd.), respectively. Expression plasmids of the heavy chain and the light chain of anti-Her3 antibody were co-transfected into HEK293F cells by PEI max reagent and expressed in a 5% CO₂ shaker at 37° C for 7 days. Supernatants were collected and purified by ProteinA magnetic beads to obtain anti-Her3 antibodies, named as antibody 202-2-1, antibody 202-3-1, and antibody 202-3-2, respectively. The heavy chain variable region, the light chain variable region, and corresponding CDRs of antibody 202-2-1, antibody 202-3-1, and antibody 202-3-2 were shown in Table 1.

**Table 1. Amino acid sequences of anti-human Her3 antibodies**

| SEQ ID NO: | Name | Amino acid sequence |
|---|---|---|
| 1 | 202-2-1 Kabat HCDR1 | TYGMH |
| 2 | 202-2-1 Kabat HCDR2 | VIWYDVSHKYYADSVKG |
| 3 | 202-2-1 Kabat HCDR3 | DWGDPDAFDI |
| 4 | 202-2-1 IMGT HCDR1 | GFTFSTYG |
| 5 | 202-2-1 IMGT HCDR2 | IWYDVSHK |
| 6 | 202-2-1 IMGT HCDR3 | ARDWGDPDAFDI |
| 7 | 202-2-1 Kabat LCDR1 | RASQGISSYLA |
| 8 | 202-2-1 Kabat LCDR2 | AASTLQS |
| 9 | 202-2-1 Kabat/IMGT LCDR3 | QQLNSYPLT |
| 10 | 202-2-1 IMGT LCDR1 | QGISSY |
| \ | 202-2-1 IMGT LCDR2 | AAS |
| 12 | 202-3-1/202-3-2 Kabat HCDR1 | GYFWS |
| 13 | 202-3-1/202-3-2 Kabat HCDR2 | YIHYSGRTNYNPSLKS |
| 14 | 202-3-1/202-3-2 Kabat HCDR3 | ETGGSGHWYFDL |
| 15 | 202-3-1/202-3-2 IMGT HCDR1 | GGSISGYF |
| 16 | 202-3-1/202-3-2 IMGT HCDR2 | IHYSGRT |
| 17 | 202-3-1/202-3-2 IMGT HCDR3 | ARETGGSGHWYFDL |
| 18 | 202-3-1/202-3-2 Kabat LCDR1 | RASQSISSYLI |
| 19 | 202-3-1/202-3-2 Kabat LCDR2 | AASSLQS |
| 20 | 202-3-1/202-3-2 Kabat/IMGT LCDR3 | HQSYSTPWT |
| 21 | 202-3-1/202-3-2 IMGT LCDR1 | QSISSY |
| \ | 202-3-1/202-3-2 IMGT LCDR2 | AAS |
| 23 | 202-2-1 VH | EVQLVESGGGVVQPGRSLRLSCAASGFTFSTYG MHWVRQAPGKGLEWVAVIWYDVSHKYYADSV KGRFTISRDNSKNTLSLQMNSLRAEDTAVYYCA RDWGDPDAFDIWGQGTMVTVSS |
| 24 | 202-2-1 VL | DIQLTQSPSFLSASVGDRVTITCRASQGISSYLAW YQQKPGKAPKLLIYAASTLQSGVPSRFSGSGSGT EFTLTISSLQPEDFATYYCQQLNSYPLTFGGGTKV EIK |
| 25 | 202-3-1 VH | QVQLQESGPGLVKPSETLSLTCTVSGGSISGYFWS WIRQSPGKGLEWIGYIHYSGRTNYNPSLKSRVTIS VDTSKNQFSLKLSSVTAADTAVYYCARETGGSG HWYFDLWGRGNLVTVSS |
| 26 | 202-3-1 VL | DIQMTQSPSSLSASVGDRVTITCRASQSISSYLIW YQQKPGKAPKLLIYAASSLQSGVPSRFSGSGSGT DFTLTISSLQPEDFATYYCHQSYSTPWTFGQGTK VEIK |
| 27 | 202-3-2 VH | QVQLQESGPGLVKPSETLSLTCTVSGGSISGYFWS WIRQSPGQGLEWIGYIHYSGRTNYNPSLKSRVTIS VDTSKNQFSLQLSSVTAADTAVYYCARETGGSG HWYFDLWGRGNLVTVSS |
| 28 | 202-3-2 VL | DIQMTQSPSSLSASVGDRVTITCRASQSISSYLIW YQQKPGQAPRLLIYAASSLQSGVPSRFSGSGSGT DFTLTISSLQPEDFATYYCHQSYSTPWTFGQGTK VEIK |
| 29 | IgG1 CH heavy chain constant region | |
| 30 | Kappa CL light chain constant region | |
| 11 | 202-2-1 HC | EVQLVESGGGVVQPGRSLRLSCAASGFTFSTYG MHWVRQAPGKGLEWVAVIWYDVSHKYYADSV KGRFTISRDNSKNTLSLQMNSLRAEDTAVYYCA RDWGDPDAFDIWGQGTMVTVSSASTKGPSVFPL APSSKSTSGGTAALGCLVKDYFPEPVTVSWNSG ALTSGVHTFPAVLQSSGLYSLSSVVTVPSSSLGTQ TYICNVNHKPSNTKVDKKVEPKSCDKTHTCPPC PAPELLGGPSVFLFPPKPKDTLMISRTPEVTCVVV DVSHEDPEVKFNWYVDGVEVHNAKTKPREEQY NSTYRVVSVLTVLHQDWLNGKEYKCKVSNKAL PAPIEKTISKAKGQPREPQVYTLPPSRDELTKNQV SLTCLVKGFYPSDIAVEWESNGQPENNYKTTPPV LDSDGSFFLYSKLTVDKSRWQQGNVFSCSVMHE ALHNHYTQ KSLSLSPGK |
| 22 | 202-2-1 LC | DIQLTQSPSFLSASVGDRVTITCRASQGISSYLAW YQQKPGKAPKLLIYAASTLQSGVPSRFSGSGSGT EFTLTISSLQPEDFATYYCQQLNSYPLTFGGGTKV EIKRTVAAPSVFIFPPSDEQLKSGTASVVCLLNNF YPREAKVQWKVDNALQSGNSQESVTEQDSKDS TYSLSSTLTLSKADYEKHKVYACEVTHQGLSSPV TKSFNRGEC |

### Example 4 Affinity assay of anti-Her3 antibodies to Her3 proteins

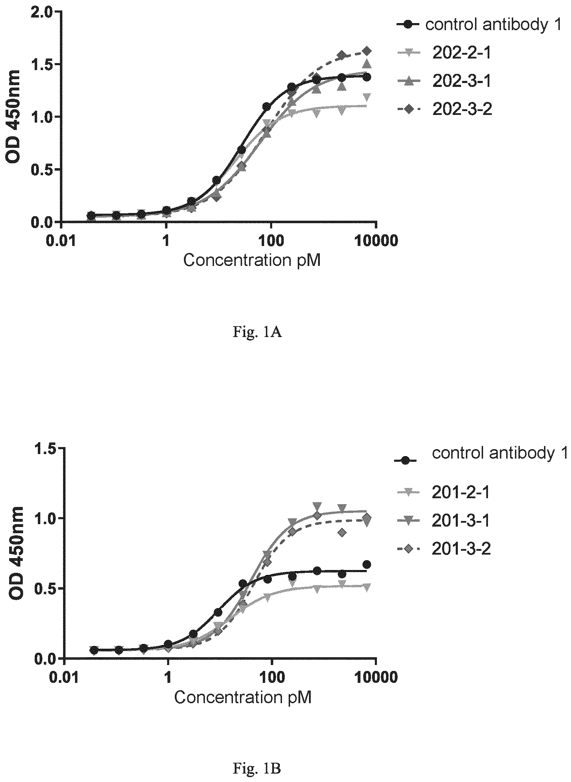

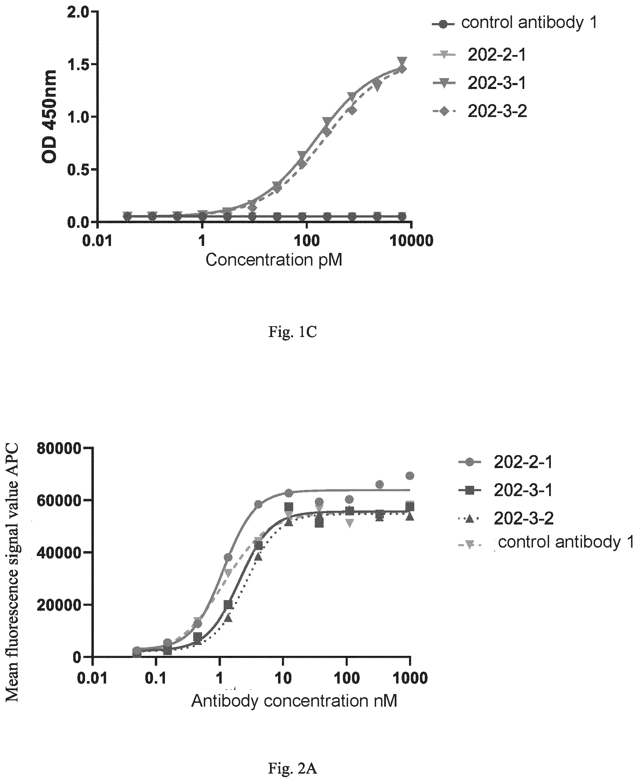

The affinity of anti-Her3 antibodies to Her3 proteins was tested by ELISA. Specific experimental procedures: human Her3-his, monkey Her3-his proteins, and rat Her3-his were diluted by carbonate buffer with pH 9.6 to a final concentration of 1 µg/mL and added into a 96-well microplate at 100 µL/well, and coated overnight at 4° C; the coating solution was discarded, and the plate was washed once with PBST, then 100 µL of PBS (containing 2% BSA) was added to each well, and blocked at 37°C for 1 h; then, 100 µL of anti-Her3 antibody diluted with PBS (containing 2% BSA) (starting at 1 µg, 3 folds serial dilution in duplicate wells) was added to each well, and then incubated at 37°C for 2 h; the plate was washed three times with PBST, and horseradish peroxidase-labeled anti-human Fc antibody (diluted at 1: 10000 with PBS) was added at 100 µL/well, then reacted for 1 h at 37°C. The plate was washed 5 times with PBST. TMB chromogenic solution was added at 100 µL/well and incubated at 37°C for 7 min for color development, and then the stop solution was added at 50 µL/well to terminate the reaction. The absorbance was measured at 450 nm, and EC₅₀ values were calculated from original data, which were imported into Graph-prism. The results were shown in Figures 1A-1C, and the detailed results were listed in Table 2. 202-2-1 had high affinity to human Her3 protein and monkey Her3 protein, and was comparable to control antibody 1, but did not have affinity to rat Her3 protein; 202-3-1 and 202-3-2 had high affinity to human Her3 protein and monkey Her3 protein, but lower than that of control antibody 1, and both of them bound to rat Her3 protein.

**Table 2. Results for affinity assay of anti-Her3 antibodies to Her3 proteins.**

| Antibody | Human Her3-his EC50 (pM) | Monkey Her3-his EC50 (pM) | Rat Her3-his EC50 (pM) |
|---|---|---|---|
| 202-2-1 | 20.97 | 15.91 | Not binding |
| 202-3-1 | 60.59 | 39.57 | 153.7 |
| 202-3-2 | 78.17 | 43.85 | 225.2 |
| Control antibody 1 | 27.72 | 9.275 | Not binding |

### Example 5 Assay of dynamic affinity of anti-Her3 antibodies

The dynamic affinity of anti-Her3 antibodies to Her3 proteins was detected by Fortibio, which is a commonly used dynamic affinity assay device. The method was briefly described as follows: serial dilution of human Her3-His was performed using PBST, to obtain 400 nM, 200 nM,100 nM, 50 nM, 25 nM, 12.5 nM, 6.25 nM, 3.125 nM, 1.5625 nM and 0 nM. The ProteinA biosensor (Pall Life Sciences) was pre-humidified with PBST buffer prior to use. Anti-Her3 antibody was diluted to 5 µg/mL with PBST and immobilized on the ProA sensor. The antibody-immobilized sensors were then equilibrated in PBST buffer for 60 seconds to obtain a baseline, then transferred to the antigen diluent for binding for 60 seconds, followed by dissociation in PBST for 180 seconds. After one analytical cycle, the sensor was regenerated with 10 mM Gly (pH 1.5). The association (Ka) and dissociation (Kd) rate constants were determined using Date analysis with a 1:1 model and used to calculate the dissociation equilibrium constant (KD). As shown in Table 3, the dynamic affinity of 202-2-1, 202-3-1, and 202-3-2 to human Her3 was higher, comparable to control antibody 1.

**Table 3: Analysis for Dynamic affinity of anti-Her3 antibodies to human Her3 proteins.**

| Antibody | KD (M) | ka (1/Ms) | kdis (1/s) |
|---|---|---|---|
| 202-2-1 | 1.31E-08 | 1.67E+05 | 2.18E-03 |
| 202-3-1 | 7.79E-09 | 1.82E+05 | 1.42E-03 |
| 202-3-2 | 6.81E-09 | 1.82E+05 | 1.24E-03 |
| Control antibody 1 | 5.30E-09 | 5.62E+05 | 2.97E-03 |

### Example 6 Assay for affinity of anti-Her3 antibodies to cells

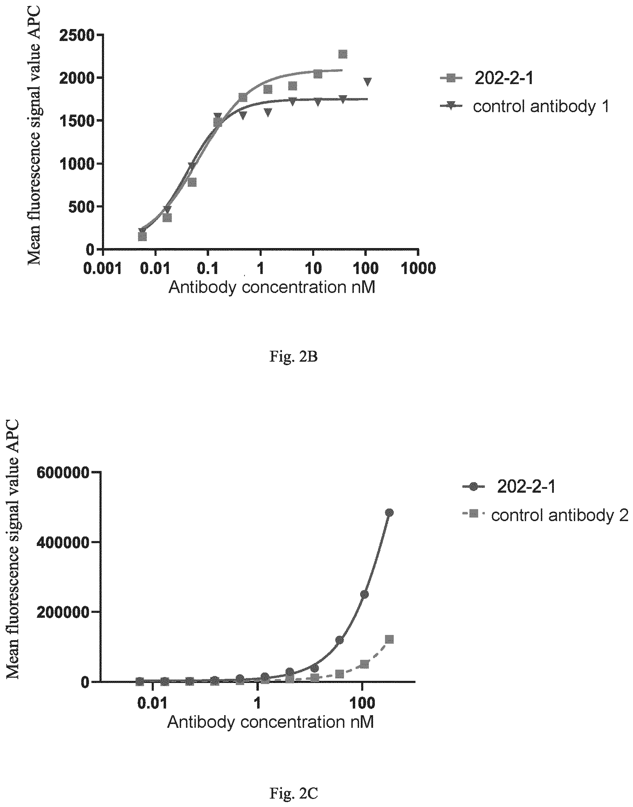

Her3 proteins were highly expressed in a variety of solid tumor cells. The affinity of Her3 antibody to tumor cells was detected by FACS assay. Specific experimental procedures: MCF-7 (Breast cancer cells, from ATCC, Article No. HTB-22), A549-human Her3 (Human non-small cell lung cancer, from ATCC, Article No. CRM-CCL-185), and T47D (Human breast ductal cancer, ATCC) cancer cells grown to logarithmic phase were digested by trypsin, resuspended to 5* 10^⁵ cells/ml in PBS buffer containing 2% BSA. 100 µL cell suspension was added to a 96-well plate. Control antibody 1 or control antibody 2, 202-2-1, 202-3-1, and 202-3-2 were diluted in PBS buffer containing 2% BSA, with 3 folds serial dilution at a starting concentration of 150 µg/mL or 50 µg/mL, to obtain about 10 concentrations. Then 100 µL antibody suspension was added into the 96-well plate and mixed; the plate was incubated at 4°C for 1 h, washed twice with pre-chilled PBS at 300 µL/well for each time, and then centrifuged at 500 g for 5 min; 1:50 fluorescent secondary antibody APC anti-Human IgG (purchased from Biolegend) was added at 100 µL/well and mixed. The plate was incubated at 4°C for 0.5 h, washed twice with pre-chilled PBS at 300 µL/well for each time, centrifuged at 500 g for 5 min, and then resuspended with 500 µL PBS. The average fluorescence signal value was measured by Beckman flow cytometry. The results were shown in Figures 2A-2C and Table 4. The affinity of 202-2-1 to cells was comparable to that of control antibody 1 and superior to that of control antibody 2, but the maximum MFI was higher than that of control antibody 1. The cell affinity of 202-3-1 and 202-3-2 was slightly weaker than that of control antibody 1.

**Table 4. Results for cell affinity assay of anti-Her3 antibodies.**

| Antibody | A549-human Her3 tumor cells | | T47D tumor cells | | MCF-7 tumor cells | |
|---|---|---|---|---|---|---|
| | EC50(nM) | Maximum MFI | EC50 (nM) | Maximum MFI | EC50 (nM) | Maximum MFI |
| 202-2-1 | 1.148 | 63823 | 0.080 | 2066 | N/A | 485095 |
| 202-3-1 | 2.036 | 55683 | Not detected | Not detected | Not detected | Not detected |
| 202-3-2 | 2.593 | 54823 | Not detected | Not detected | Not detected | Not detected |
| Control antibody 1 | 1.148 | 55461 | 0.043 | 1743 | Not detected | Not detected |
| Control antibody 2 | Not detected | Not detected | Not detected | Not detected | N/A | 121782 |

| | | | | | | |
|---|---|---|---|---|---|---|
| N/A in Table 4 indicates that the affinity is too weak to obtain a fitted EC50 result. | | | | | | |

### Example 7 Assay for inhibition of anti-Her3 antibody on NRG1-induced AKT phosphorylation

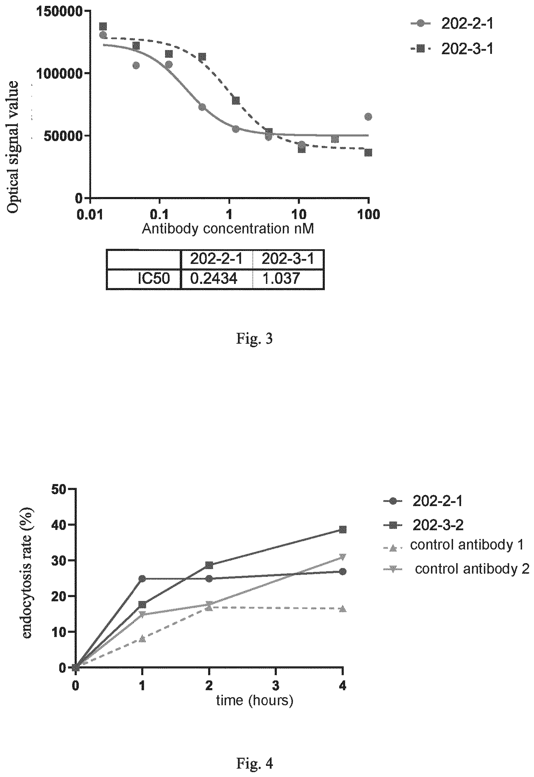

After binding to Her3/Her2 heterodimer with high affinity, NRG1 induced the phosphorylation of AKT in downstream signaling pathway, and stimulated the proliferation of tumor cells. The inhibition of anti-Her3 antibodies on NRG1-induced AKT phosphorylation pathway was detected using cell blotting. Specific experimental procedures: one day in advance, MCF-7 tumor cells (human breast cancer cells) were plated into a 96-well plate at 50000/well, rested overnight, and then starved in the serum-free RPMI1640 medium for about 24 h. Antibodies 202-2-1 and 202-3-1 were diluted with 3 folds serial dilution at a starting concentration of 100 nM, and then added to the cells, followed by incubation at 37°C for 30 min, to which was then added NRG1 (Sino Biological, Article No.: 11609 HNCH) at a final concentration of 10 ng/ml. The plate was incubated at 37°C for 30 min, and then the reaction was terminated to discard the supernatant. The plate was washed once with PBS, and then formaldehyde was added at 100 µL/well to immobilize at room temperature for 30 min. The plate was washed twice with PBS, to which was added pre-chilled methanol at 100 µL/well for 30 min for membrane penetration, followed by washing three times with PBS. PBS (containing 2% BSA) was added to each well, and the plate was blocked for 2 h. 100 µL of 1:500 anti-Phospho Akt (Ser473) antibody (CST, Article No. 9271-S) was added, and the plate was incubated overnight at 4°C. Then, the plate was washed three times with PBST, and 1:1000 HRP alpaca anti-rabbit IgG antibody (NB-biolab, Sichuan) was added, followed by incubation at 37°C for 1 h. The plate was washed 5 times with PBST. 100 µL of ECL developer was added (Vazyme, Article No. E412-01), and the luminescence value was read using a microplate reader (MD, Model: i3x). The IC50 values were calculated using Graph-prism, as shown in Figure 3. Both anti-Her3 antibodies 202-2-1 and 202-3-1 significantly inhibited NRG1-induced AKT phosphorylation, but antibody 202-2-1 was significantly stronger than antibody 202-3-1, with IC50 values of 0.2434 nM and 1.037 nM, respectively.

### Example 8 Assay for specificity of anti-Her3 antibody

The specificity of Her3 antibodies binding to Her3 proteins was determined by an ELISA method, without binding to its homologous proteins EGFR, Her2 and Her4. Specific experimental procedures: human EGFR-His, human Her2-His, human Her4-His (purchased from Sino Biological Inc.), and human Her3-his proteins were diluted by carbonate buffer with pH 9.6 to a final concentration of 1 µg/mL, respectively, and added into 96-well microplate at 100 µL/well, and coated overnight at 4°C; the coating solution was discarded, and the plate was washed once with PBST, then 100 µL PBS (containing 2% BSA) was added to each well, and the plate was blocked at 37°C for 1 h; then, 100 µL of Her3 antibody diluted with PBS (containing 2% BSA) was added to each well at 1 µg/mL, 200 ng/mL, and 40 ng/mL, and the plate was incubated at 37°C for 2 h; the plate was washed 3 times with PBST, and horseradish peroxidase-labeled anti-human Fc antibody (1:10000 dilution with PBS) was added at 100 µL/well, then the plate was incubated at 37°C for 1 h; the plate was washed 5 times with PBST. TMB chromogenic solution for color development was added at 100 µL/well, the plate was incubated at 37°C for 7 min, and then the stop solution was added to terminate the reaction at 50 µL/well, and the plate was read on a microplate readerat 450 nm. The results are shown in Table 5. 202-2-1, 202-3-1, and 202-3-2 specifically bound to human Her3 protein, but did not bind to human EGFR, human Her2, and human Her4 proteins.

**Table 5. Assay for binding of anti-Her3 antibodies to Her3 family proteins**

| Antigen name | EGFR-his | | | Her2-his | | | Her3-his | | | Her4-his | | |
|---|---|---|---|---|---|---|---|---|---|---|---|---|
| Antibody concentrati on | 1000 | 200 | 40 | 1000 | 200 | 40 | 1000 | 200 | 40 | 1000 | 200 | 40 |
| ng/mL | | | | | | | | | | | | |
| Control antibody 1 | 0.05 1 | 0.04 9 | 0.04 9 | 0.05 2 | 0.04 9 | 0.04 7 | 0.85 0 | 0.85 4 | 0.76 3 | 0.05 1 | 0.05 0 | 0.04 9 |
| OD450 nM | | | | | | | | | | | | |
| 202-2-1 | 0.05 1 | 0.05 1 | 0.05 1 | 0.04 9 | 0.05 0 | 0.04 9 | 0.61 0 | 0.69 5 | 0.57 3 | 0.05 1 | 0.05 1 | 0.05 0 |
| OD450 nM | | | | | | | | | | | | |
| 202-3-1 | 0.05 0 | 0.05 0 | 0.05 5 | 0.04 8 | 0.04 9 | 0.05 2 | 0.33 3 | 0.34 4 | 0.28 1 | 0.05 0 | 0.05 1 | 0.04 9 |
| OD450 nM | | | | | | | | | | | | |
| 202-3-2 | 0.05 1 | 0.05 0 | 0.05 1 | 0.05 0 | 0.05 1 | 0.05 0 | 0.42 7 | 0.40 7 | 0.30 6 | 0.05 0 | 0.05 1 | 0.05 1 |
| OD450nM | | | | | | | | | | | | |

### Example 9 Assay of endocytosis of anti-Her3 antibodies

The endocytosis of anti-Her3 antibody by A549-human Her3 (human non-small cell lung cancer cells) was detected by FACS. The A549-human Her3 cells grown to the logarithmic growth phase were digested with trypsin, and then the cells were collected by centrifugation and washed three times with pre-chilled PBS; the cells were resuspended in PBS (containing 1% BSA), to which was added the anti-Her3 antibody to be tested at a concentration of 10 µg/mL, followed by incubation at 4°C for 1 h; the cells were collected by centrifugation, washed three times with PBS, resuspended in DMEM+10% FBS, divided into 4 parts, and incubated at 37°C for 0, 1, 2, and 4 h respectively; after completion of the incubation phase, the cells were collected by centrifugation, washed three times with pre-chilled PBS, resuspended in 50 µL of 1% BSA (in PBS), and then 1 µL of fluorescent secondary antibody APC anti-Human IgG (Biolegend) was added to each well, the mixture was mixed well, and the plate was incubated at 4°C for 0.5 h; the cells were collected by centrifugation, washed three times with pre-chilled PBS, resuspended in 200 µL of PBS, and loaded on the flow cytometer (Beckman, Model: CytoFlex) for detection. According to the formula: Endocytosis rate (%) = [1-(The average fluorescence value of the test sample at this time point - The average fluorescence value of the negative control sample at this time point)/ (The average fluorescence value of the test sample at 0 h - The average fluorescence value of the negative control sample at 0 h)] * 100. The results were shown in Figure 4. Anti-Her3 antibody 202-2-1 had strong endocytosis activities for A549-human Her3 tumor cells, reaching about 26.9% at 4 h, which was stronger than that of control antibody 2 at 16.6% and comparable to that of control antibody 1. However, anti-Her3 antibody 202-2-1 reached its maximum endocytosis rate before the control antibody, and has a faster endocytosis rate than the control antibody. The endocytic activity of antibody 202-3-2 was stronger than that of control antibody 1 and control antibody 2.

### Example 10. Assay of antigenic epitopes

### 10.1 Assay of anti-Her3 antibody epitope competition

A competitive ELISA was used to determine whether anti-Her3 antibodies 202-2-1 competed with the control antibodies 1 and 2 for binding epitopes. The specific steps were as follows: human Her3-his antigen was coated with CBS coating solution at 100 ng/well, and then the plate was incubated at 4°C overnight; the coating solution was removed, and the wells were washed once with 300 µL of PBS, then each well was blocked with PBS (containing 2% BSA) solution at 100 µL/well, followed by incubation at 37°C for 2 h; the blocking solution was discarded, and antibody 202-2-1, control antibody 1, and control antibody 2 were diluted with 3 folds dilution starting from 15 µg/mL with a total of 11 dilution points, but the 12th point was replaced by the diluent; the antibody was added to the wells at 50 µL/well in duplicate; biotin-labeled control antibody 1 diluted to 10 ng/mL and control antibody 2 diluted to 88 ng/mL were added to each well at 50 µL/well, and then the plate was incubated at 25°C for 2 h; the wells were washed with 300 µL of PBST, repeated 3 times, and then added with HRP anti-biotin secondary antibody diluted with PBS (containing 2% BSA) at a ratio of 1:5000 at 100 µL/well, followed by incubation at 25°C for 1 h; the wells were washed with 300 µL of PBST, repeated 5 times; 100 µL of TMB chromogenic solution (Huzhou InnoReagents, Anhui) was added to each well, and after 5 min of color development at room temperature, 50 µL of H₂SO₄ was added to each well to stop the reaction, and the plate was read immediately on a microplate reader at OD450 nm. The original data were imported into Graph-prism to calculate EC50 values, and the results were shown in Figure 5A. The results showed that anti-Her3 antibody 202-2-1 did not compete with control antibody 2 for binding epitopes.

### 10.2 Determination of antigenic epitope of anti-Her3 antibody

In order to further study the specific mode of action of the Her3 antibody and the epitope recognized by it, a complex of 202-2-1 Fab fragment and Her3 protein was prepared, and the structure of the antibody antigen complex was then analyzed by cryo-electron microscopy.
(1) Preparation of antigen: The DNAthat encodes the amino acid sequence of HER3 extracellular domain (20-643) acquired from the uniprot database was synthesized after sequence optimization (293F as the host), a 6 * His tag was added to the C-terminal of the protein. The gene was constructed into the pLVX (MIAO LING BIO, Article No. P0249) expression vector and transfected into 293F cells (ATCC; PTA-5077), followed by screening to obtain a stable cell pool. The culture supernatant containing antigens was obtained by fermentation and cultivation of cells. The antigens were captured by Ni affinity column chromatography and further purified by molecular sieves. The final antigens were dissolved in Tris-NaCl solution at pH 7.5 (0.7 mg/ml). Antigen quality characterization: 1. SDS-PAGE experiment showed that the band was sharp under both non-reducing and reducing conditions. 2. The purity was 95.6% by SEC-HPLC. 3. The purity was 97.44% by reversed phase chromatography HPLC. 4. The affinity of antigen and antibody tested by ELISA showed that the affinity of this batch of antigen and antibody 202-2-1 was less than 0.1 nM, and the next step of the experiment can be carried out.
(2) Preparation of Fab-antigen complex: Antibody 202-2-1 was dissolved in 20 mM acetic acid+150 mM sodium chloride to the concentration of 20.7 mg/ml. Pepsin and the antibody were incubated at 37°C for 240 min, and the digestion products were analyzed by SDS-PAGE and separated by SEC (Superdex 200 Increase 10/300 GL column) column, the main peak was collected. The above products were incubated with Papain for 120 min at 37°C, the digestion products were analyzed by SDS-PAGE and separated by SEC SEC (Superdex 200 Increase 10/300 GL column) column , the main peak was collected to obtain the Fab of antibody 202-2-1. The antigen prepared in step (1) was mixed with Fab and then incubated overnight at 4°C. The incubated products were concentrated by centrifugation and analyzed by SDS-PAGE, followed by separation using over SEC (Superdex 200 Increase 10/300 GL column) column, the main peak was collected to obtain the Fab-antigen complex. Before preparing samples for cryoelectron microscopy, the quality of the complex was examined by negative staining method (VitrobotTM MarkIV, easiGlow^{™} Glow Discharge, 200 kV TF20, CCD camera 895). The negative staining results showed that most of the complexes had consistent morphology. 80 photos were selected for reconstruction of negative staining, and the PDB model (4LEO) was used for simulation of density maps. The simulation results showed that the complex can overlap with most part of the PBD model (including Fab binding domains), indicating that the quality of the complex met the requirements for cryoelectron microscopy observation.
(3) Sample preparation for cryo-microscopy/screening on cryo-microscopy Glacios/data collection: the complex prepared in (2) was diluted with 25 mM Tris-HCl (pH 7.5) and 150 mM NaCl to 0.2 mg/ml, and centrifuged at 12000 g for 15 min; all relevant equipment s and reagents were prepared, and 3 µL of sample was added dropwise to 300 mesh R 1.2/1.3 holey carbon Film (Quantifoil) grid, and 200 KV Glacios electron microscopy was used to observe and screen photos of the complex. High resolution cryo-electron micrographs were collected by a 300 kV Titan Krios electron microscope (equipped with a K3 direct electron detector, Gatan brand), a total of 3272 photos were collected for further analysis. Parameter settings: magnification 105 K, physical pixel 0.819 A, a total of 56.3 e-/A² doses of electrons divided into 40 frames, and underfocus range set from -1.0 µM to -2.4 µM.
(4) Photos processing/atomic model establishment and epitope analysis: all 3272 photos data were processed using CryoSPARC v3.3.1, including evaluation and correction of contrast transfer function, 2D classification, heterogeneous refinement, and homogeneous refinement. To build a structural model of Her3-Fab complex, Her3 structure (PDB 1M6B) and Fab structure with high homology to antibody 202-2-1 (light chain: PDB 7CDJ, heavy chain: PDB 6D01) were fitted into the cryo-EM map using the UCSF Chimera software. The model was manually built in Coot (Emsley P et al., Acta Crystallogr D Biol Crystallogr. 2010 Apr;66(Pt 4):486-501) with the guidance of the cryo-EM map, and in combination with real space refinement using CCP-EM. The results were shown in Figure 5B-1, 5B-2, and 5B-3.

The structural analysis results showed: the antigenic epitope to which 202-2-1 antibody Fab bound was located in the domain 3 of human Her3, within amino acids 328-499 of SEQ ID No.31, and provided that Her3 antigen was numbered according to the amino acid sequence of SEQ ID No.31, specifically located in Histidine at position 466, (Tryptophan-Threonine) at positions 470-471, Threonine at position 478, (Aspartic acid-Isoleucine-Lysine-Histidine-Asparagine) at positions 483-487, Isoleucine at position 484, and (Arginine-Arginine) at positions 490-491; based on the amino acid number of SEQ ID No.23, (Serine) at position 30, (Tyrosine-Aspartic acid) at positions 53-54, (Serine-Histidine) at positions 56-57, Tyrosine at position 59, and Tryptophan at position 100 of Fab heavy chain were involved in the binding of the antigen; Asparagine at position 92 of Fab light chain numbered according to amino acid sequence of SEQ ID No.24 were involved in binding of the antigen.

### Example 11 Synthesis of bioactive molecules and intermediates used in the synthesis of "drug-linker compounds"

### Example 11.1: Synthesis of (S)-7-ethyl-7-hydroxy-14-(3-hydoxypropyl)-10,13-dihydro-11H-[1,3]dioxolo[4,5-g]pyrano[3',4':6,7]indolizino[1,2-b]quinolin-8,11(7H)-dione(A1)

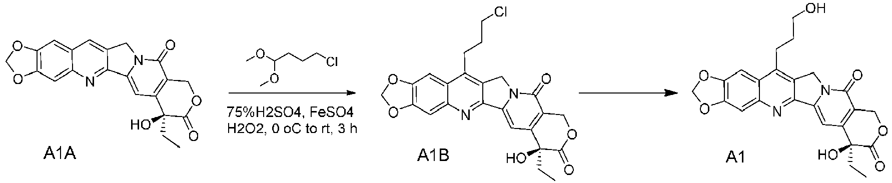

### Step 1: Synthesis of (S)-14-(3-chloropropyl)-7-ethyl-7-hydroxyl-10,13-dihydro-11H-[1,3]dioxolo[4,5-g]pyrano[3',4':6,7]indolizino[1,2-b]quinolin-8,11(7H)-dione (A1B)

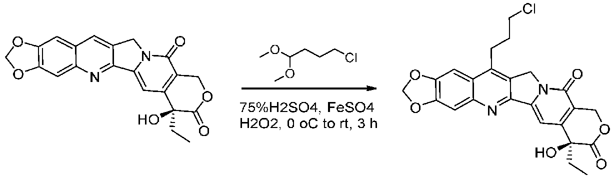

In ice bath, to a solution of compound (S) 7-ethyl-7-hydroxy-10,13-dihydro-11H-[1,3]dioxolo[4,5-g]pyrano[3',4':6,7]indeno[1,2-b]quinolin-8,11(7H)-dione (A1.5-A, 500 mg) in 75% sulfuric acid solution (5 mL), was added ferrous sulfate heptahydrate (570 mg of ferrous sulfate heptahydrate was dissolved in 1 mL of water) and 4-dimethoxychlorobutane (3.89 g), and after the reaction solution was stirred for 3 minutes, hydrogen peroxide (29%, 2.5 mL) was added dropwise. The reaction solution was stirred at 0 °C for 5 minutes, warmed to room temperature, and then reacted for 3 h under stirring. The reaction solution was diluted with water (50 mL), and extracted with ethyl acetate (80 mL × 2). The organic phase was washed with saturated brine, dried over anhydrous sodium sulfate, and concentrated to obtain the crude product, which was further purified by C18 column (acetonitrile/0.05% formic acid in water: 5%-60%), to obtain the target compound AlB(yellow solid, 400 mg, yield: 67%).
LCMS (ESI) [M+H]⁺: 468.9;
1H NMR (400 MHz, DMSO-d6) δ 7.65 (s, 1H), 7.51 (s, 1H), 7.24 (s, 1H), 6.50 (s, 1H), 6.30 (s, 2H), 5.42 (s, 2H), 5.26 (s, 2H), 3.81 (d, J = 5.9 Hz, 2H), 3.22 (s, 2H), 1.98 (d, J = 6.7 Hz, 4H), 0.88 (t, J = 7.2 Hz, 3H).

### Step 2: Synthesis of (S)-7-ethyl-7-hydroxyl-14-(3-hydroxylpropyl)-10,13-dihydro-11H-[1,3]dioxolo[4,5-g]pyrano[3',4':6,7]indolizino[1,2-b]quinolin-8,11(7H)-dione (A1)

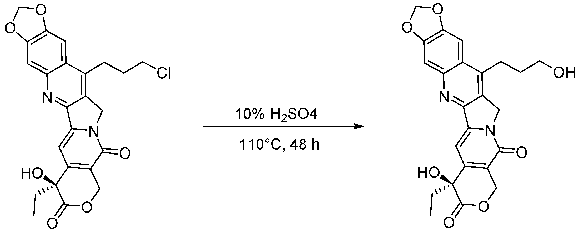

Compound (S)-7-ethyl-7-hydroxyl-14-(3-chloropropyl)-10,13-dihydro-11H-[1,3]dioxolo[4,5-g]pyrano[3',4':6,7]indolizino[1,2-b]quinolin-8,11(7H)-dione (100 mg, 0.213 mmol) was dissolved in 10% sulfuric acid (5 mL) solution and reacted at 110 °C for 48 h. To the reaction solution, was added saturated sodium bicarbonate (30 mL) solution, and then the resultant solution was extracted with dichloromethane (10 mL × 5), dried over anhydrous sodium sulfate, filtered by suction, and concentrated under reduced pressure to obtain crude products, which was purified by preparative HPLC (acetonitrile/water containing 0.05% formic acid), to obtain (S)-7-ethyl-7-hydroxyl-14-(3-hydroxylpropyl)-10,13-dihydro-11H-[1,3]dioxolo[4,5-g]pyrano[3',4':6,7]indolizino[1,2-b]quinolin-8,11(7H)-dione (Al, 1.78 mg).
LCMS (ESI) [M+H]⁺: 451.0;
¹HNMR(400 MHz, DMSO-*d6*) δ 7.63 (s, 1H), 7.50 (s, 1H), 7.24 (s, 1H), 6.48 (s, 1H), 6.28 (s, 2H), 5.47 - 5.37 (m, 2H), 5.32 - 5.19 (m, 2H), 3.51 - 3.46 (m, 2H), 3.17 - 3.13 (m, 2H), 1.92 - 1.76 (m, 4H), 0.90 - 0.84 (m, 3H).

### Example 11.2: Synthesis of (S)-4-ethyl-8-fluoro-4-hydroxyl-11-(3-hydroxylpropyl)-9-methyl-1,12-dihydro-14H-pyrano[3',4':6,7]indolizino[1,2-b]quinolin-3,14(4H)-dione (A2)

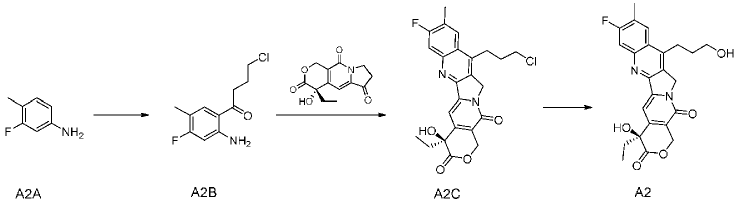

### Step 1:

At 0 °C, to a solution of compound A2A (10 g) in 1,2-dichloroethane (200 mL), were successively added 1 mol/L boron trichloride (96 mL) and 4-chlorobutyronitrile (9.9 g) dropwise. The reaction was stirred at 80 °C for 2 hours. The reaction solution was cooled to room temperature, and then 2 mol/L hydrochloric acid (90 mL) was added, and the mixture was stirred at 80 °C under reflux for 0.5 hours. The reaction solution was cooled to room temperature, diluted with a small amount of water, and extracted with dichloromethane (200 mL × 3). The organic phase was dried over anhydrous sodium sulfate, filtered, and concentrated to obtain a crude product, which was separated and purified by column chromatography (petroleum ether : ethyl acetate=10/1) to obtain the target compound A2B (4 g).
LCMS (ESI) [M+H]⁺: 230.0.

### Step 2:

To compound A2B (50 mg), were successively added (S)-4-ethyl-4-hydroxy-7,8-dihydro-1H-pyrano[3,4-fjindolizin-3,6,10(4H)-trione (35 mg) and p-toluenesulfonic acid monohydrate (41.4 mg), and then the mixture was dissolved in dichloromethane (30 mL). The solution was mixed to be clear, concentrated under reduced pressure, pumped to vacuum with an oil pump, and the reaction was performed under vacuum at 120 ° C for 3 hours. LCMS showedthe reaction was complete. The reaction mixture was cooled to room temperature, and then water (20 mL) was added. The resulting mixture was extracted with dichloromethane (20 mL × 3). The combined organic phases were successively dried over anhydrous sodium sulfate, filtered, and the filtrate was concentrated under reduced pressure to obtain a crude product. The crude product was separated and purified by column chromatography (dichloromethane : methanol = 20 : 1) to obtain the target compound A2C (80 mg) as a white solid.
LCMS (ESI) [M+H]⁺: 457.0.

### Step 3

Compound A2C (75 mg) was dissolved in hexamethylphosphoric triamide, to which was added pure water (0.8 mL), and then the reaction solution was stirred at 100 °C for 72 h. LCMS test showed thatthe reaction was complete. The target compound A2(10 mg) was purified by preparative chromatography (0.01% TFA in water, MeCN).
LCMS (ESI) [M+H]¹: 439.2;
¹H NMR (400 MHz, DMSO-d6) δ 8.22 (d, *J* = 8.4 Hz, 1H), 7.87 (d, *J* = 10.9 Hz, 1H), 7.31 (s, 1H), 6.50 (s, 1H), 5.43 (s, 2H), 5.30 (s, 2H), 4.67 (t, *J* = 4.9 Hz, 1H), 3.55 - 3.47 (m, 2H), 3.28 - 3.20 (m, 2H), 2.51 (s, 3H), 1.93 - 1.81 (m, 4H), 0.88 (t, *J* = 7.3 Hz, 3H).

### Example 11.3: Synthesis of (S,E)-14-(3-amino-1-propylen-1-yl)-7-ethyl-7-hydroxyl-10,13-dihydro-11H-[1,3]dioxolo[4,5-g]pyrano[3',4':6,7]indolizino[1,2-b]quinolin-8,11(7H)-dione (A3)

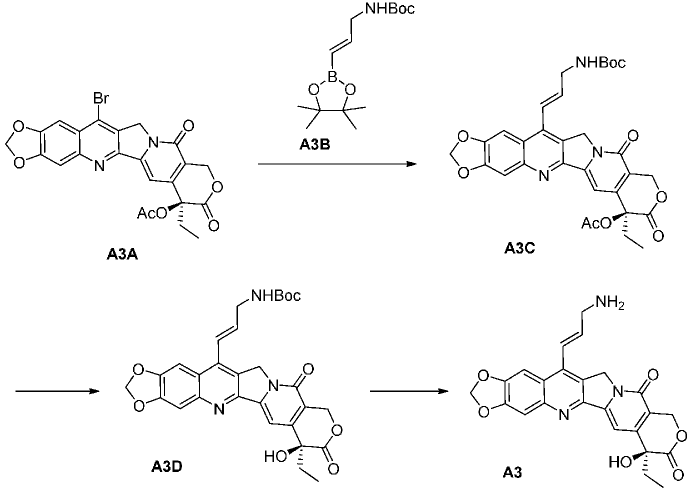

### Step 1:

Compound A3A (200 mg, 0.39 mmol), compound A3B (112 mg, 0.39 mmol), cesium fluoride (152 mg, 0.975 mmol) and tetrakis(triphenylphosphine)palladium (45 mg, 0.039 mmol) were added in 1,4-dioxane (8 mL). The microwave reaction was carried out at 120 °C for 0.5 hour under nitrogen atmosphere. LCMS indicatedthe reaction was complete. A mixed solvent of dichloromethane (20 mL) and methanol (10 mL) was added to the reaction, and then the resulting solution was filtered. The filtrate was concentrated, and the crude product was purified by preparative TLC (dichloromethane:methanol = 30:1) to obtain the target compound (S,E)-14-(3-((tert-butoxycarbonyl)amino)-1-propylen-1-yl)-7-ethyl-8,11-dioxo-7,8,11,13-tetrahydro-10H-[1,3]dioxolo[4,5-g]pyrano[3',4':6,7]indolizino[1,2-b]quinolin-7-yl acetate (A3C, 60 mg, yield: 26%) as brown solid.
LCMS (ESI) [M+H]⁺ = 590.3;
¹HNMR(400 MHz, DMSO-d6) δ 7.63 (s, 1H), 7.49 (s, 1H), 7.35 (s, 1H), 7.09 (d, *J* = 16.7 Hz, 1H), 6.93 (s, 1H), 6.45 (d, *J =* 16.6 Hz, 1H), 6.30 (s, 2H), 5.47 (s, 2H), 5.34 - 5.24 (m, 2H), 3.94 (s, 2H), 2.21 (br s, 3H), 2.03 - 1.96 (m, 2H), 1.45 (s, 9H), 0.91 (t, *J* = 6.7 Hz, 3H).

### Step 2:

To a solution of compound (S,E)-14-(3-((tert-butoxycarbonyl)amino)-1-propylen-1-yl)-7-ethyl-8,11-dioxo-7,8,11,13-tetrahydro-10H-[1,3]dioxolo[4,5-g]pyrano[3',4':6,7]indolizino[1,2-b]quinolin-7-yl acetate (A3C, 50 mg, 0.085 mmol) in methanol (15 mL), was added sodium methoxide (9.2 mg, 0.17 mmol), and then the reaction was stirred at 50 °C for 2 hours. LCMS indicated the reaction was complete. The reaction solution was concentrated to obtain the crude target compound (S,E)-14-(3-((tert-butoxycarbonyl)amino)-1-propylen-1-yl)-7-ethyl-7-hydroxy-10,13-dihydro-11H-[1,3]dioxolo[4,5-g]pyrano[3',4':6,7]indolizino[1,2-b]quinolin-8,11(7H)-dione (A3D, 50 mg) as a brown solid. LCMS (ESI) [M+H]⁺ = 548;

### Step 3:

To a solution of compound (S,E)-14-(3-((tert-butoxycarbonyl)amino)-1-propylen-1-yl)-7-ethyl-7-hydroxy-10,13-dihydro-11H-[1,3]dioxolo[4,5-g]pyrano[3',4':6,7]indolizino[1,2-b]quinolin-8,11(7H)-dione (A3D, 50 mg, 0.091 mmol) in dichloromethane (2 mL), was added trifluoroacetic acid (1 mL), and then the reaction was stirred at room temperature for 30 minutes. LCMS showed the reaction was complete. The reaction solution was concentrated, and the crude product was purified by preparative HPLC (acetonitrile/0.05% formic acid in water) to obtain the target compound (S,E)-14-(3-amino-1-propylen-1-yl)-7-ethyl-7-hydroxy-10,13-dihydro-11H-[1,3]dioxolo[4,5-g]pyrano[3',4':6,7]indolizino[1,2-b]quinolin-8,11(7H)-dione (A3) (8.1 mg, yield 21%), as brown solid.
LCMS (ESI) [M+H]⁺ = 448.3;
¹H NMR (400 MHz, DMSO-d6) δ 8.20 (s, 2H), 7.72 (s, 1H), 7.54 (s, 1H), 7.40 (d, *J* = 16.5 Hz, 1H), 7.27 (s, 1H), 6.52 - 6.46 (m, 2H), 6.31 (s, 2H), 5.42 (s, 2H), 5.27 (s, 2H), 3.86 (br s, 2H), 1.90 - 1.83 (m, 2H), 0.88 (t, *J* = 7.1 Hz, 3H).

### Example 11.4: Synthesis of (S)-2-amino-N-((4-(4-ethyl-8-fluoro-4-hydroxyl-9-methyl-3,14-dioxo-3,4,12,14-tetrahydro-1H-pyrano[3',4':6,7] indolizino[1,2-b]quinolin-11-yl)propoxy)methyl)acetamide (B1)

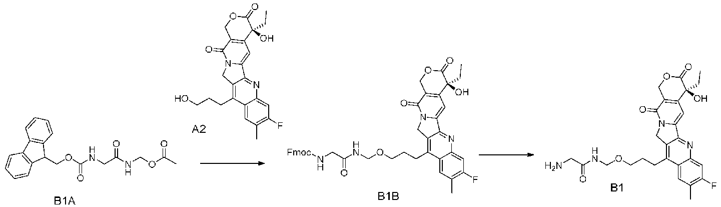

### Step 1:

Compound A2 (160 mg, 0.365 mmol) was dissolved in N,N-dimethylformamide (3 mL), to which was added (2-((((9H-fluoren-9-yl)methoxy)carbonyl)amino)acetamido)methyl acetate ((B1A, 672 mg, 1.83 mmol), and then hydrochloric acid in ethyl acetate (0.073 mL, 3M) was added to the reaction solution. The reaction solution was stirred at room temperature overnight. LCMS detection showed the reaction was complete. The reaction solution was directly purified by reversed phase chromatography (acetonitrile/0.05% FA in water: 5% to 50%) to obtain the target compound B1B (80 mg, yield 29.0%) as a white solid.
LCMS (ESI) [M+H]⁺ = 747.4;
¹H NMR (400 MHz, DMSO-d6) δ 8.74 (t, *J* = 6.4 Hz, 1H), 8.28 - 8.17 (m, 1H), 7.99 - 7.88 (m, 3H), 7.73 (d, *J* = 7.3 Hz, 2H), 7.63 (t, *J* = 5.7 Hz, 1H), 7.44 (t, *J* = 7.4 Hz, 2H), 7.35 (t, *J* = 7.2 Hz, 3H), 6.58 (s, 1H), 5.48 (s, 2H), 5.29 (s, 2H), 4.66 (d, *J* = 6.3 Hz, 2H), 4.32 (d, *J* = 6.9 Hz, 2H), 4.26 (d, *J* = 6.1 Hz, 1H), 3.71 (d, *J* = 5.8 Hz, 2H), 3.57 (t, *J* = 5.7 Hz, 2H), 3.29 - 3.20 (m, 2H), 2.55 (s, 3H), 2.00 - 1.86 (m, 4H), 0.93 (t, *J* = 7.2 Hz, 3H).

Step 2: B1B (240 mg) was dissolved in DMF (5 ml), to which was added piperidine (1 ml), and then the compounds were stirred for 20 min. The solution was evaporated under reduced pressure to remove low-boiling components, and the residue was directly used in the next step.
ESI-MS (m/z): 525.2 [M+H]⁺.

A small amount of crude product was purified by reversed-phase chromatography (acetonitrile/0.05% FA in water: 5% to 50%) to give the target compound.
ESI-MS (m/z): 525.1 [M+H]⁺;
¹H NMR (400 MHz, DMSO) δ 9.13 (t, *J* = 6.6 Hz, 1H), 8.21 (d, *J* = 8.1 Hz, 1H), 8.02 (brs, 2H), 7.89 (d, *J* = 10.8 Hz, 1H), 7.32 (s, 1H), 6.54 (s, 1H), 5.44 (s, 2H), 5.28 (s, 2H), 4.66 (d, *J* = 6.5 Hz, 2H), 3.64 (s, 2H), 3.53 (t, *J* = 6.1 Hz, 2H), 3.25-3.18 (m, 2H), 2.52 (s, 3H), 1.98-1.84 (m, 4H), 0.88 (t, *J* = 7.3 Hz, 3H).

### Example 11.5: Synthesis of (S)-2-amino-N-((3-(7-ethyl-7-hydroxyl-8,11-dioxo-7,8,11,13-tetrahydro-10H-[1,3]dioxolo[4,5-g]pyrano[3',4':6,7]indolizino [1,2-b]quinolin-14-yl)propoxy)methyl)acetamide (B2)

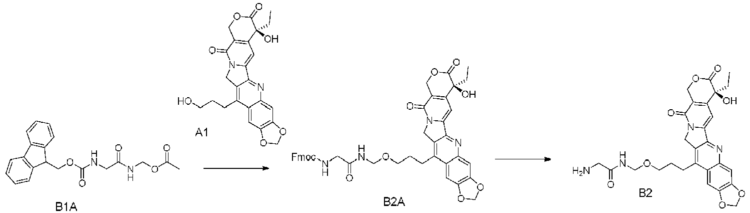

Step 1: Compound (B1A) (368 mg), compound (A1) (440 mg) and pyridinium p-toluenesulfonic acid (PPTS) (25 mg) were refluxed in dichloromethane (20 ml) for 20 h, and then the reaction solution was washed with aqueous sodium bicarbonate solution and aqueous hydrochloric acid solution, respectively. The organic solvent was removed under reduced pressure to obtain a crude product, which was separated and purified by column chromatography (dichloromethane:methanol = 10/1) to obtain the target compound B2A (240 mg).
LCMS (ESI) [M+H]⁺: 759.

Step 2: B2A (240 mg) was dissolved in DMF (5 ml), to which was added piperidine (1ml), and then the compound was stirred for 20 min. The low boiling point components were removed under reduced pressure, and the residue was directly used in the next synthesis step. A small amount of the crude product was purified by reversed phase chromatography (acetonitrile/0.05% FA in water: 5% to 50%) to obtain the target compound B2.
ESI-MS (m/z): 537 [M+H]⁺;
¹H NMR (400 MHz, DMSO-d6) δ 9.13 (t, 1H), 8.04 (br, 2H), 7.58 (s, 1H), 7.51 (s, 1H), 7.25 (s, 1H), 6.29 (s, 2H), 5.43 (S, 2H), 5.21 (s, 2H), 4.65 (d, 2H), 3.63 (m, 2H), 3.53 (m, 2H), 3.11 (m, 2H), 1.87 (m, 4H), 0.88 (t, 3H).

### Example 11.6: (S,E)-2-amino-N-(3-(7-ethyl-7-hydroxyl-8,11-dioxo-7,8,11,13-tetrahydro-10H-[1,3]dioxolo[4,5-g]pyrano[3',4':6,7]indolizino[1,2-b]quinolin-14-yl)allyl)acetamide (B3)

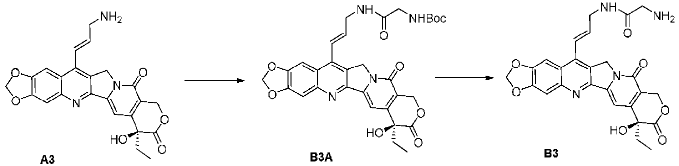

### Step 1:

At room temperature, to a solution of compound A3 (200 mg, 0.447 mmol) in N,N-dimethylformamide (5 mL), were added triethylamine (135 mg, 1.341 mmol) and N-hydoxy-2,5-dioxopyrrolidin-1-yl (tert-butoxycarbonyl)glycinate (183 mg, 0.671 mmol), and then the mixture was allowed to react at room temperature for 1 h. LCMS showed the reaction has completed. Water (20 mL) was added to the reaction solution, and the resultant mixture was extracted with ethyl acetate (20 mL×3). The organic phase was washed with saturated brine, dried over anhydrous sodium sulfate, and concentrated to obtain the target compound B3A (130 mg, yield: 48%) as a brown solid.
LCMS (ESI) [M+H]⁺ = 605.6.

### Step 2:

At room temperature, compound B3A (130 mg, 0.215 mmol) was dissolved in a solution of HCl in1,4-dioxane (5 mL) and then reacted at room temperature for 30 min. LCMS showed the reaction was complete. The reaction solution was purified by reversed phase column chromatography, to obtain the target compound B3 (60 mg, yield: 52%) as a brown solid.
LCMS (ESI) [M+H]⁺ = 505.2;
¹H NMR (400 MHz, DMSO-d6) δ 8.85 (s, 1H), 8.15 (s, 2H), 7.70 (s, 1H), 7.54 (s, 1H), 7.26 (s, 1H), 7.21 (d, *J* = 16.2 Hz, 1H), 6.51 (d, *J* = 16.2 Hz, 1H), 6.31 (s, 2H), 5.42 (s, 2H), 5.28 (s, 2H), 4.19 (s, 2H), 1.86 - 1.82 (m, 4H), 0.87 (t, *J* = 7.3 Hz, 3H).

### Example 11.7: N⁶,N⁶-dimethyl-N²-((6-(2-(methanesulfonyl)pyrimidin-5-yl)hexan-5-ynoyl)- L-valinyl)-L-lysine (Cl)

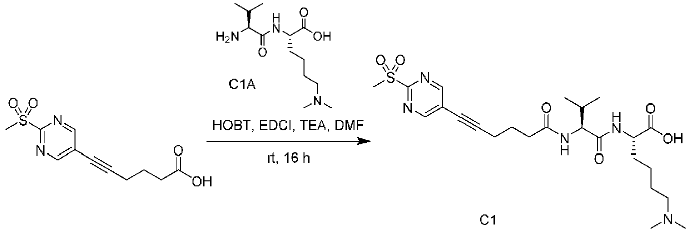

6-(2-(Methanesulfonyl)pyrimidin-5-yl)hex-5-ynoic acid (268 mg), compound C1A (328 mg), and triethylamine (322 mg) were dissolved in N,N-dimethylformamide (5 mL). Then, 1-hydroxybenzotriazole (HOBT, 162 mg) and 1-(3-dimethylaminopropyl)-3-ethylcarbodiimide hydrochloride (EDCI, 229 mg) were added, and the reaction solution was stirred at room temperature for 16 h. The reaction solution was directly purified over reversed-phase C18 column (acetonitrile and 0.05% formic acid aqueous solution system) to obtain the target compound N⁶,N⁶-dimethyl-N²-((6-(2-(methanesulfonyl)pyrimidin-5-yl)hexan-5-ynoyl)-L-valinyl)-L-lysine (C1, white solid, 327 mg).
LCMS (ESI) [M+H]¹: 524.4.
¹H NMR (400 MHz) δ 9.13 (s, 2H), 7.95 (t, *J* = 8.8 Hz, 2H), 4.21 (dd, *J* = 8.8, 6.9 Hz, 1H), 4.08 - 4.03 (m, 1H), 3.41 (s, 3H), 2.55 (t, *J* = 7.0 Hz, 2H), 2.42 - 2.32 (m, 4H), 2.27 (s, 6H), 1.98 (dd, *J* = 13.6, 6.8 Hz, 1H), 1.86 - 1.77 (m, 2H), 1.74 - 1.55 (m, 2H), 1.47 - 1.37 (m, 2H), 1.31 - 1.23 (m, 2H), 0.85 (dd, *J* = 12.8, 6.8 Hz, 6H).

### Example 11.8: N⁶,N⁶-dimethyl-N²-((6-(4-(2-(methanesulfonyl)pyrimidin-5-yl)-1H-1,2,3-triazol-1-yl)hexanoyl)-L-valinyl)-L-lysine (C2)

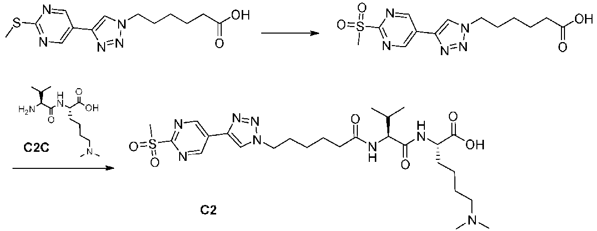

### Step 1:

Compound 6-(4-(2-(methylthio)pyrimidin-5-yl)-1H-1,2,3-triazol-1-yl)hexanoic acid (C2A, 1.0 g, 3.3 mmol) was dissolved in a mixed solvent of tetrahydrofuran and water (40 mL, 3:1), to which was added potassium peroxymonosulfonate (10.0 g, 16.3mmol). The reaction was stirred for 3 h at room temperature, and completed as shown by LCMS detection. The reaction solution was filtered. The cake was washed with DMSO, and the filtrate was combined and subj ected to reversed phase purification (C18, acetonitrile: 0.1% formic acid = 5%-55%), to afford 6-(4-(2-(methanesulfonyl)pyrimidin-5-yl)-1H-1,2,3-triazol-1-yl)hexanoic acid (C2B, 750 mg, yield 67.9%) as white solid.
LCMS (ESI) [M+H]⁺ = 340.1;
¹H NMR (400 MHz, DMSO-d6) δ 9.48 (s, 2H), 8.95 (s, 1H), 4.49 (t, *J* = 7.0 Hz, 2H), 3.45 (s, 3H), 2.22 (t, *J* = 7.3 Hz, 2H), 1.95 - 1.84 (m, 2H), 1.61 - 1.50 (m, 2H), 1.36 - 1.26 (m, 2H).

### Step 2:

Compound 6-(4-(2-(methanesulfonyl)pyrimidin-5-yl)-1H-1,2,3-triazol-1-yl)hexanoic acid (C2B, 300 mg, 0.88 mmol) was dissolved in N,N-dimethylformamide (6 mL), to which were added HATU (337 mg, 0.88mmol) and DIPEA (286 mg, 2.21 mmol), and the reaction was stirred at room temperature for 30 min. Then, dipeptide C1.16-D (243 mg, 0.88 mmol) was added, and the reaction was stirred at room temperature for 2 h. LCMS indicated completion of the reaction, and the reaction solution was separated and purified over C18 column (acetonitrile/0.01% FA aqueous solution: 5%-50%) to obtain the target compound N⁶,N⁶-dimethyl-N^{z}-((6-(4-(2-(methanesulfonyl)pyrimidin-5-yl)-1H-1,2,3-triazol-1-yl)hexanoyl)-L-valinyl)- L-lysine (C2, 300 mg, yield 57%) as white solid.
LCMS (ESI) [M+H]⁺ = 595.5, t_{R} = 2.024 min.
¹H NMR (400 MHz, DMSO-d6) δ 9.49 (s, 2H), 9.01 (s, 1H), 7.85 (br s, 1H), 7.84 (d, *J* = 8.9 Hz, 1H), 4.48 (t, *J* = 6.8 Hz, 2H), 4.17 - 4.15 (m, 1H), 4.02 (br s, 1H), 3.44 (s, 3H), 2.42 (br s, 2H), 2.30 (s, 6H), 2.18 - 2.14 (m, 2H), 1.99 - 1.96 (m, 1H), 1.88 (dd, *J* = 14.6, 7.1 Hz, 2H), 1.67 (br s, 1H), 1.59 - 1.53 (m, 2H), 1.43 (br s, 2H), 1.28 - 1.26 (m, 5H), 0.83 - 0.89 (m, 6H).

### Example 11.9: N⁶,N⁶-diethyl-N²-((6-(2-(methanesulfonyl)pyrimidin-5-yl)hexan-5-ynoyl)- L-valinyl)-L-lysine (C3)

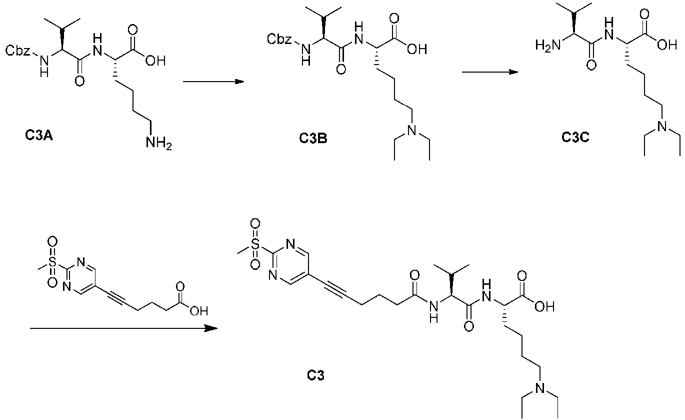

### Step 1:

Compound C3A (5.0 g, 12.05 mmol) was dissolved in dichloromethane (100 mL), to which was added acetaldehyde (3.2 g, 72.3 mmol), and the reaction was stirred for 10 min at room temperature. Then, sodium triacetoxyborohydride (12.8 g, 60.25 mmol) was added to the reaction solution, and the reaction was stirred for 1 h at room temperature. LCMS showed completion of the reaction. Saturated aqueous solution of ammonium chloride was added to the reaction solution, and then stirred for 1 h. The reaction solution was evaporated under rotation to dryness. The crude product was separated and purified over reversed-phase C18 column (acetonitrile to 0.05% formic acid aqueous solution: 5% to 55%) to obtain the target compound C3B (4.57 g, yield 82.0%) as white solid.
LCMS (ESI) [M+H]⁺ = 436.4;
¹H NMR (400 MHz, DMSO-d6) δ 7.70 (d, *J* = 7.0 Hz, 1H), 7.41 (d, *J* = 9.0 Hz, 1H), 7.38 - 7.26 (m, 5H), 5.08 - 4.99 (m, 2H), 4.00 (dd, *J* = 12.6, 6.5 Hz, 1H), 3.86 (dd, *J* = 8.6, 6.8 Hz, 1H), 2.74 (dd, *J* = 14.0, 6.9 Hz, 4H), 2.64 - 2.54 (m, 2H), 2.05 - 1.94 (m, 1H), 1.72 - 1.52 (m, 2H), 1.52 - 1.38 (m, 2H), 1.38 - 1.18 (m, 2H), 1.04 (t, *J* = 7.1 Hz, 6H), 0.87 - 0.81 (m, 6H).

### Step 2:

At room temperature, compound C3B (1.6 g, 3.68 mmol) was dissolved in methanol (80 mL), to which was then added Pd/C (0.16 g), and the reaction was stirred at room temperature under hydrogen atmosphere for 12 h. LCMS indicated completion of the reaction. The reaction solution was filtered, and the filtrate was concentrated under reduced pressure to obtain the target compound C3C (900 mg, yield 82%) as off-white solid.
¹H NMR (400 MHz, DMSO-d6) δ 8.04 (br s, 1H), 4.02 - 3.99 (m, 1H), 3.10 (d, *J* = 4.5 Hz, 1H), 2.65 (q, *J* = 7.1 Hz, 4H), 2.55 - 2.51 (m, 2H), 2.06 - 1.93 (m, 1H), 1.73 - 1.54 (m, 2H), 1.47 - 1.38 (m, 2H), 1.30 - 1.21 (m, 2H), 1.01 (t, *J* = 7.1 Hz, 6H), 0.89 (d, *J* = 6.9 Hz, 3H), 0.79 (d, *J* = 6.8 Hz, 3H).

### Step 3:

Compound 6-(2-(methanesulfonyl)pyrimidin-5-yl)hex-5-ynoic acid (268 mg, 1 mmol) was dissolved in DMF (8 mL), to which were successively added HATU (380 mg, 1 mmol) and triethylamine (322 mg, 2.5 mmol), and then stirred at room temperature for 20 min, followed by addition of compound C3C (301 mg, 1 mmol). The reaction solution was further stirred for 30 min at room temperature. After completion of the reaction by LCMS detection, the reaction solution was directly purified over reversed phase C18 column (acetonitrile and 0.05% formic acid aqueous solution system) to obtain the target compound C3 (280 mg, yield 51%) as a white solid.
LCMS (ESI) [M+H]⁺ = 552.3;
¹H NMR (400 MHz, DMSO-d6) δ 9.13 (s, 2H), 7.95 (d, *J* = 8.9 Hz, 1H), 7.86 (d, *J* = 7.2 Hz, 1H), 4.18 (dd, *J* = 8.8, 6.8 Hz, 1H), 4.02 (dd, *J* = 12.8, 7.2 Hz, 1H), 3.41 (s, 3H), 2.74 - 2.69 (m, 4H), 2.62 - 2.52 (m, 4H), 2.44 - 2.29 (m, 2H), 2.04 - 1.94 (m, 1H), 1.86 - 1.77 (m, 2H), 1.72 - 1.54 (m, 2H), 1.51 - 1.39 (m, 2H), 1.33 - 1.23 (m, 2H), 1.02 (t, *J* = 7.2 Hz, 6H), 0.87 - 0.82 (m, 6H).

### Example 11.10: N⁶,N⁶-dipropyl-N²-((6-(2-(methanesulfonyl)pyrimidin-5-yl)hexan-5-ynoyl)- L-valinyl)-L-lysine (C4)

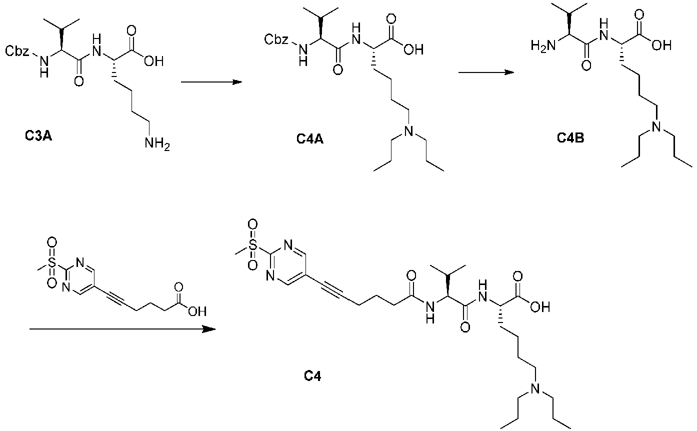

### Step 1:

Compound C3A (5.0 g, 12 mmol) was dissolved in dichloromethane (100 mL), to which was added n-propionaldehyde (4.2 g, 72.3 mmol), and then the reaction was stirred at room temperature for 10 min, followed by addition of sodium triacetoxyborohydride (12.8 g, 60.25 mmol). The reaction was stirred at room temperature for 1 h. LCMS showed the reaction was complete. To the reaction solution, was added saturated aqueous solution of ammonium chloride, and then the solution was stirred for 1 h and evaporated under rotation to dryness. After filtration, the filtrate was separated and purified over reversed phase C18 column (acetonitrile to 0.05% formic acid aqueous solution: 5% to 55%), to obtain the target compound C4A (4.57 g, yield 82.0%) as a white solid.
LCMS (ESI) [M+H]⁺ = 464.0;
¹H NMR (400 MHz, DMSO-d6) δ 7.81 (d, *J* = 7.3 Hz, 1H), 7.38 - 7.28 (m, 5H), 5.04 (d, *J* = 1.7 Hz, 2H), 4.12 - 4.02 (m, 1H), 3.94 - 3.82 (m, 1H), 2.65 - 2.52 (m, 6H), 2.06 - 1.94 (m, 1H), 1.76 - 1.64 (m, 1H), 1.64 - 1.53 (m, 1H), 1.52 - 1.40 (m, 6H), 1.34 - 1.18 (m, 2H), 0.92 - 0.80 (m, 12H).

### Step 2:

At room temperature, compound C4A (2.0 g, 4.32 mmol) was dissolved in methanol (80 mL), to which was then added Pd/C (0.16 g), and the reaction was stirred at room temperature under hydrogen atmosphere for 12 h. LCMS indicated completion of the reaction. The reaction solution was filtered, and the filtrate was concentrated under reduced pressure to obtain the target compound C4B (1.2 g, yield 85.5%) as a white solid.

### Step 3:

Compound 6-(2-(methanesulfonyl)pyrimidin-5-yl)hex-5-ynoic acid (100 mg, 0.373 mmol) was dissolved in N,N-dimethylformamide (1 mL), to which were then added HATU (142 mg, 0.373 mmol) and N,N-diisopropylethylamine (120 mg, 0.93 mmol), and the reaction was stirred for 30 min. Then, compound C4B (122 mg, 0.371 mmol) was added, and the reaction solution was stirred at room temperature for 1 h. After LCMS indicated that the reaction was complete, the reaction solution was directly purified over a reversed-phase C18 column (acetonitrile and 0.05% formic acid aqueous solution system), to obtain the target compound N⁶,N⁶-dipropyl-N²-((6-(2-(methanesulfonyl)pyrimidin-5-yl)hexan-5-ynoyl)-L-valinyl)-L-lysine(C4, 50 mg, yield 28%) as a pale yellow solid.
LCMS (ESI) [M+H]⁺ = 580.0;
¹H NMR (400 MHz, DMSO-d6) δ 8.24 (s, 2H), 7.98 - 7.93 (m, 2H), 4.24 - 4.16 (m, 1H), 4.10 (d, *J* = *5.2* Hz, 1H), 3.41 (s, 3H), 2.79 - 2.64 (m, 6H), 2.55 (t, *J* = 7.1 Hz, 2H), 2.45 - 2.26 (m, 2H), 2.06 - 1.91 (m, 1H), 1.89 - 1.78 (m, 2H), 1.76 - 1.66 (m, 1H), 1.64 - 1.57 (m, 1H), 1.57 - 1.42 (m, 6H), 1.37 - 1.24 (m, 2H), 0.93 - 0.78 (m, 12H).

### Example 12 Synthesis of drug-linker compound

### Example 12.1: N-((11S,14S)-11-(4-(dimethylamino)butyl)-1-((S)-4-ethyl-8-fluoro-4-hydroxyl-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methanesulfonyl)pyrimidin-5-yl)hexan-5-ynamide (DL-01)

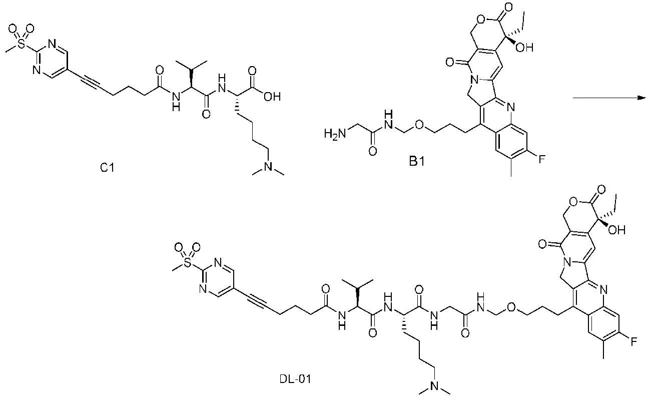

Compound N⁶,N⁶-dimethyl-N²-((6-(2-(methanesulfonyl)pyrimidin-5-yl)hexan-5-ynoyl)-L-valinyl)-L-lysine (126 mg) (Cl), Bl (150 mg), 1-hydroxybenzotriazole (49 mg), 1-(3-dimethylaminopropyl)-3-ethylcarbodiimide hydrochloride (69 mg) and diisopropylethylamine (156 mg, 1.21 mmol) were dissolved in N,N-dimethylformamide (10 mL), and then the reaction was stirred at 40 °C for 12 h. LCMS indicated completion of the reaction. The reaction solution was filtered and purified by preparative HPLC (acetonitrile/water containing 0.05% formic acid) to obtain compound N-((11S,14S)-11-(4-(dimethylamino)butyl)-1-((S)-4-ethyl- 8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methanesulfonyl)pyrimidin-5-yl)hex-5-ynamide (DL-01) (12 mg, yield 6.0%) as a yellow solid.
LCMS (ESI) [M+H]⁺ = 1030.6;
¹H NMR (400 MHz, DMSO-d6) δ 9.10 (s, 2H), 8.62 (t, *J* = 6.4 Hz, 1H), 8.23 - 8.13 (m, 2H), 8.07 (d, *J* = 7.2 Hz, 1H), 7.95 - 7.83 (m, 2H), 7.31 (s, 1H), 6.53 (s, 1H), 5.44 (s, 2H), 5.29 (s, 2H), 4.64 - 4.53 (m, 2H), 4.28 - 4.20 (m, 1H), 4.19 - 4.11 (m, 1H), 3.76 - 3.69 (m, 2H), 3.50 (t, *J* = 5.7 Hz, 2H), 3.41 (s, 3H), 3.23 - 3.19 (m, 2H), 2.67 - 2.62 (m, 2H), 2.57 - 2.54 (m, 2H), 2.54 (s, 3H), 2.42 - 2.26 (m, 3H), 2.02 - 1.77 (m, 6H), 1.68 - 1.66 (m, 1H), 1.58 - 1.45 (m, 3H), 1.36 - 1.25 (m, 2H), 0.88 (t, *J* = 7.4 Hz, 3H), 0.82 (t, J= 6.3 Hz, 6H).

### Example 12.2: N-((S)-1-(((S)-6-(diethylamino)-1-((2-(((E)-3-((S)-7-ethyl-7-hydroxyl-8,11-dioxo-7,8,11,13-tetrahydro-10H-[1,3]dioxolo[4,5-g]pyrano[3',4':6,7]indolizino[1,2-b]quinolin-14-yl)allyl)amino)-2-oxoethyl)amino)-1-oxohexan-2-yl)amino)-3-methyl-1-oxobutan-2-yl)-6-(2-(methanesulfonyl)pyrimidin-5-yl)hexan-5-ynamide(DL-02)

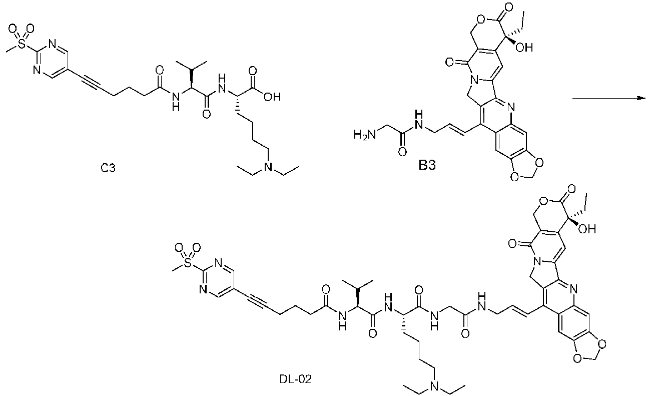

To a solution of compound B3 (40 mg, 0.074 mmol) and compound C3 (40 mg, 0.072 mmol) in N,N-dimethylformamide (4 mL), were sequentially added HBTU (30 mg, 0.079 mmol) and DIPEA (26 mg, 0.2 mmol). The reaction solution was stirred for 1 h at room temperature. LCMS indicated completion of the reaction. The reaction solution was purified by preparative HPLC (acetonitrile/0.05% trifluoroacetic acid in water), to provide compound N-((S)-1-(((S)-6-(diethylamino)-1-((2-(((E)-3-((S)-7-ethyl-7-hydroxy-8,11-dioxo-7,8,11,13- tetrahydro-10H-[1,3]dioxolo[4,5-g]pyrano[3',4':6,7]indolizino[1,2-b]quinolin-14-yl)allyl)amino)-2-oxoethyl)amino)-1-oxohexan-2-yl)amino)-3-methyl-1-oxobutan-2-yl)-6-(2-(methanesulfonyl)pyrimidin-5-yl)hex-5-ynamide (DL-02, 11.1 mg, yield 14%).
LCMS (ESI) [M+H]¹ =1038.6;
¹H NMR (400 MHz, DMSO-d6) δ 9.10 (s, 2H), 9.02 (s, 1H, TFA), 8.33 - 8.25 (m, 2H), 8.12 (d, *J* = 7.3 Hz, 1H), 7.92 (d, *J* = 8.4 Hz, 1H), 7.68 (d, *J* = 4.1 Hz, 1H), 7.52 (d, *J* = 2.8 Hz, 1H), 7.26 (s, 1H), 7.15 (d, *J* = 16.2 Hz, 1H), 6.52 - 6.50 (m, 2H), 6.30 (s, 2H), 5.43 (s, 2H), 5.28 - 5.24 (m, 2H), 4.27 (d, *J* = 6.2 Hz, 2H), 4.12 (br s, 2H), 3.81 (br s, 2H), 3.41 (s, 3H), 3.11 - 3.07 (m, 6H), 2.97 (br s, 2H), 2.53 - 2.50 (m, 1H), 2.39 - 2.24 (m, 4H), 1.85 - 1.79 (m, 8H), 1.16 (t, *J* = 6.4 Hz, 6H), 0.88 (t, *J* = 6.4 Hz, 3H), 0.78 (d, *J* = 6.4 Hz, 6H).

### Example 12.3: (S)-6-(dimethylamino)-N-(2-(((3-((S)-4-ethyl-8-fluoro-4-hydroxyl-9-methyl-3,14-dioxo-3,4,12,14-tetrahydro-1H-pyrano[3',4':6,7]indolizino[1,2-b]quinolin-11-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (DL-03)

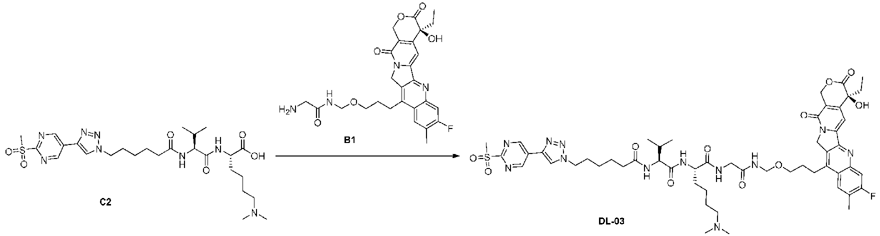

Compound C2 (113 mg, 0.19 mmol) and compound B1 (100 mg, 0.19 mmol) were dissolved in DMF (1 mL), to which were then added HBTU (72 mg, 0.19 mmol) and N,N-diisopropylethylamine (61 mg, 0.48 mmol). After addition, the reaction solution was stirred for 1 h at room temperature. LCMS indicated completion of the reaction. The reaction solution was purified by preparative chromatography (0.01% TFA in water, MeCN), to provide the target compound (S)-6-(dimethylamino)-N-(2-(((3-((S)-4-ethyl-8-fluoro-4-hydroxy-9-methyl- 3,14-dioxo-3,4,12,14-tetrahydro-1H-pyrano[3',4':6,7]indolizino[1,2-b]quinolin-11-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (DL-03, 16 mg, yield 8%) as a white solid.
LCMS (ESI) [M+H]⁺ = 1101.6;
¹H NMR (400 MHz, DMSO) δ 9.46 (s, 2H), 8.93 (s, 1H), 8.63 (t, *J* = 6.3 Hz, 1H), 8.20 - 8.28 (m, 2H), 8.04 (d, *J* = 7.2 Hz, 1H), 7.88 (d, *J* = 10.8 Hz, 1H), 7.81 (d, *J* = 8.3 Hz, 1H), 7.31 (s, 1H), 6.53 (s, 1H), 5.44 (s, 2H), 5.29 (s, 2H), 4.63 - 4.54 (m, 2H), 4.46 (t, *J* = 7.0 Hz, 2H), 4.29 - 4.19 (m, 1H), 4.15 - 4.08 (m, 1H), 3.73 (d, *J* = 6.9 Hz, 2H), 3.50 (t, *J* = 5.8 Hz, 2H), 3.44 (s, 3H), 3.24 - 3.14 (m, 2H), 3.01 - 2.91 (m, 2H), 2.72 (s, 6H), 2.53 (s, 3H), 2.23 - 2.07 (m, 2H), 1.93 - 1.84 (m, 6H), 1.72 -1.64 (m, 1H), 1.61 - 1.45 (m, 6H), 1.34 - 1.22 (m, 4H), 0.87 (t, *J* = 7.4 Hz, 3H), 0.82 - 0.76 (m, 6H).

### Example 12.4: N-((11S,14S)-11-(4-(di-n-propylamino)butyl)-1-((S)-7-ethyl-7-hydroxyl-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methanesulfonyl)pyrimidin-5-yl)hexan-5-ynamide(DL-04)

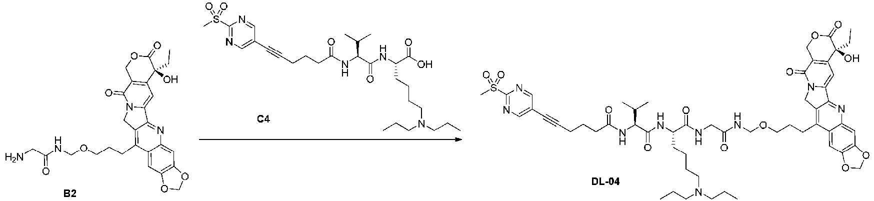

Compound C4 (43 mg, 0.074 mmol) and compound B2 (40 mg, 0.075 mmol) were dissolved in N,N-dimethylformamide (1 mL), to which were then added HBTU (28 mg, 0.075 mmol) and N,N-diisopropylethylamine (24 mg, 0.187 mmol). The reaction solution was stirred for 1 h at room temperature. LCMS detection indicated completion of the reaction. The reaction solution was directly purified by preparative chromatography (0.01% Trifluoroacetic Acid in water, acetonitrile), to provide the the target compound (DL-04, 8.5 mg, yield 10%) as a yellow solid.
LCMS (ESI) [M+H]⁺ = 1098.6;
¹H NMR (400 MHz, DMSO-d6) δ 9.10 (s, 2H), 9.03 (s, 1H), 8.64 (t, *J* = 6.4 Hz, 1H), 8.19 (t, *J* = 5.9 Hz, 1H), 8.08 (d, *J* = 7.4 Hz, 1H), 7.92 (d, *J* = 8.5 Hz, 1H), 7.59 (s, 1H), 7.51 (s, 1H), 7.24 (s, 1H), 6.49 (s, 1H), 6.29 (s, 2H), 5.42 (s, 2H), 5.24 (s, 2H), 4.66 - 4.52 (m, 2H), 4.31 - 4.21 (m, 1H), 4.19 - 4.10 (m, 1H), 3.74 (d, *J* = 5.5 Hz, 2H), 3.49 - 3.48 (m, 2H), 3.40 (s, 3H), 3.15 - 3.06 (m, 2H), 3.02 - 2.95 (m, 6H), 2.59 - 2.52 (m, 3H), 2.41 - 2.29 (m, 2H), 2.05 - 1.90 (m, 2H), 1.91 - 1.77 (m, 6H), 1.63 - 1.57 (m, 6H), 1.31 - 1.29 (m, 2H), 0.92 - 0.80 (m, 15H).

### Example 12.5: (S)-6-(dimethylamino)-N-(2-(((3-((S)-7-ethyl-7-hydroxyl-8,11-dioxo-7,8,11,13-tetrahydro-10H-[1,3]dioxolo[4,5-g]pyrano[3',4':6,7]indolizino[1,2-b]quinolin-14-yl)propyloxy)methyl)amino)-2-oxoethyl)-2-((S)-3-methyl-2-(6-(4-(2-(methanesulfonyl)pyrimidin-5-yl)-1H-1,2,3-triazol-1-yl)hexanamido)butanamido)hexanamide (DL-05)

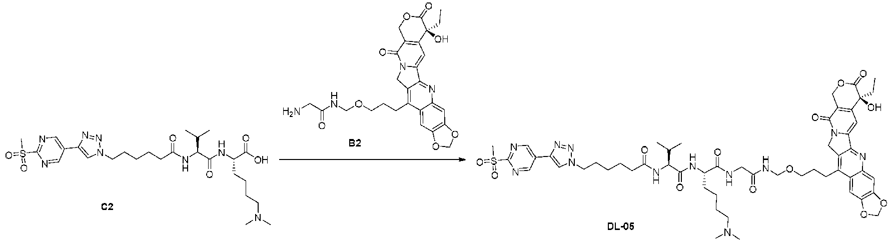

Compound C2 (40 mg, 0.06 mmol) was dissolved in N,N-dimethylformamide (1.5 mL), to which were successively added HBTU (26 mg, 0.06 mmol), B2 (36 mg, 0.06mmol) and DIPEA (66 mg, 0.17 mmol), and then the reaction was stirred for 1 h at room temperature. LCMS detection indicated completion of the reaction. The reaction solution was separated and purified by preparative chromatography (acetonitrile/0.05% trifluoroacetic acid in water), to provide the target compound DL-05 (13.88 mg, TFA salt, yield 16%) as a maize-yellow solid.
LCMS (ESI) [M+H]⁺ = 1113.7;
¹H NMR (400 MHz, DMSO-d6) δ 9.46 (s, 2H), 9.29 (s, 1H, TFA &), 8.92 (s, 1H), 8.63 (t, *J* = 6.6 Hz, 1H), 8.18 (br s, 1H), 8.03 (d, *J* = 7.6 Hz, 1H), 7.81 (d, *J* = 8.3 Hz, 1H), 7.59 (s, 1H), 7.50 (s, 1H), 7.24 (s, 1H), 6.48 (s, 1H), 6.28 (s, 2H), 5.42 (s, 2H), 5.24 (s, 2H), 4.64 - 4.55 (m, 2H), 4.46 (t, *J* = 7.0 Hz, 2H), 4.28 - 4.20 (m, 1H), 4.15 - 4.09 (m, 1H), 3.76 - 3.71 (m, 4H), 3.53 - 3.48 (m, 4H), 3.44 (s, 3H), 3.10 - 3.08 (m, 2H), 2.97 - 2.95 (m, 2H), 2.75 (d, *J* = 4.9 Hz, 6H), 2.20 - 2.09 (m, 2H), 1.92 - 1.83 (m, 5H), 1.68 - 1.65 (m, 1H), 1.63 - 1.50 (m, 5H), 0.90 - 0.74 (m, 9H).

### Exmaple 13 Preparation of anti-Her3 antibody-drug conjugates

### Example 13.1: Preparation of Her3-ADC-07

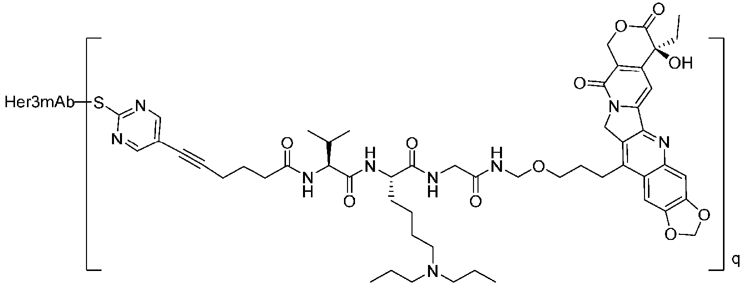

q can be inferred from the data provided in the determination of DAR.

### 13.1.1 Preparation of Her3-ADC-07 (DAR8) sample

30 mg of anti-Her3 antibody 202-2-1 was diluted with diluent (20 mM PB + 105 mM NaCl, pH 7.7), to which was added sodium edetate solution at a final concentration of 5 mM, and then the solution was mixed homogeneously; 20 mmol/L of TCEP solution was added at 4.5-fold molar equivalent of the antibody, and then the solution was mixed homogeneously and allowed to stand at room temperature for 30 min; to the above solution, was added 10 mmol/L of DL-04 dissolved in dimethyl sulfoxide at 10-fold molar equivalent (relative to the antibody), and then the solution was mixed homogeneously, and allowed to stand at room temperature for 2h to obtain a conjugated sample. After completion of the reaction, a 30 KDa ultrafiltration tube was used to transfer the sample into a 10 mM histidine buffer with a pH of 5.5 and remove low molecular weight substances, and finally the sample was concentrated to obtain a solution containing antibody 202-2-1 ADC composition Her3-ADC-07 (DAR8). The average DAR value was measured to be 8.0 by mass spectrometry of Example 14.

According to the mass spectrometry results of Her3-ADC-07 (DAR8), the antibody light chain was conjugated with 0-1 toxin molecule (the percentages of LC, DAR1 are 0%, 100%, respectively), and the heavy chain was conjugated with 0-3 toxin molecules (the percentages of mAb, DAR1, DAR2, DAR3 were 0%, 0%, 0%, 100%, respectively). It can be inferred that the value of q is 8.

### 13.1.2 Preparation of Her3-ADC-07(DAR4) sample

30 mg of anti-Her3 antibody 202-2-1 was diluted with diluent (20 mM PB + 105 mM NaCl, pH 7.7), to which was added sodium edetate solution at a final concentration of 5 mM, and then the solution was mixed homogeneously; 20 mmol/L of TCEP solution was added at 4.5-fold molar equivalent of the antibody, and then the solution was mixed homogeneously and allowed to stand at room temperature for 30 min; to the above solution, 10 mmol/L of DL-04 dissolved in dimethyl sulfoxide was added at 4.8-fold molar equivalent (relative to the antibody), and then the solution was mixed homogeneously, and allowed to stand at room temperature for 2h to obtain a conjugated sample. After completion of the reaction, a 30KDa ultrafiltration tube was used to transfer the sample into a 10 mM histidine buffer with a pH of 5.5 and remove low molecular weight substances, and finally the sample was concentrated to obtain a solution containing antibody 202-2-1 ADC composition Her3-ADC-07 (DAR4). The average DAR value was measured to be 4.5 by mass spectrometry.

According to the mass spectrometry results of Her3-ADC-07 (DAR4), the antibody light chain was conjugated with 0-1 toxin molecules (the percentages of LC, DAR1 were 93.67%, 6.33%, respectively), and the heavy chain was conjugated with 0-3 toxin molecules (the percentages of mAb, DAR1, DAR2, DAR3 were 19.23%, 7.01%, 10.66%, 63.1%, respectively). It can be inferred that q value may be 1, 2, 3, 4, 5, 6, 7, or 8.

### 13.1.3 Preparation of Her3-ADC-07 (DAR6) sample

30 mg of anti-Her3 antibody 202-2-1 was diluted with diluent (20 mM PB + 105 mM NaCl, pH 7.7), to which was added sodium edetate solution at a final concentration of 5 mM, and then the solution was mixed homogeneously; 20 mmol/L of TCEP solution was added at 4.5-fold molar equivalent of the antibody, and then the solution was mixed homogeneously and allowed to stand at room temperature for 30 min; to the above solution 10 mmol/L of DL-04 dissolved in dimethyl sulfoxide was added at 7.2-fold molar equivalent (relative to the antibody), and then the solution was mixed homogeneously, and allowed to stand at room temperature for 2h to obtain a conjugated sample. After completion of the reaction, a 30KDa ultrafiltration tube was used to transfer the sample into a 10 mM histidine buffer with a pH of 5.5 and remove low molecular weight substances, and finally the sample was concentrated to obtain a solution containing antibody 202-2-1 ADC composition Her3-ADC-07 (DAR6). The average DAR value was measured to be 5.8 by mass spectrometry.

According to the mass spectrometry results of Her3-ADC-07 (DAR6), the antibody light chain was conjugated with 0-1 toxin molecules (the percentages of LC, DAR1 were 81.31%, 18.69%, respectively), and the heavy chain was conjugated with 0-3 toxin molecules (the percentages of mAb, DAR1, DAR2, DAR3 were 7.17%, 2.36%, 4.44%, 86.03%, respectively). It can be inferred that q value may be 1, 2, 3, 4, 5, 6, 7 or 8.

### Example 13.2: Preparation of Her3-ADC-05 (DAR4)

Compound DL-04 in Example 13.1.2 was replaced by compoundDL-01 to obtain Her3-ADC-05(DAR4), a coupling product of DL-01 and antibody 202-2-1. The average DAR value was measured to be 4.0 by mass spectrometry.

According to the mass spectrometry results of Her3-ADC-05 (DAR4), the antibody light chain was conjugated with 0-1 toxin molecules (the percentages of LC, DAR1 were 90.89%, 9.11%, respectively), and the heavy chain was conjugated with 0-3 toxin molecules (the percentages of mAb, DAR1, DAR2, DAR3 were 25.1%, 7.17%, 18.19%, 49.53%, respectively). It can be inferred that q value may be 1, 2, 3, 4, 5, 6, 7 or 8.

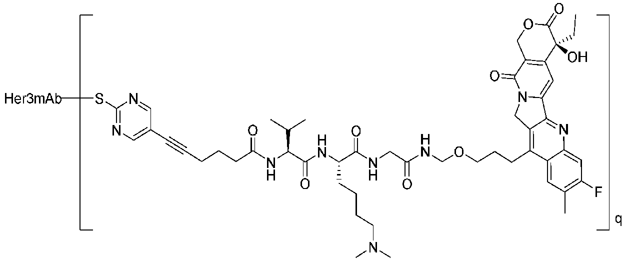

### Example 13.3: Preparation of Her3-C3-ADC-05(DAR4) (DAR4) sample

Compound DL-04 in Example 13.1.2 was replaced by compound DL-01, and anti-Her3 antibody 202-2-1 in Example 13.1.2 was replaced by control antibody 2, to obtain the coupling product Her3-C3-ADC-05(DAR4) of DL-01 and control antibody 2. The average DAR value was measured to be 4.7 by mass spectrometry.

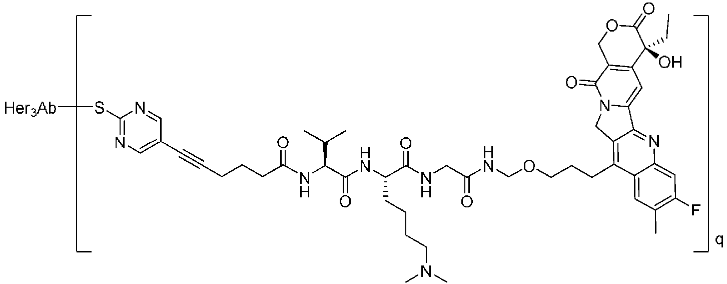

### Example 13.4: Preparation of Her3-ADC-15

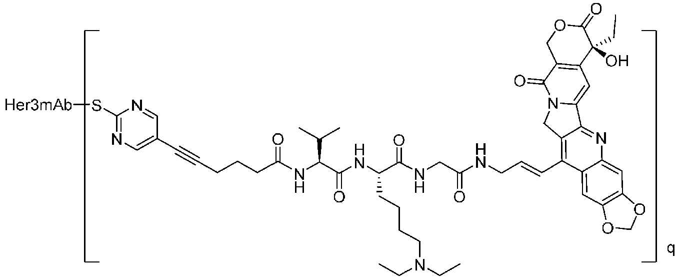

q can be inferred from the data provided in the determination of DAR.

### 13.4.1 Preparation of Her3-ADC-15(DAR8) sample

Compound DL-04 in Example 13.1.1 was replaced by DL-02 to obtain the coupling product Her3-ADC-15(DAR8) of DL-02 and antibody 202-2-1. The average DAR value was measured to be 7.9 by mass spectrometry.

According to the mass spectrometry results of Her3-ADC-15(DAR8), the antibody light chain was conjugated with 0-1 toxin molecule (the percentages of LC, DAR1 were 0%, 100%, respectively), and the heavy chain was conjugated with 0-3 toxin molecules (the percentages of mAb, DAR1, DAR2, DAR3 were 0%, 0%, 6.31%, 93.69%, respectively). It can be inferred that q value may be 3, 4, 5, 6, 7 or 8.

### 13.4.2 Preparation of Her3-ADC-15(DAR2) sample

Compound DL-04 in Example 13.1.1 was replaced by two molar equivalents of DL-02, to obtain the coupling product Her3-ADC-15(DAR2) of DL-02 and antibody 202-2-1. The average DAR value was measured to be 2.0 by mass spectrometry.

According to the mass spectrometry results of Her3-ADC-15(DAR2), the antibody light chain was conjugated with 0-1 toxin molecule (the percentages of LC, DAR1 were 93.82%, 6.18%, respectively), and the heavy chain was conjugated with 0-3 toxin molecules (the percentages of mAb, DAR1, DAR2, DAR3 were 61.16%, 5.77%, 11.02%, 22.05%, respectively). It can be inferred that q value may be 1, 2, 3, 4, 5, 6, 7 or 8.

### Example 13.5: Preparation of Her3-ADC-23(DAR4)

Compound DL-04 in Example 13.1.2 was replaced by DL-03 to obtain the coupling product Her3-ADC-23(DAR4) of DL-03 and antibody 202-2-1. The average DAR value was measured to be 4.2 by mass spectrometry.

According to the mass spectrometry results of Her3-ADC-23(DAR4), the antibody light chain was conjugated with 0-1 toxin molecule (the percentages of LC, DAR1 were 91.28%, 8.72%, respectively), and the heavy chain was conjugated with 0-3 toxin molecules (the percentages of mAb, DAR1, DAR2, DAR3 were 19.24%, 8.67%, 21.97%, 50.13%, respectively). It can be inferred that q value may be 1, 2, 3, 4, 5, 6, 7 or 8.

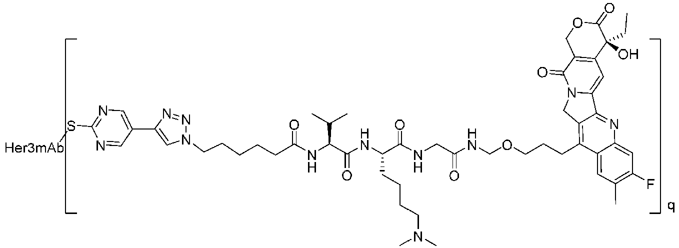

### Example 13.6: Preparation of Her3-C3-ADC-23(DAR4) sample

Compound DL-04 in Example 13.1.2 was replaced by DL-03, and Her3 antibody 202-2-1 in 13.1.2 was replaced by control antibody 2, to obtain the coupling product Her3-C3-ADC-23(DAR4) of DL-03 and control antibody 2. The average DAR value was measured to be 4.9 by mass spectrometry.

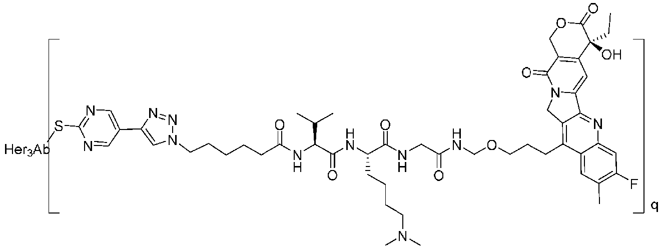

### Example 13.7: Preparation of Her3-C3-ADC-21(DAR4) sample

With reference to CN104755494B, the following drug-linker compound was prepared,

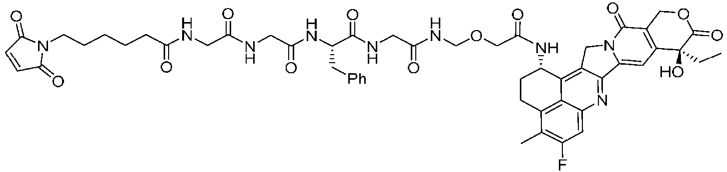

Compound DL-04 in Example 13.1.2 was replaced by the above drug-linker compound, and anti-Her3 antibody 202-2-1 in Example 13.1.2 was replaced by control antibody 2, to obtain Her3-C3-ADC-21(DAR4). The average DAR value was measured to be 4.4 by mass spectrometry.

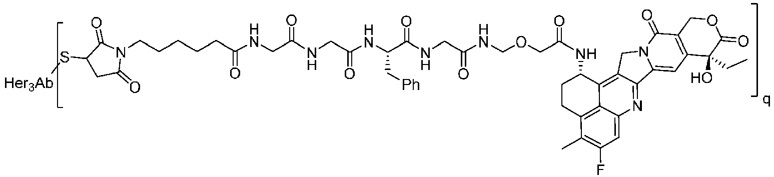

### Example 13.8: Preparation of Her3-C3-ADC-21(DAR8) sample

Compound DL-04 in Example 13.1.1 was replaced by the drug-linker compound in example 13.7, and anti-Her3 antibody 202-2-1 in Example 13.1.1 was replaced by control antibody 2, to obtain Her3-C3-ADC-21(DAR8). The average DAR value was measured to be 9.5 by mass spectrometry.

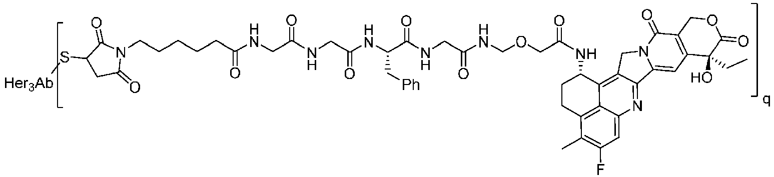

### Example 13.9: Preparation of Her3-ADC-21(DAR4) sample

Compound DL-04 in Example 13.1.2 was replaced by the drug-linker compound in example 13.7, to obtain the coupling product Her3-ADC-21(DAR4) of the above drug-linker compound and 202-2-1 antibody. The average DAR value was measured to be 4.1 by mass spectrometry.

According to the mass spectrometry results of Her3-ADC-21(DAR4), the antibody light chain was conjugated with 0-1 toxin molecule (the percentages of LC, DAR1 were 100%, 0%, respectively), and the heavy chain was conjugated with 0-3 toxin molecules (the percentages of mAb, DAR1, DAR2, DAR3 were 20.57%, 8.01%, 17.3%, 54.12%, respectively). It can be inferred that q value may be 1, 2, 3, 4, 5 or 6.

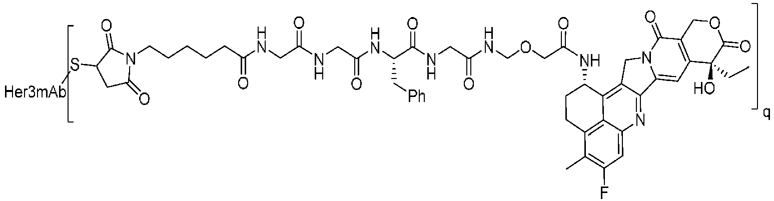

### Example 13.10: Preparation of Her3-ADC-33

With reference to examples 4-1 of WO2019195665, the following drug-linker compound was prepared,

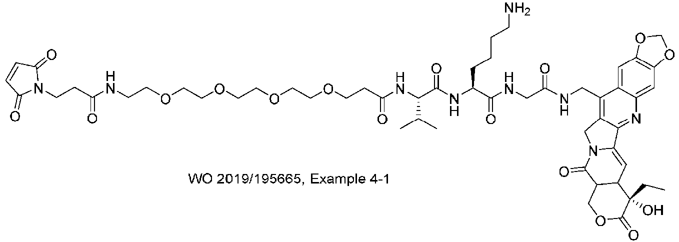

Compound DL-04 in Example 13.1.2 was replaced by the above drug-linker compound, to obtain the coupling product Her3-ADC-33(DAR4) of the above drug-linker compound and antibody 202-2-1. The average DAR value was measured to be 5.6 by mass spectrometry.

According to the mass spectrometry results of Her3-ADC-33(DAR4), the antibody light chain was conjugated with 0-1 toxin molecule (the percentages of LC, DAR1 were 49%, 51%, respectively), and the heavy chain was conjugated with 0-3 toxin molecules (the percentages of mAb, DAR1, DAR2, DAR3 were 18.01%, 3.25%, 9.13%, 69.62%, respectively). It can be inferred that q value may be 1, 2, 3, 4, 5, 6, 7 or 8.

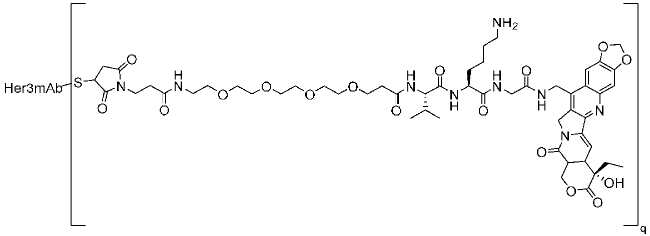

### Example 13.11: Preparationof IgG-ADC-07(DAR8)

Antibody 202-2-1 in Example 13.1.1 was replaced by IgG1 isotype antibody (anti-Hen Egg Lysozyme antibody from MediLink Therapeutics (Suzhou) Co., Ltd.), to obtain the coupling product IgG1-ADC-07 of DL-04 and antibody IgG1. The average DAR value was measured to be 8.0 by mass spectrometry. 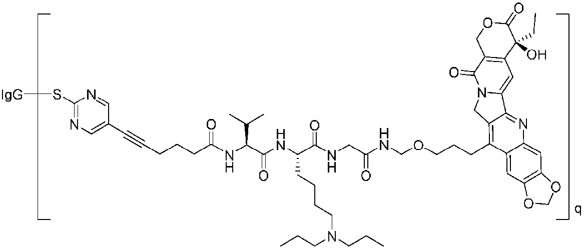 Note: Her3mAb and Her3Ab both refer to an anti-Her3 antibody or antigen-binding fragment thereof, and in particular, in the above examples, Her3mAb is antibody 202-2-1, and Her3Ab is the control antibody 2.

### Example 13.12: Preparation of Her3-ADC-08

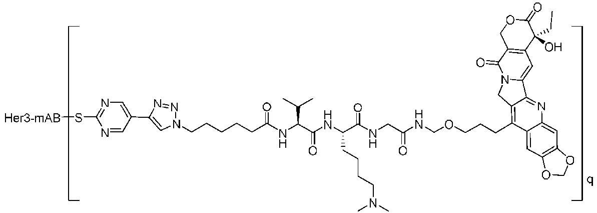

q can be inferred from the data provided in the determination of DAR.

### (1) Preparation of Her3-ADC-08(DAR6) sample

Compound DL-04 in Example 13.1.3 "Preparation of Her3-ADC-07(DAR6) sample" was replaced by DL-05, to obtain the coupling product Her3-ADC-08(DAR6) of DL-05 and antibody 202-2-1. The average DAR value was measured to be 4.9 by mass spectrometry. According to the mass spectrometry results of Her3-ADC-08(DAR6), the antibody light chain was conjugated with 0-1 toxin molecule (LC, DAR1 percentages were 66.58%, 33.42%, respectively), and the heavy chain was conjugated with 0-3 toxin molecules (the percentages ofmAb, DAR1, DAR2, DAR3 were 16.51%, 11.72%, 16.63%, 55.59%, respectively). It can be inferred that q value may be 1,2, 3, 4, 5, 6, 7 or 8.

### (2) Preparation of Her3-ADC-08(DAR2) sample

Compound DL-04 in Example 13.1.1 was replaced by two molar equivalents of DL-05, to obtain the coupling product Her3-ADC-08(DAR2) of DL-05 and antibody 202-2-1. The average DAR value was measured to be 1.9 by mass spectrometry. According to the mass spectrometry results of Her3-ADC-08(DAR2), the antibody light chain was conjugated with 0-1 toxin molecule (the percentages of LC, DAR1 were 96.61%, 3.39%, respectively), and the heavy chain was conjugated with 0-3 toxin molecules (the percentages of mAb, DAR1, DAR2, DAR3 were 58.72%, 9.05%, 15.41%, 16.83%, respectively). It can be inferred that q value may be 1, 2, 3, 4, 5, 6, 7 or 8.

### Example 14 Determination of DAR values of conjugated samples by mass spectrometry

Detection: The analysis of the molecular weight and the DAR value for Her3-ADC-07(DAR8) was performed by LC-MS, using the following conditions

Chromatographic conditions:
Column: PLRP-S, 2.1 * 50 mm, 5 µm;
Mobile phase A: 0.1% PA/H₂O; mobile phase B: 0.1% FA/ACN
Column temperature: 30°C; sample chamber temperature: 8°C; flow rate: 0.6 ml/min; injection volume: 2 µl

| | | | | | |
|---|---|---|---|---|---|
| Time (min) | 1 | 5 | 5.1 | 7 | 10 |
| Mobile phase A | 90 | 40 | 10 | 90 | 90 |
| Mobile phase B | 10 | 60 | 90 | 10 | 10 |

Sample processing: to 50 µg of sample, was added 1M DTT (2 µl), to which was added ultrapure water to 50 µl, and the solution was diluted to a concentration of about 1.0 mg/ml, then mixed homogeneously, followed by reducing at room temperature for 30 min.

### LC/MS model: Agilent 1290-6545XT Q-TOF

MS conditions: Gas temp: 320°C, Drying Gas: Nebulizer: 35 psi; Sheath Gas Temp: 350°C; sheath Gas flow: 11 l/min: m/z 500-3000.

The results were shown below:

**Table 6. Theoretical and experimental molecular weights.**

| Peptide chain | | mAb | DAR1 | DAR2 | DAR3 |
|---|---|---|---|---|---|
| LC | Theoretical value | 23243.8 | 24261.2 | 25278.6 | 26296 |
| | Experimental value | Not detected | 24258 | Not detected | Not detected |
| HC | Theoretical value | 50598.6 | 51616 | 52633.4 | 53650.8 |
| | Experimental value | Not detected | Not detected | Not detected | 53646 |

In Table 6, mAb represents an unconjugated antibody; LC represents a light chain of the antibody; HC represents a heavy chain of the antibody; DAR1 represents a conjugate containing a light or heavy chain conjugated with one toxin molecule; DAR2 represents a conjugate containing a light or heavy chain conjugated with 2 toxin molecules; DAR3 represents a conjugate containing a light or heavy chain conjugated with 3 toxin molecules; where the theoretical molecular weight of the monoclonal antibody is calculated as G0F glycoform. Hereinafter, mAb, LC, HC, DAR1, DAR2, and DAR3 are defined as above.

The detection results shown, the light chain of the antibody in Her3-ADC-07(DAR8) was conjugated with 0-1 toxin molecule (the percentages of LC and DAR1 were 0% and 100%, respectively), while the heavy chain was conjugated with 0-3 toxin molecules (the percentages of mAb, DAR1, DAR2, DAR3 were 0%, 0%, 0%, 100%, respectively), and thus the antibody-drug coupling ratio (DAR value) of Her3-ADC-07(DAR8) was calculated to be 8.0.

### Example 15 In vitro killing activity assay of anti-Her3 ADC drugs on tumor cell lines

### A. Determination of the inhibitory activity of Her3 ADC against PC-9 and NCI-H358 tumors

PC-9 tumor cells (human lung adenocarcinoma, Nanjing COBIOER Bioscience Co., Ltd.) and NCI-H358 tumor cells (human non-small cell lung cancer, ATCC, CRL-5807) were selected to detect the killing activity of Her3-ADC-07 on the tumor cell lines with endogenous expression of human Her3. The specific experimental steps were as follows: PC-9 cells and NCI-H358 cells were digested, collected by centrifugation, resuspended in 1640 + 4% FBS culture medium to 50000 cells/mL, and then plated to a 96 well plate at 100 µL/well; ADC molecules were gradiently diluted with 1640 basic culture medium in 3-fold dilutions starting from the final concentration 150 µM, with a total of 11 dilution points, which were added to the corresponding wells at 100 µL/well, and the final serum concentration was 2%; the plate was incubated for 120 h in a 37 °C, 5% CO₂ incubator; CCK8 (Rhinogen) was added to each well at 20 µL/well, and then the plate was incubated for 0.5-2.5 h in a 37 °C, 5% CO₂ incubator. The plate was read on a microplate reader (MD, model ABS Plus) at 450 nm every half hour, and then the data were imported into Graphpad Prism for curve fitting.

The experimental results were shown in Figure 6A and 6B. Her3-ADC-07 had a killing activity against PC-9 tumor cells with an EC50 value of 49.26 nM, as well as against NCI-H358 tumor cells with an EC50 value of 183.3 nM.

### B. Determination of the inhibitory activity of Her3 ADC against KYSE520 tumor

KYSE520 (human esophageal squamous cell carcinoma tumor cell, ATCC) were digested by conventional methods using trypsin, and cells were collected and the cells in tube were counted, and then resuspended in the corresponding assay media (containing 2% FBS), which was added to a 96-well plate at 2000-5000 cells/well. 100 µL ADC diluted with the media containing 2% FBS (as shown in table 7) was added to the 96-well plate, the initial concentration was 50 µg/ml, followed by 3-fold dilutions (11 concentration gradients). The plate was incubated for 5 days at 37 °C under 5% CO₂. Then, 20 µL of CCK8 reagent was added to each well, and the reaction was carried out for 1-4 h. The plate was read on a microplate reader (detection wavelength was 450 nm). The cells used in experiment and the experimental results were shown in Table 7.

**Table 7**

| ADC sample | KYSE520 (EC50, ng/mL) |
|---|---|
| Her3-ADC-05 (DAR4) | 19.01 |
| Her3-C3-ADC-05(DAR4) | 32.94 |

The experimental results indicated that both Her3-ADC-05 and Her3-C3-ADC-05 had strong killing effects on tumor cells.

### C. Determination of the inhibitory activity of Her3 ADC against NCI-H358 tumor

The assay method of *In vitro* cell activity: NCI-H358 tumor cells were digested by conventional methods using trypsin, cells were collected and the cells in tube were counted, and then resuspended in the corresponding assay media (containing 2% FBS), which was added to a 96-well plate at 2000-5000 cells/well. 100µL ADC diluted with the media containing 2% FBS was added to the 96-well plate, the initial concentration was 150µg/ml, followed by 3-fold dilutions (11 concentration gradients). The plate was incubated for 7 days at 37 °C under 5% CO₂. Then, 20µL of CCK8 reagent was added to each well, and the reaction was carried out for 2-6 h. The plate was read on a microplate reader (detection wavelength was 450 nm). Each group of samples and the experimental results were shown in Table 8.

**Table 8**

| Test group No. | ADC sample | NCI-H358 (EC50, ng/mL) |
|---|---|---|
| 1 | Her3-ADC-05(DAR4) | 47742 |
| | Her3-C3-ADC-05(DAR4) | 65109 |
| | Her3-C3-ADC-21(DAR4) | 76365 |
| 2 | Her3-ADC-23(DAR4) | 49330 |
| | Her3-C3-ADC-23(DAR4) | weak |
| 3 | Her3-ADC-15(DAR2) | 5572 |
| | Her3-ADC-15(DAR8) | 2562 |
| | Her3-ADC-33(DAR4) | 50774 |

The experimental results of this example indicate that the ADCs with various DAR values in the present disclosure all had killing effects on tumor cells.

### Example 16 In vivo activity assay of anti-Her3 antibody drug conjugates and IgG-ADC

16.1 Efficacy assay of anti-Her3 antibody drug conjugate on NCI-H358 xenograft tumor The *in vivo* efficacy was demonstrated by evaluating the antitumor effect of an anti-Her3 antibody ADC drug in subcutaneously transplanted tumor model of human non-small cell lung cancer cell line NCI-H358 cells (available from ATCC, about 10% of Her3 expression positive rate) of Balb/c Nude mice.

The specific steps are as follows: Balb/c nu nude mice, female, 5-6 weeks of age, were purchased from Vital River Laboratory Animal Co., Ltd. NCI-H358 cells were cultured in a 15 cm diameter petri dish with 1640 medium (containing 10% FBS), digested by trypsin-EDTA when the confluence reached about 80-90%, washed twice with PBS, and then centrifuged and resuspended in pre-cooled PBS. Cells were counted by a cell counter, and then the cells were diluted with PBS to a cell concentration of 5×10⁷ cells/ml.After adapting to the laboratory environment for 7 days, Balb/c nu mice were subcutaneously inoculated with NCI-H358 cells in the right rib, with an inoculation amount of 5×10⁶ cells/mouse and an inoculation volume of 0.2 ml (containing 50% Matrigel). When the tumor grew to about 300 mm³, mice were randomly grouped according to the tumor size, 5 mice for each group, said groups were: anti-Hen Egg lysozyme human IgG1 isotype antibody (negative control, from MediLink Therapeutics (Suzhou) Co., Ltd.) group, IgG1-ADC-07(DAR8), Her3-C3-ADC-21(DAR4), Her3-ADC-21(DAR4) and Her3-ADC-07(DAR8) dose groups, respectively. All samples were injected via the tail vein, once a week, for a total of 3 weeks. Tumor volume and body weight of mice were observed and measured periodically after administration. The relative tumor proliferation rate, T/C(%), is the percentage of the relative tumor volume or tumor weight of the treatment group to those of control group at a specific time point. Relative tumor inhibition rate, TGI (%), was calculated as the formula: TGI% = (1-T/C) × 100% (T and C are the relative tumor volume (RTV) or tumor weight (TW) of the treatment and control groups at a specific time point, respectively).

The results are shown in Table 9 and Figure 7.

**Table 9. Effect of Her3-ADC-07 on subcutaneously xenograft of human non-small cell lung cancer NCI-H358 in nude mice.**

| Group | Mean tumor volume (mm³) | Mean tumor volume (mm³) | T/C (%) | Tumor inhibition rate TGI(%) | *P* value (D29) |
|---|---|---|---|---|---|
| | D1 | D29 | D29 | D29 | |
| IgG 1 | 309.0±72.0 | 1425.0±241.2 | - | - | - |
| 5mg/kg | | | | | |
| IgG1-ADC-07(DAR8) | 309.4±53.4 | 725.2±415.1 | 50.83 | 49.17 | 0.203 |
| 5mg/kg | | | | | |
| Her3-C3-ADC-21(DAR4) | 308.8±67.7 | 1080.8±741.8 | 75.89 | 24.11 | 0.996 |
| 1 mg/kg | | | | | |
| Her3-ADC-21(DAR4) | 309.6±60.6 | 346.4±123.8** | 24.26 | 75.74 | 0.002 |
| 5 mg/kg | | | | | |
| Her3-ADC-21(DAR4) | 309.6±58.6 | 1000.6±184.1 | 70.08 | 29.92 | 0.214 |
| 1 mg/kg | | | | | |
| Her3-ADC-07(DAR8) | 309.4±43.4 | 205.4±56.7** | 14.40 | 85.60 | 0.003 |
| 5mg/kg | | | | | |
| Her3-ADC-07(DAR8) | 309.6±47.1 | 339.0±95.8** | 23.74 | 76.26 | 0.003 |
| 1mg/kg | | | | | |

Based on the experimental results, compared with IgG1 group, both 5 mg/kg and 1 mg/kg of Her3-ADC-07(DAR8) significantly inhibited the tumor growth of NCI-H358 human non-small cell lung cancer xenograft model, and presented a dose gradient trend. The TGI value of Her3-ADC-07 1mg/kg group was 76.26%, slightly greater than the TGI value 75.74% of Her3-ADC-21 5 mg/kg group. The TGI value of Her3-ADC-21 1 mg/kg group was 29.92%, slightly greater than the TGI value 24.11% of Her3-C3-ADC-21 1 mg/kg group.

In addition, the results of animal body weight showed that the animals in each test group tolerated well during the administration period and the recovery period.

### 16.2. Efficacy assay I of anti-Her3 antibody drug conjugate on SW480 xenograft tumor

The present disclosure demonstrated the *in vivo* efficacy by evaluating the antitumor effect of the anti-Her3 antibody ADC drug in subcutaneously transplanted tumor model of human colon cancer cell line SW480 cells (available from ATCC, about 30% of Her3 expression positive rate) of Balb/c Nude mice. The specific steps are as follows: 36 Balb/c nu nude mice, female, 5-6 weeks of age, were purchased from Vital River Laboratory Animal Technology Co., Ltd.. SW480 cells were cultured in a 15 cm diameter petri dish with 1640 medium (containing 10% FBS), digested with trypsin-EDTA when the confluence reached about 80-90%, washed twice with PBS, and then centrifuged and resuspended in pre-cooled PBS. Cells were counted by a cell counter, and then the cells were diluted with PBS to a concentration of 5×10⁷ cells/ml. After adapting to the laboratory environment for 7 days, Balb/c nu mice were subcutaneously inoculated with SW480 cells in the right rib, with an inoculation amount of 5×10⁶ cells/mouse and an inoculation volume of 0.2 ml (containing 50% Matrigel). When the tumor grew to about 250 mm³, mice were randomly grouped according to the tumor size, 5 mice for each group, said groups were: anti-Hen lysozyme human IgG1 isotype control antibody (negative control, from MediLink Therapeutics (Suzhou) Co., Ltd.) group, IgG1-ADC-07 and Her3-ADC-07 dose groups, respectively. All samples were injected via the tail vein, once a week, for a total of 3 weeks. Tumor volume and body weight of mice were observed and measured periodically after administration.
1) The relative tumor proliferation rate, T/C (%), is the percentage of the relative tumor volume or tumor weight of the treatment group to those of control group at a specific time point.
2) Relative tumor inhibition rate, TGI (%), was calculated as the formula: TGI% = (1-T/C) × 100% (T and C are the relative tumor volume (RTV) or tumor weight (TW) of the treatment and control groups at a specific time point, respectively).

The specific results are shown in Table 10 and Figure 8. According to the experimental results, both 10 mg/kg and 3 mg/kg doses of Her3-ADC-07 had a significant inhibitory effect on the tumor growth of SW480 human colorectal cancer xenograft model, and presented a dose gradient trend.

**Table 10. Efficacy results of SW480 + Balb/c Nude mouse model.**

| Group | Mean tumor volume (mm³) | Mean tumor volume (mm3) | T/C (%) | Tumor inhibitio n rate TGI (%) | *P* value D18 |
|---|---|---|---|---|---|
| | D1 | D18 | D18 | D18 | |
| IgG1 10 mg/kg | 269.80±32.42 | 2437.80±378.82 | - | - | - |
| IgG1-ADC-07(DAR8) 10 mg/kg | 270.60±33.13 | 1129.40±856.17** | 46.2 | 53.8 | 0.007 |
| Her3-ADC-07(DAR8) 10 mg/kg | 269.20±31.17 | 164.80±39.81*** | 6.8 | 93.2 | 0.000 |
| Her3-ADC-07(DAR8) 3 mg/kg | 270.20±31.40 | 939.00±744.80** | 38.5 | 61.5 | 0.002 |
| Her3-ADC-07 (DAR8) 1 mg/kg | 271.60±38.35 | 2708.80±535.14 | 110.4 | -10.4 | 0.876 |

| | | | | | |
|---|---|---|---|---|---|
| 16.3. Efficacy assay II of anti-Her3 antibody drug conjugate on SW480 xenograft tumor | | | | | |

The antitumor effects of IgG1, IgG1-ADC-07(DAR8), Her3-ADC-07(DAR8) and Her3-C3-ADC-21(DAR8) in a transplanted tumor model of human colon cancer cell lines SW480 (available from ATCC, about 30% of Her3 expression positive rate) were determined by referring to the same model and method as Example 16.2.

The specific results are shown in Table 11 and Figure 9.

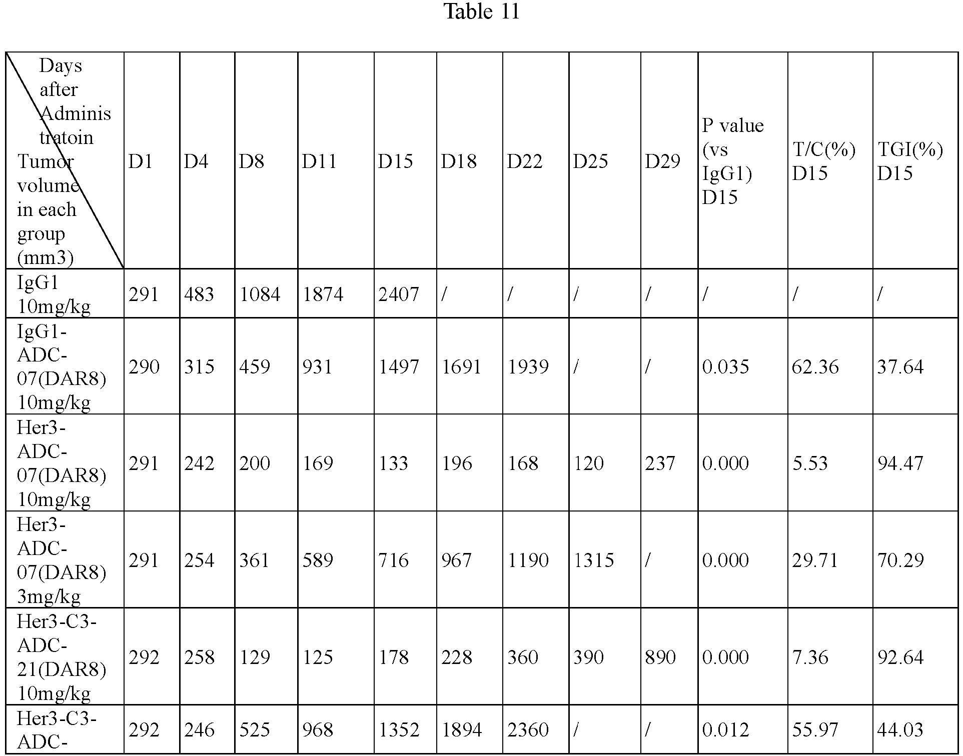

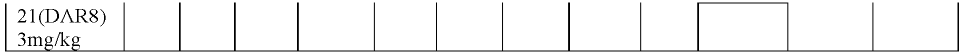

According to the experimental results, both 10 mg/kg and 3 mg/kg doses of Her3-ADC-07 had a significant inhibitory effect on the tumor growth of SW480 human colorectal cancer xenograft model, and presented a dose gradient trend.

Unexpectedly, the tumor-inhibitory effect of Her3-ADC-07(DAR8) 3 mg/kg group was significantly stronger than that of Her3-C3-ADC-21(DAR8) 3 mg/kg group, and the tumor-inhibitory effect of Her3-ADC-07(DAR8) 10 mg/kg group was significantly stronger than that of Her3-C3-ADC-21(DAR8) 10 mg/kg group.

In addition, the results of animal body weight showed that the animals in each test group tolerated well during the administration period and the recovery period.

### Example 17. Her3- antibody drug conjugate plasma stability experiment

The stability of Her3-ADC-07(DAR8) in plasma was evaluated by measuring the release of bioactive molecule A1 in human plasma in which Her3-ADC-07(DAR8) was incubated.

Her3-ADC-07 (DAR8) was respectively incubated in triplicate with filtered and sterilized plasma of mouse, rat, dog, monkey, and human. The incubation was performed at 37 °C under 5% CO₂, with a final concentration of 50 µg/mL for Her3-ADC-07 (DAR8) and a total incubation time of 504 h. During the incubation process, samples were taken at time points 0 h, 24 h, 72 h, 168 h, 336 h, and 504 h, and a methanol solution containing internal standard (0.5 µM tolbutamide) and 0.1% formic acid was added to terminate the reaction. The test samples were analyzed using UPLC-MS/MS under the following conditions:

| | | | | |
|---|---|---|---|---|
| Analyte | A1 | | | |
| Detection method | LC-MS/MS-6500-001 | | | |
| Internal standard | Tolbutamide | | | |
| MS conditions | Positive ion, ESI | | | |
| Mobile phase | A: 0.1%FA in H2O | | | |
| | B: 0.1%FA in ACN | | | |
| Column | Waters, BEH C18, 1.7 µm, 2.1*50 mm | | | |
| Flow rate | 0.5 mL/min | | | |
| Liquid chromatograph y conditions | Time (min) | | Pump B (%) | |
| | 0.4 | | 5 | |
| | 1.8 | | 95 | |
| | 2.2 | | 95 | |
| | 2.21 | | 5 | |
| | 2.5 | | termination | |
| Detection | Compound | *m*/*z* | De-clustering voltage (v) | collision energy (v) |
| | Tolbutamide | 271.0-172.0 | 50 | 30 |
| | A1.9 | 451.1-407.2 | 50 | 25 |

And the concentration and release percentage of the bioactive molecule A1 was calculated according to the following formula: Release percentage = the concentration of A1 at the measured time/the theoretical maximum release concentration;

The theoretical maximum release concentration = ADC incubation concentration/ADC drug molecular weight × DAR × the molecular weight of biologically active molecule Al.

At the test concentration of 50 µg/mL, the stability results of Her3-ADC-07 (DAR8) in mouse, rat, dog, monkey, and human plasma are shown in Table 12.

**Table 12. A 1 release of Her3-ADC-07 (DAR8) in plasma of mice, rats, dogs, monkeys, and humans.**

| **Species** | **Release percentage of bioactive molecule A1 (mean ± SD %)** | | | | | |
|---|---|---|---|---|---|---|
| | **0 h** | **24 h** | **72 h** | **168 h** | **336 h** | **504 h** |
| Mice | 0.0272 ± 0.00635 | 0.0805 ± 0.00540 | 0.178 ± 0.00528 | 0.286 ± 0.0124 | 0.576 ± 0.00526 | 0.726 ± 0.0301 |
| Rats | 0.0240 ± 0.00336 | 0.0774 ± 0.00668 | 0.148 ± 0.0155 | 0.236 ± 0.00507 | 0.424 ± 0.00974 | 0.497 ± 0.00956 |
| Dogs | 0.0210 ± 0.00191 | 0.0330 ± 0.00144 | 0.0751 ± 0.00587 | 0.191 ± 0.0104 | 0.419 ± 0.0384 | 0.521 ± 0.0480 |
| Monkeys | 0.0324 ± 0.000840 | 0.0914 ± 0.00349 | 0.193 ± 0.0119 | 0.330 ± 0.0187 | 0.399 ± 0.0122 | 0.474 ± 0.0117 |
| Humans | <0.0260* | 0.0460 ± 0.00411 | 0.0991 ± 0.00132 | 0.170 ± 0.00997 | 0.332 ± 0.0238 | 0.372 ± 0.0201 |

| | | | | | | |
|---|---|---|---|---|---|---|
| *: If the concentration in one of the parallel tests is lower than the lower limit of quantification, the release percentage is reported as "<maximum value". | | | | | | |

The experimental results indicated that at the test concentration of 50 µg/mL, Her3-ADC-07 (DAR8) was incubated in the plasma of mice, rats, dogs, monkeys, and humans for 504 h, and the release percentages ofbioactive molecule A1 were 0.726%, 0.497%, 0.521%, 0.474%, and 0.372%, respectively. The stability of Her3-ADC-07 (DAR8) in the plasma of mice, rats, dogs, monkeys, and humans was good. After incubation for 504 h (21 days) *in vitro,* the release of A1 was less than 1% of the theoretical maximum release concentration.

### Example 18. Safety evaluation of Her3 antibody-drug conjugate

The therapeutic window was evaluated according to the highest non-severe toxic dose (HNSTD) in the toxicological testing and the minimum effective dose (MED) in the pharmacodynamic testing. The therapeutic window of Her3-ADC-07 (DAR8) was calculated according to the ratio of the *in vivo* exposures at the HNSTD and MED doses.

### 18.1 GLP toxicology experiment

In the GLP toxicology experiment of Her3-ADC-07(DAR8) for 6 consecutive weeks followed by 4 weeks of recovery, each dose group included 10 animals, half male and half female, and Her3-ADC-07(DAR8) was repeatedly administered to cynomolgus monkeys via intravenous infusion at doses of 0, 3, 10, and 20 mg/kg, once every 3 weeks, for 3 successive times. Then the animal was allowed to recover for 4 weeks after administration. The toxicity of Her3-ADC-07(DAR8) was evaluated in cynomolgus monkeys, and the concentration of ADC in serum samples was detected in toxicokinetic (TK) studies.

The GLP toxicity experiment results showed that the highest non-severe toxicity dose was 10 mg/kg, and at this dose, there were no significant abnormalities in the clinical observation, body weight, blood biochemistry, coagulation, etc. of the animals.

Compared with similar ADC products of Her3-ADC-07(DAR8), Patritumab Deruxtecan is the ADC targeting HER3 of Daiichi Sankyo, and the dose of toxicological experiment in cynomolgus monkeys was 0 mg/kg, 3 mg/kg, 10 mg/kg, and 30 mg/kg, which was administered once every three weeks for five consecutive administrations. The results showed that at a dose of ≥ 3 mg/kg, a decrease in reticulocytes, lymphocytes, and platelets was observed; at a dose of ≥ 10 mg/kg, transferases increased; there was no significant abnormality in body weight at all doses; the toxic target organs were thymus, bone marrow, and skin (Hashimoto Y, et al. Clin Cancer Res. 2019 Dec 1; 25 (23): 7151-7161).

It should be noted that Patritumab Deruxtecan exhibited thrombocytopenia at a dose of ≥ 3 mg/kg, and in its clinical studies, thrombocytopenia was one of the dose-limiting toxicities in dose escalation studies (Jänne PA, et al. Cancer Discov. 2022 Jan; 12(1): 74-89.), and was also one of the most common (30%) treatment emergent adverse events (TEAEs) in multicenter clinical studies (Yu-H, et al., Abstract # MA21.06, WCLC 2019). By comparison, it was found that Her3-ADC-07 (DAR8) had not shown significant platelet count abnormalities at the highest dose of 20 mg/kg, and no toxic reactions were observed in important organs such as liver, kidney, and lung at the highest dose of 20 mg/kg.

### 18.2 Nude mouse PK/PD experiment

NCI-H358 tumor-bearing nude mice were given 0.3 mg/kg or 3 mg/kg of Her3-ADC-07 (DAR8) via a single tail vein injection, and the serum and tumors were collected at 0 h, 3 h, 6 h, 24 h, 48 h, 96 h, 168 h, and 336 h (5 animals at each time point). The tumor volume and weight were analyzed, while the ADC concentration in the serum was measured.

After a single intravenous injection of 0.3 mg/kg or 3 mg/kg of Her3-ADC-07 (DAR8) into NCI-H358 tumor bearing mice, tumor growth was inhibited at a dose of ≥ 0.3 mg/kg. It showed that the lowest effective dose of Her3-ADC-07 (DAR8) in this experiment was 0.3 mg/kg.

The ratio of the AUC₀₋₃₃₆ₕ or Cₘₐₓ under the highest non-severe toxic dose of cynomolgus monkeys to the AUC₀₋₃₃₆ₕ or Cₘₐₓ under the lowest effective dose of CDX mice was defined as the therapeutic index (TI), and the therapeutic index of each parameter was as follows:

| | **Therapeutic index** |
|---|---|
| **AUC₀₋₃₃₆ₕ(µg/mL*h)** | 83.2 |
| **Cₘₐₓ (µg/mL)** | 194.4 |

To sum up, Her3-ADC-07 (DAR8) was well tolerated at a dose of 10 mg/kg (HNSTD) in cynomolgus monkeys, and the therapeutic index was close to 100 times. Moreover, no platelet decline occurred at this dose, and no toxicity in important organs such as lung, liver, kidney, etc. was observed. The above results indicated that Her3-ADC-07 (DAR8) had high safety in long-term use.

Although specific embodiments of the present disclosure had been described in detail, persons skilled in the art would appreciate that in light of all the teachings that had been published, various modifications and changes in details can be made and they all will be within the scope of the present disclosure. The claimed scope of the present disclosure was given by the appended claims and any equivalents thereof.

## Claims

1. An antibody or antigen-binding fragment thereof that binds Her3, said antibody or antigen-binding fragment comprises the complementarity determining regions CDRs as shown below:
(a) HCDR1 or a variant of its sequence, HCDR2 or a variant of its sequence, and HCDR3 or a variant of its sequence as comprised in the heavy chain variable region VH as shown in SEQ ID NO: 23, 25, or 27; and/or
(b) LCDR1 or a variant of its sequence, LCDR2 or a variant of its sequence, and LCDR3 or a variant of its sequence as comprised in the light chain variable region VL as shown in SEQ ID NO: 24, 26, or 28;
preferably, the variant of sequence is a CDR which has one or several amino acid substitutions, deletions or additions, e.g., 1, 2, or 3 amino acid substitutions, deletions or additions compared with the CDR from which it is derived; more preferably, the substitution(s) are conservative substitution(s).

2. The antibody or antigen-binding fragment thereof of claim 1, wherein the antibody or antigen-binding fragment thereof comprises:
(1) VH and/or VL, wherein defined by the Kabat numbering system:
(a) the VH comprises: HCDR1 consisting of the sequence of SEQ ID NO: 1, HCDR2 consisting of the sequence of SEQ ID NO:2, HCDR3 consisting of the sequence of SEQ ID NO:3; and/or,
the VL comprises: LCDR1 consisting of the sequence of SEQ ID NO:7, LCDR2 consisting of the sequence of SEQ ID NO:8, LCDR3 consisting of the sequence of SEQ ID NO:9;
(b) the VH comprises: HCDR1 consisting of the sequence of SEQ ID NO: 12, HCDR2 consisting of the sequence of SEQ ID NO: 13, HCDR3 consisting of the sequence of SEQ ID NO: 14; and/or,
the VL comprises: LCDR1 consisting of the sequence of SEQ ID NO: 18, LCDR2 consisting of the sequence of SEQ ID NO: 19, LCDR3 consisting of the sequence of SEQ ID NO:20;
or,
(2) VH and/or VL, wherein defined by the IMGT numbering system:
(a) the VH comprises: HCDR1 consisting of the sequence of SEQ ID NO: 4; HCDR2 consisting of the sequence of SEQ ID NO: 5; HCDR3 consisting of the sequence of SEQ ID NO: 6; and/or,
the VL comprises: LCDR1 consisting of the sequence of SEQ ID NO: 10; LCDR2 consisting of a sequence of AAS; LCDR3 consisting of the sequence of SEQ ID NO: 9;
(b) the VH comprises: HCDR1 consisting of the sequence of SEQ ID NO: 15; HCDR2 consisting of the sequence of SEQ ID NO: 16; HCDR3 consisting of the sequence of SEQ ID NO: 17; and/or
the VL comprises: LCDR1 consisting of the sequence of SEQ ID NO: 21; LCDR2 consisting of a sequence of AAS; LCDR3 consisting of the sequence of SEQ ID NO: 20;
optionally,, the antibody or antigen binding fragment thereof comprises the following heavy chain variable region VH and/or a light chain variable region VL, wherein at least one CDR of the heavy chain variable region VH and/or light chain variable region VL contains a mutation, as compared with the CDR(s) defined by the Kabat or IMGT numbering system, wherein said mutation(s) is one or several amino acid substitutions, deletions or additions or any combination thereof, such as 1, 2 or 3 amino acid substitutions, deletions or additions or any combination thereof; preferably, the substitution(s) are conservative substitution(s);
more preferably, the antibody or antigen binding fragment thereof binds to human Her3, monkey Her3, and/or rat Her3.

3. The antibody or antigen-binding fragment thereof of claim 1 or 2, wherein the antibody or antigen-binding fragment thereof comprises:
(a) the VH set forth in any one of SEQ ID NO:23, 25, or 27, and/or, the VL set forth in any one of SEQ ID NO: 24, 26, or 28;
(b) a VH having at least 70%, at least 80%, at least 85%, at least 90%, at least 91%, at least 92%, at least 93%, at least 94%, at least 95%, at least 96%, at least 97%, at least 98%, or at least 99% sequence identity as compared with any one of the VH in (a); and/or, a VL having at least 70%, at least 80%, at least 85%, at least 90%, at least 91%, at least 92%, at least 93%, at least 94%, at least 95%, at least 96%, at least 97%, at least 98%, or at least 99% sequence identity compared with any one of the VL in (a); or
(c) a VH having one or several amino acid substitutions, deletions or additions or any combination thereof, such as 1, 2, 3, 4, 5, 6, 7, 8, 9 or 10 amino acid substitutions, deletions or additions or any combination thereof, compared with any one of the VH in (a); and/or, a VL having one or several amino acid substitutions, deletions or additions or any combination thereof, such as 1, 2, 3, 4, 5, 6, 7, 8, 9 or 10 amino acid substitutions, deletions or additions or any combination thereof, compared with any one of the VL in (a); preferably, the substitution(s) are conservative substitution(s).

4. The antibody or antigen-binding fragment thereof of any one of claims 1 to 3, wherein the antibody or antigen-binding fragment thereof comprises:
(a) the VH consisting of the sequence as set forth in SEQ ID NO: 23 and the VL consisting of the sequence as set forth in SEQ ID NO: 24;
(b) the VH consisting of the sequence as set forth in SEQ ID NO: 25 and the VL consisting of the sequence as set forth in SEQ ID NO: 26;
(c) the VH consisting of the sequence as set forth in SEQ ID NO: 27 and the VL consisting of the sequence as set forth in SEQ ID NO: 28;
(d) VH and VL, wherein the VH has at least 70%, at least 80%, at least 85%, at least 90%, at least 91%, at least 92%, at least 93%, at least 94%, at least 95%, at least 96%, at least 97%, at least 98% or at least 99% sequence identity; and/or, the VL has at least 70%, at least 80%, at least 85%, at least 90%, at least 91%, at least 92%, at least 93%, at least 94%, at least 95%, at least 96%, at least 97%, at least 98% or at least 99% sequence identity, as compared with the VH and VL in any one set of (a) to (c); or
(e) VH and VL, wherein the VH has one or several amino acid substitutions, deletions or additions or any combination thereof, such as 1, 2, 3, 4, 5, 6, 7, 8, 9 or 10 amino acid substitutions, deletions or additions or any combination thereof, and/or, the VL has one or several amino acid substitutions, deletions or additions or any combination thereof, such as 1, 2, 3, 4, 5, 6, 7, 8, 9 or 10 amino acid substitutions, deletions or additions or any combination thereof, as compared with the VH and VL in any one set of (a) to (c), preferably, the substitution(s) are conservative substitution(s).

5. An anti-Her3 antibody or antigen-binding fragment thereof that competes with the antibody or antigen-binding fragment thereof of any one of claims 1 to 4 for binding, and is different from the antibody or antigen-binding fragment thereof of any one of claims 1 to 4; preferably, the anti-Her3 antibody or antigen-binding fragment thereof competes with antibody 202-2-1 for binding, **characterized in that** it is not an antibody 202-2-1 or antigen-binding fragment thereof, and has different CDR(s) from antibody 202-2-1 or antigen-binding fragment thereof;
optionally, the anti-Her3 antibody or antigen-binding fragment thereof that binds to domain 3 of human Her3 competitively with antibody 202-2-1, and preferably, binds to amino acids from position 328 to position 499 of human Her3 as set forth in SEQ ID No.31; preferably, the anti-Her3 antibody or antigen-binding fragment thereof that binds to the following spatial epitopes competitively with antibody 202-2-1: Histidine at position 466, Tryptophan at position 470, Threonine at position 471, Threonine at position 478, (Aspartic acid-Isoleucine-lysine-Histidine-Asparagine) at positions 483 to 487, Isoleucine at position 484, Arginine at position 490 and Arginine at position 491 of human Her3 set forth in SEQ ID No. 31;
preferably, the anti-Her3 antibody or antigen-binding fragment thereof blocks Her3 ligand-dependent and Her3 ligand-independent signaling;
preferably, the anti-Her3 antibody or antigen-binding fragment thereof inhibits Her3 ligand-dependent and Her3 ligand-independent AKT phosphorylation.

6. The antibody or antigen-binding fragment thereof of any one of claims 1 to 5, wherein the antibody or antigen-binding fragment thereof is a chimeric antibody, a humanized antibody, or a fully human antibody;
optionally, the antibody or antigen-binding fragment thereof is selected from Fab, Fab', (Fab')₂, Fv fragment such as scFv or disulfide-linked Fv (dsFv), diabody, and multi-specific antibody.

7. The antibody or antigen-binding fragment thereof of any one of claims 1 to 6, wherein the antibody or antigen-binding fragment thereof further comprises:
(a) a heavy chain constant region CH of a human immunoglobulin or a variant thereof, and/or
(b) a light chain constant region CL of a human immunoglobulin or a variant thereof,
wherein, the variant has at least 70%, at least 80%, at least 85%, at least 90%, at least 91%, at least 92%, at least 93%, at least 94%, at least 95%, at least 96%, at least 97%, at least 98% or at least 99% sequence identity compared with the wild type sequence from which it is derived; or the variant has one or more amino acid substitutions, deletions or additions or any combination thereof, such as up to 50, up to 45, up to 40, up to 35, up to 30, up to 25, up to 20, up to 15, up to 10, or up to 5 amino acid substitutions, deletions or additions or any combination thereof; such as 1, 2, 3, 4, 5, 6, 7, 8, 9, or 10 amino acid substitutions, deletions or additions or any combination thereof, compared with the wild type sequence from which it is derived; preferably, the substitution(s) are conservative substitution(s);
preferably, the heavy chain constant region is an IgG heavy chain constant region, e.g., an IgG1, IgG2, IgG3, or IgG4 heavy chain constant region; and/or, the light chain constant region is a κ or λ light chain constant region;
more preferably, the antibody or antigen-binding fragment thereof comprises a human IgG1 heavy chain constant region; and/or the antibody or antigen-binding fragment thereof comprises a human κ light chain constant region.

8. The antibody or antigen-binding fragment thereof of claim 7, wherein,
the heavy chain constant region comprises a CH as set forth in SEQ ID NO: 29 or a variant thereof, said variant has conservative substitutions of up to 20 amino acids, such as conservative substitutions of up to 20, up to 15, up to 10 or up to 5 amino acids, such as conservative substitutions of 1, 2, 3, 4, 5, 6, 7, 8, 9 or 10 amino acids compared with SEQ ID NO: 29; or has at least 70%, at least 80%, at least 85%, at least 90%, at least 91%, at least 92%, at least 93%, at least 94%, at least 95%, at least 96%, at least 97%, at least 98%, or at least 99% sequence identity compared with SEQ ID NO: 29; and/or
the light chain constant region comprises a CL as set forth in SEQ ID NO:30 or a variant thereof, said variant has conservative substitutions of up to 20 amino acids, such as conservative substitutions of up to 20, up to 15, up to 10 or up to 5 amino acids, such as conservative substitutions of 1, 2, 3, 4, 5, 6, 7, 8, 9 or 10 amino acids compared with SEQ ID NO: 30; or has at least 70%, at least 80%, at least 85%, at least 90%, at least 91%, at least 92%, at least 93%, at least 94%, at least 95%, at least 96%, at least 97%, at least 98%, or at least 99% sequence identity compared with SEQ ID NO: 30;
preferably, the antibody or antigen-binding fragment thereof comprises the heavy chain constant region CH as set forth in SEQ ID NO: 29 and the light chain constant region CL as set forth in SEQ ID NO: 30.

9. The antibody or antigen-binding fragment thereof of any one of claims 1 to 8, wherein the antibody is selected from the following groups comprising
(a) a heavy chain comprising the VH as set forth in SEQ ID NO: 23 and the CH as set forth in SEQ ID NO: 29, and a light chain comprising the VL as set forth in SEQ ID NO: 24 and the CL as set forth in SEQ ID NO: 30; preferably the heavy chain as set forth in SEQ ID NO: 11 and the light chain as set forth in SEQ ID NO: 22;
(b) a heavy chain comprising the VH as set forth in SEQ ID NO: 25 and the CH as set forth in SEQ ID NO: 29, and a light chain comprising the VL as set forth in SEQ ID NO: 26 and the CL as set forth in SEQ ID NO: 30; or
(c) a heavy chain comprising the VH as set forth in SEQ ID NO: 27 and the CH as set forth in SEQ ID NO: 29, and a light chain comprising the VL as set forth in SEQ ID NO: 28 and the CL as set forth in SEQ ID NO: 30.

10. An antibody of any one of claims 1 to 9, wherein the antibody is a multi-specific antibody;
preferably, the multi-specific antibody is a bispecific antibody or a tri-specific antibody or a tetra-specific antibody.

11. An isolated nucleic acid molecule, encoding the antibody or antigen-binding fragment thereof of any one of claims 1 to 10.

12. A vector, comprising the isolated nucleic acid molecule of claim 11; preferably, the vector is a cloning vector or an expression vector.

13. A host cell, comprising the isolated nucleic acid molecule of claim 11 or the vector of claim 12.

14. A method of preparing the antibody or antigen-binding fragment thereof of any one of claims 1 to 10, comprising culturing the host cell of claim 13 under conditions that allow expression of the antibody or antigen-binding fragment thereof, and recovering the antibody or antigen-binding fragment thereof from the cultured host cell culture.

15. An antibody-drug conjugate, a stereoisomer thereof, a prodrug thereof, a pharmaceutically acceptable salt thereof, or a pharmaceutically acceptable solvate thereof, wherein the antibody is the antibody or antigen-binding fragment thereof of any one of claims 1 to 10, which is linked to a coupling moiety via a connector;
the coupling moiety can be selected from a detectable marker, a radioisotope, a fluorescent agent, a luminescent agent, a colored agent, an enzyme, polyethylene glycol, a nuclide, a nucleic acid, a small molecule toxin, a polypeptide having binding activity, a protein, a receptor, a ligand, and other active agent that inhibits tumor cell growth, promotes tumor cell apoptosis or necrosis.

16. An antibody-drug conjugate of claim 15, a stereoisomer thereof, a prodrug thereof, a pharmaceutically acceptable salt thereof, or a pharmaceutically acceptable solvate thereof, **characterized in that**,
the antibody-drug conjugate is shown by the following formula:
Th-(L-D)_{q},
wherein:
Tb is the antibody or antigen-binding fragment thereof of any one of claims 1 to 10, and;
D is a small molecule toxin drug moiety;
L is a bond or linker which covalently links Tb and D;
q is an integer between 1 and 16, and represents the number of L-D covalently linked to Tb.

17. The antibody-drug conjugate of claim 16, a stereoisomer thereof, a prodrug thereof, a pharmaceutically acceptable salt thereof, or a pharmaceutically acceptable solvate thereof, **characterized in that**,
the antibody-drug conjugate has the structure represented by formula I: 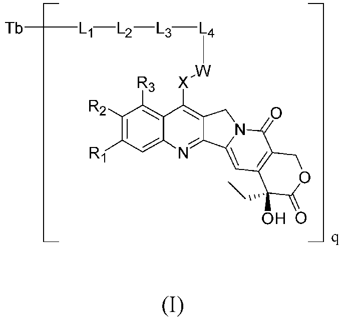 wherein,
L₁ is selected from 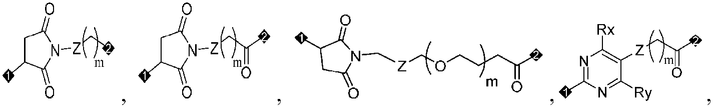 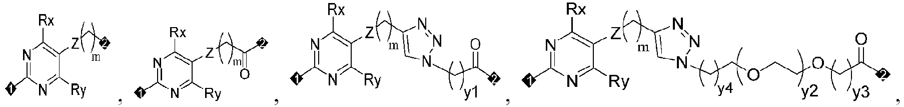 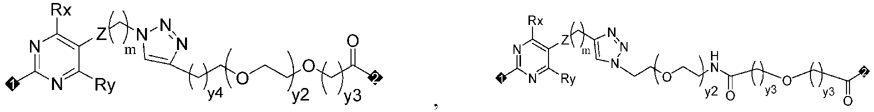 and 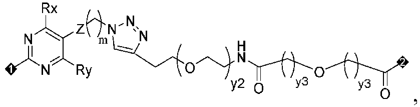 each Z is independently selected from a direct bond, a carbon-carbon triple bond, a carbon-carbon double bond, and amido; Rx and Ry are each independently selected from Hand C1-4 allcyl; each m is independently selected from 0, 1, 2, 3, 4, 5 and 6; y1 is selected from any integer between 1 and 6; each y2 is independently selected from any integer between 0 and 15; each y3 is independently selected from 1, 2, and 3; each y4 is independently selected from 0 and 1; position 1 is attached to Tb via an S atom, and position 2 is attached to L₂ or L₃;
L₂ is absent or present, and when L₂ is present, L₂ is selected from: 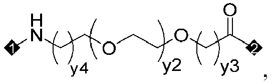 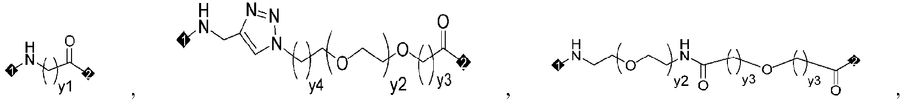 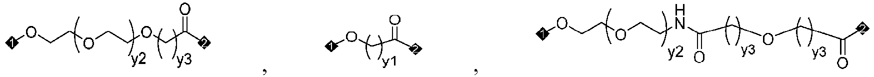 and 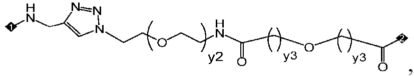 y1 is selected from any integer between 1 and 6; each y2 is independently selected from any integer between 0 and 10; each y3 is independently selected from 1 or 2; each y4 is independently selected from 0 or 1, position 1 is attached to L₁, and position 2 is attached to L3;
L₃ is selected from an amino acid residue or a short peptide consisting of 2-10 amino acid residues; the amino acid residue is selected from natural amino acid residues, non-natural amino acid residues, or selected from amino acid residue represented by AA¹ or stereoisomer thereof; 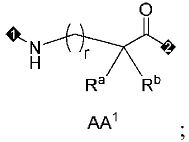 in the amino acid residue represented by AA¹, any one of R^{a} and R^{b} is H, and the other is H, 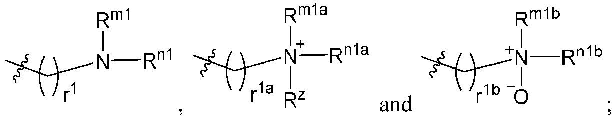 or, R^{a} and R^{b}, together with the carbon atom to which they are both attached, form a 5-6 membered heterocyclic ring, and said 5-6 membered heterocyclic ring is piperidine ring or piperazine ring,
r, r¹, r^{1a} and r^{1b} are each independently selected from 0, 1, 2, 3, 4 or 5;
R^{m1}, Rⁿ¹, R^{m1a}, R^{n1a}, R^{m1b} and R^{n1b} are each independently H, C₁₋₆ alkyl or -COOR^{x1}, wherein, R^{x1} is C₁₋₆ alkyl;
or, R^{m1} and Rⁿ¹, R^{m1a} and R^{n1a}, and R^{m1b} and R^{n1b}, together with the nitrogen atom to which they are both attached, form a 5-6 membered heterocyclic ring, and of said 5-6 membered heterocyclic ring, the heteroatom is selected from 1 or 2 N atoms; said 5-6 membered heterocyclic ring is optionally substituted with one or more R^{0'};
R^{z} is selected from C₁₋₆ alkyl;
R⁰ and R^{0'} are each independently selected from C₁₋₆ alkyl, -NR^{m2}Rⁿ² or 5-6 membered heterocyclyl optionally substituted with C₁₋₆ alkyl; of said 5-6 membered heterocyclyl, the heteroatom is selected from 1 or 2 N atoms;
R^{m2} and Rⁿ² are each independently selected from H and C₁₋₆ alkyl;
L₄ is absent or present, when L₄ is present, L₄ is selected from 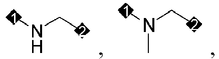 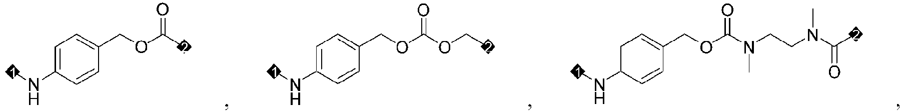 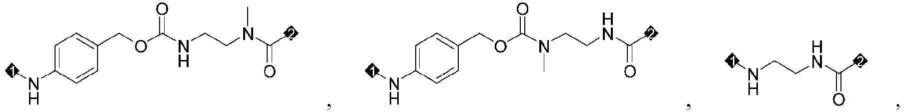 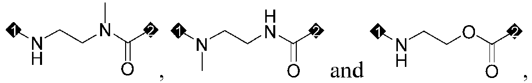 position 1 is attached to L₃, and position 2 is attached to D;
R₁ and R₂ are each independently selected from H, halogens and C1-4 alkyl; or, R₁ and R₂, together with the carbon atom to which they are both attached form a 5-6 membered heterocyclic ring, the heterocyclic ring contains 1, 2, or 3 O, S, or N or any combination thereof;
R₃ is selected from H and C₁₋₄ alkyl; or R₃ and X, together with the carbon atom to which they are both attached, form a 5-6 membered carbon ring;
W is absent or present, when W is present, W is selected from -O-, -S-, -NR₄-, 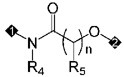 and 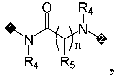 position 1 is attached to X, and position 2 is attached to L₄ or L₃;
X is selected from optionally substituted -(CH₂)ₙ₁-, 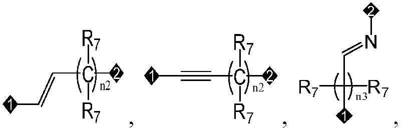 position 1 is attached to the parent ring and position 2 is attached to W or L₄; the substituent is selected from one or two C1-4 alkyls;
R₄, R₅, and R₇ are each independently selected from H and C1-4 alkyl;
n, n1, n2, n3 are each independently selected from any integer between 0 and 6.

18. The antibody-drug conjugate of claim 17, a stereoisomer thereof, a prodrug thereof, a pharmaceutically acceptable salt thereof, or a pharmaceutically acceptable solvate thereof, **characterized in that** one or more of the following conditions are met:
(1) L₁ is selected from 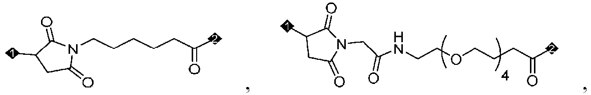 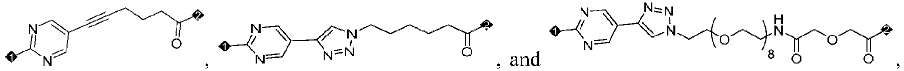 position 1 is attached to Tb via an S atom, and position 2 is attached to L₂ or L₃.
(2) L₂ is absent or present, and when L₂ is present, L₂ is selected from 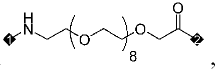 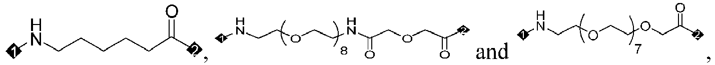 position 1 is attached to L₁, and position 2 is attached to L₃;
(3) L₃ is selected from AA¹, AA¹-Gly, Val-Cit, Val-AA¹-Gly, AA¹-Ala-Asn, and Gly-Gly-Phe-Gly;
(4) the amino acid residue represented by AA¹ is selected from 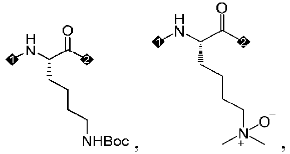 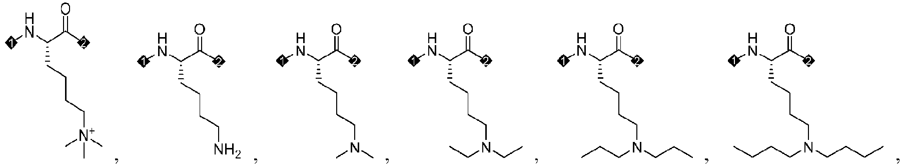 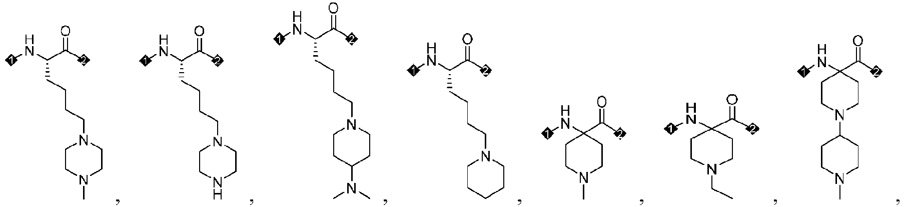 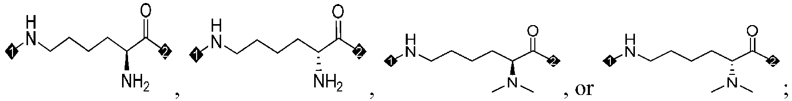
(5) L₄ is absent or present, when L₄ is present, L₄ is selected from 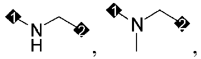 and 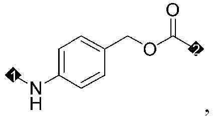 position 1 is attached to L₃, and position 2 is attached to D;
(6) R₁ is selected from H and halogen, R₂ is selected from H and C1-4 alkyl; or, R₁ and R₂, together with the carbon atoms to which they are attached, form 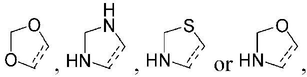 the dotted line indicates where the heterocyclic ring is fused to the benzene ring;
(7) R₃ is H; or, R₃ and X together with the carbon atoms to which they are attached form 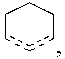 the dotted line indicates where the carbocyclic ring is fused to the benzene ring and pyridine ring;
(8) each R₄ is independently selected from H, methyl, ethyl, n-propyl, isopropyl, and t-butyl, and R₅ is H;
(9) each R₇ is independently selected from H and C1-4 alkyl;
(10) n is selected from 1, 2 and 3;
(11) n1 is selected from 1, 2, 3 and 4;
(12) n2 is 1;
(13) n3 is 0;
(14) W is selected from -O-, -NR₄- and 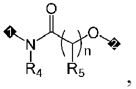 position 1 is attached to X and position 2 is attached to L₄ or L₃;
(15) X is selected from optionally substituted 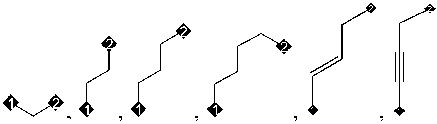 and 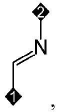 position 1 is attached to the parent ring and position 2 is attached to W or L₄; the substituent is selected from 1 or 2 C1-4 alkyl groups, or 2 C1-4 alkyl groups.

19. The antibody-drug conjugate of claim 18, a stereoisomer thereof, a prodrug thereof, a pharmaceutically acceptable salt thereof, or a pharmaceutically acceptable solvate thereof, **characterized in that** one or more of the following conditions are met:
(1) L₁ is selected from 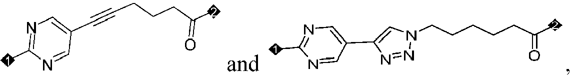 position 1 is attached to Tb via an S atom, and position 2 is attached to L2 or L3;
(2) L₂ is absent;
(3) L₃ is selected from 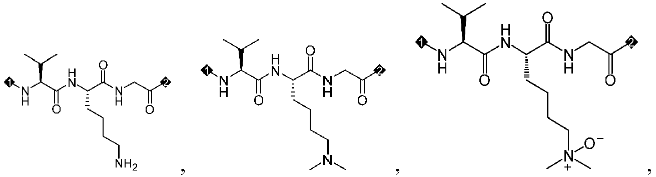 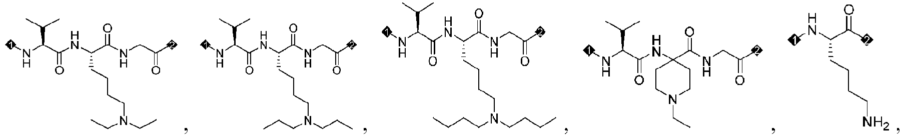 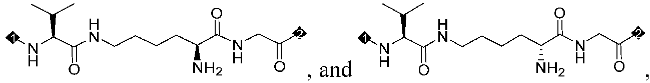 position 1 is attached to L₁ or L₂, and position 2 is attached to L₄ or D;
(4) L₄ is selected from 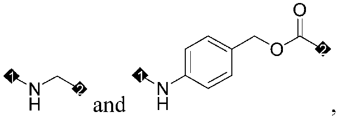 position 1 is attached to L₃, and position 2 is attached to D;
(5) R₁ is H or F, R₂ is H or methyl; or R₁ and R₂ together with the carbon atoms to which they are attached form 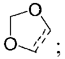
(6) R₃ is H;
(7)W is selected from -O-, -NR₄-, and 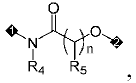 position 1 is attached to X, and position 2 is attached to L₄ or L₃;
(8) X is selected from 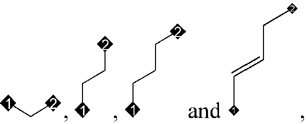 position 1 is attached to the parent ring, and position 2 is attached to W.

20. The antibody-drug conjugate of claim 17 or 18, a stereoisomer thereof, a prodrug thereof, a pharmaceutically acceptable salt thereof, or a pharmaceutically acceptable solvate thereof, **characterized in that** one or more of the following conditions are met:
(1) the structure 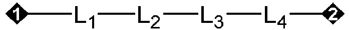 is selected from the following structural fragments: 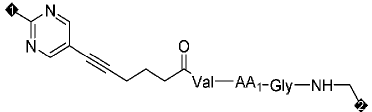 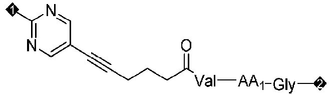 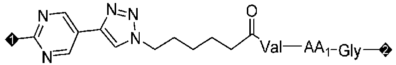 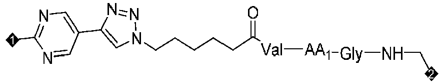
wherein, position 1 is attached to Tb, and position 2 is attached to D;
for example, the structure 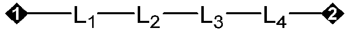 is selected from following structural fragments: 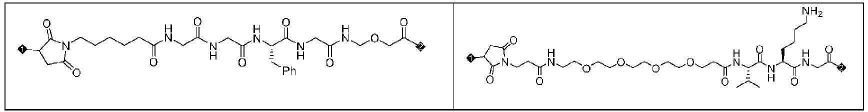 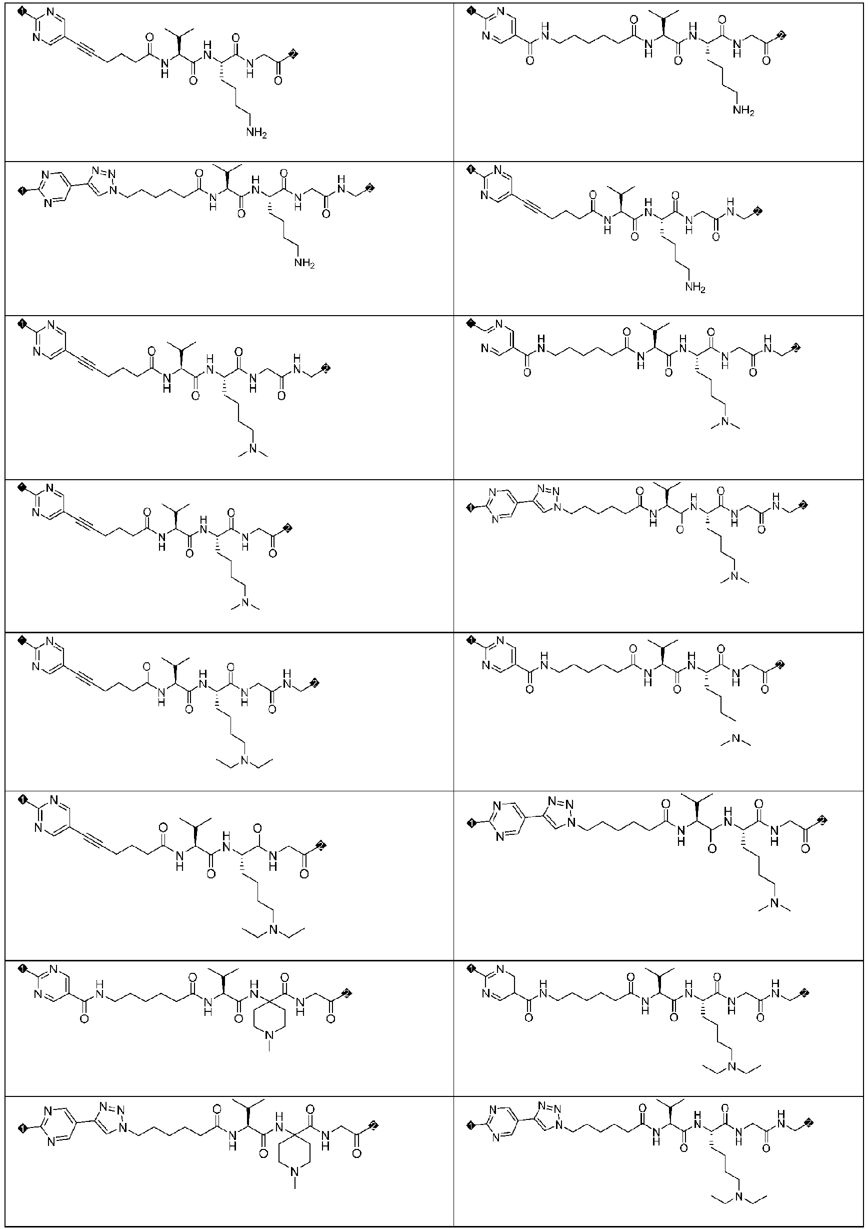 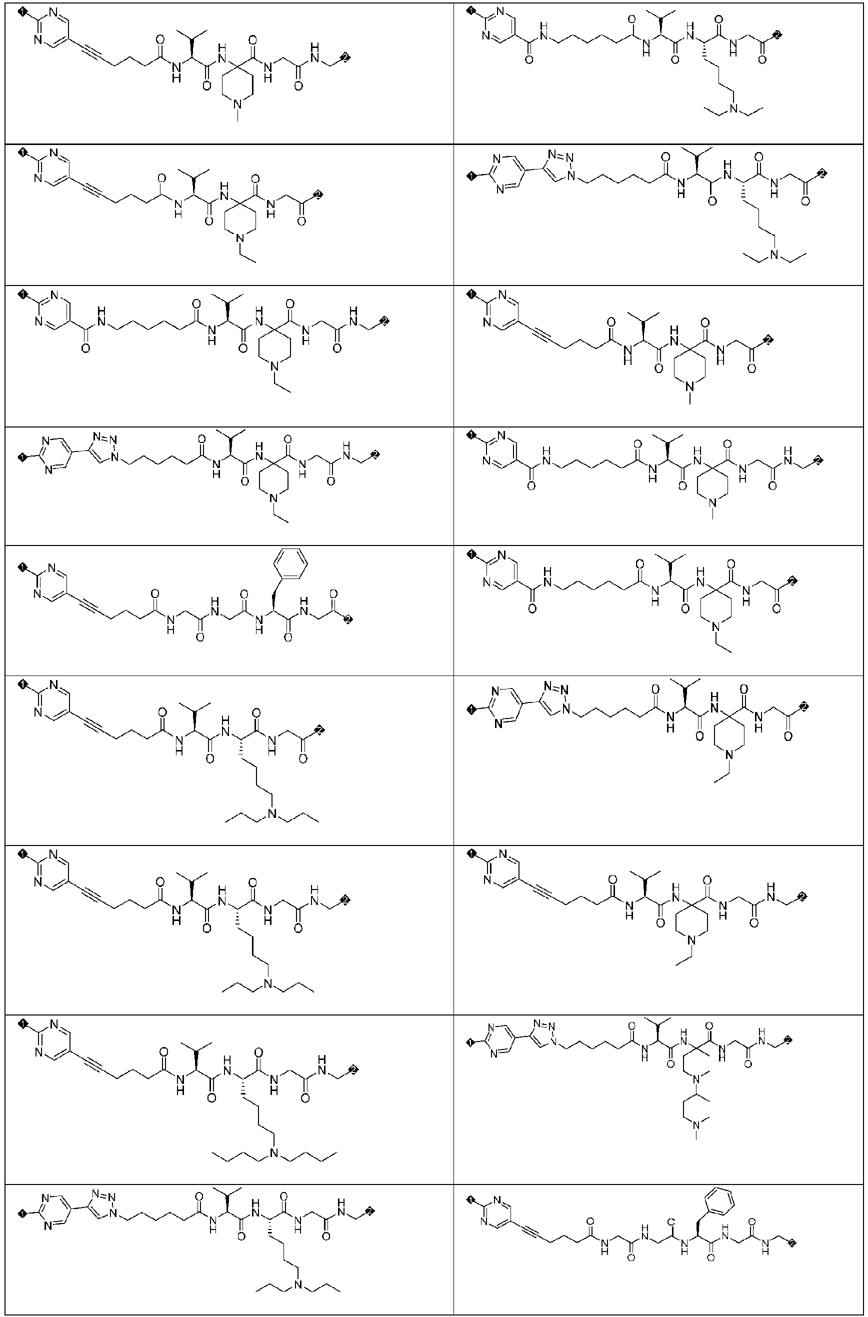 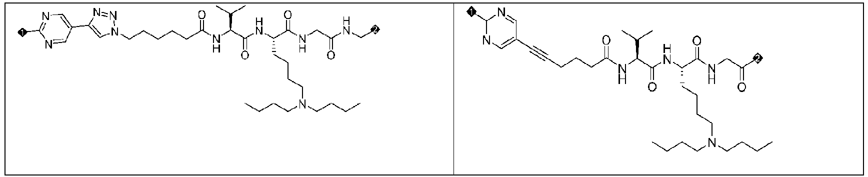
wherein, position 1 is attached to Tb, and position 2 is attached to W;
(2) the structural fragments represented by 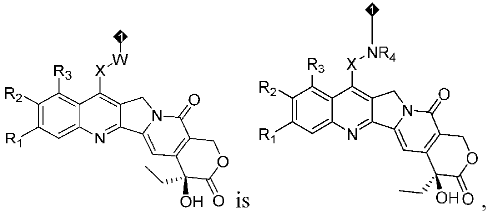 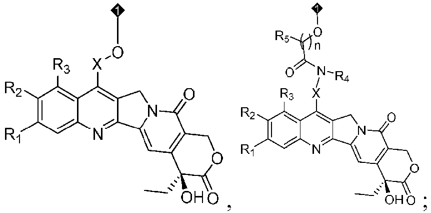 position 1 is attached to L₄;
for example, the structure 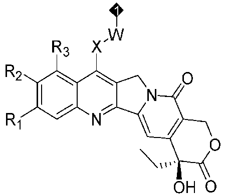 is selected from the following structural fragments: 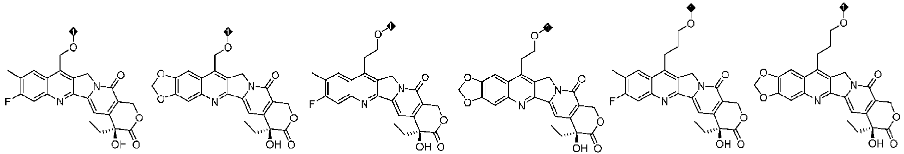 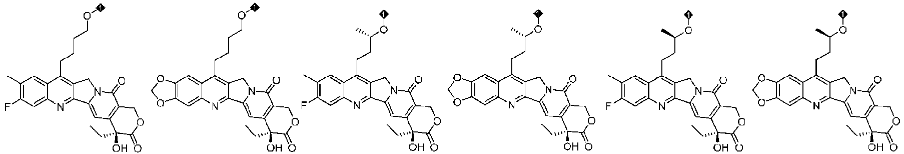 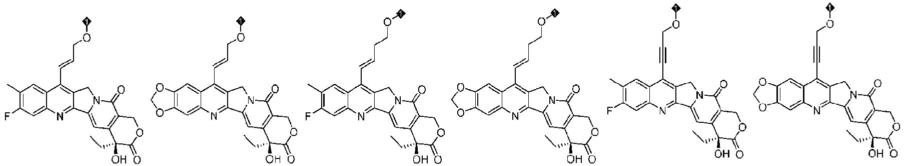 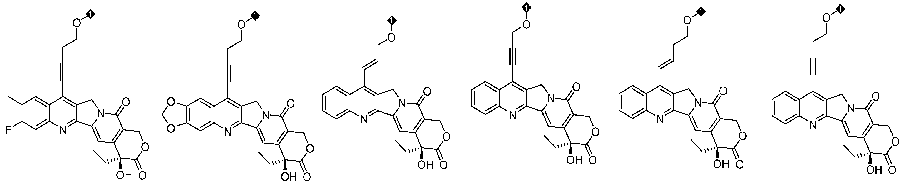 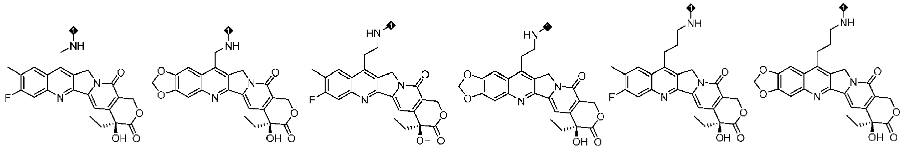 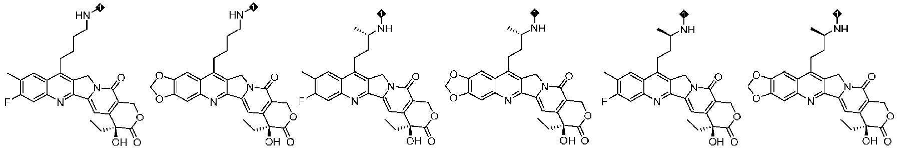 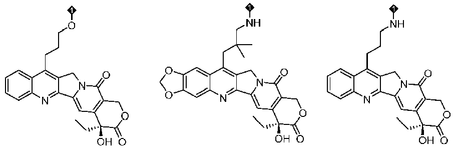 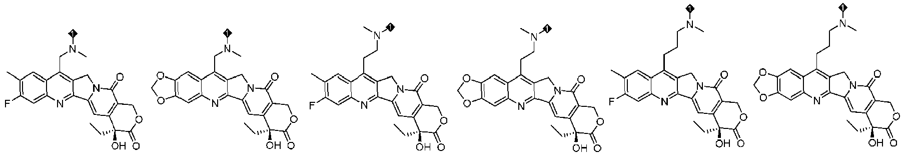 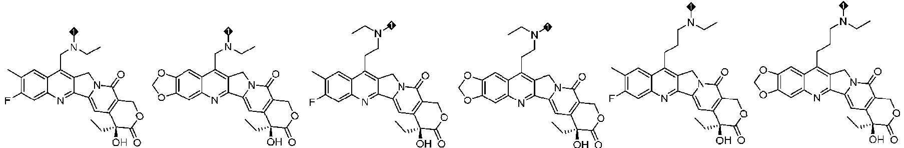 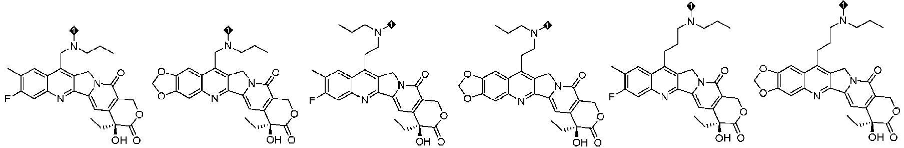 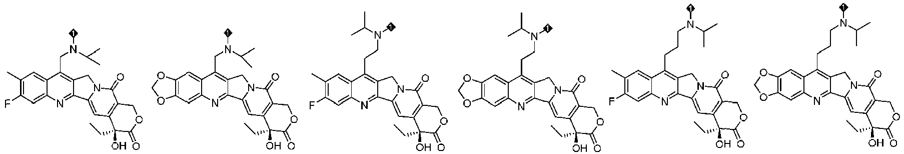 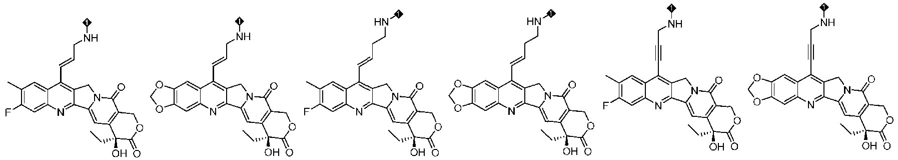 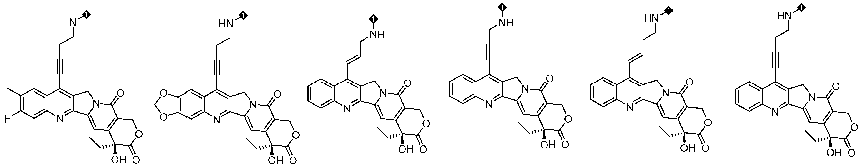 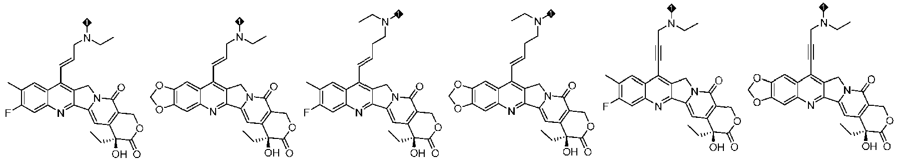 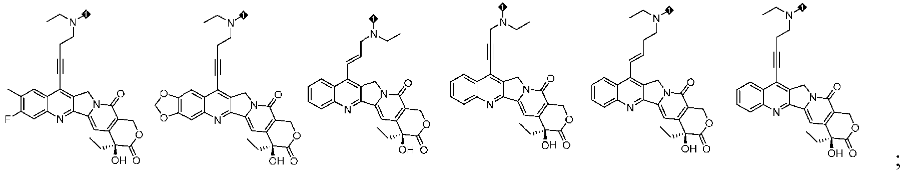
(3) the structure 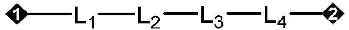 is selected from the following structural fragments: 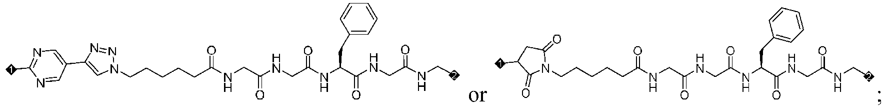 wherein, position 1 is attached to Tb via an S atom, position 2 is attached to W;
(4). the structure 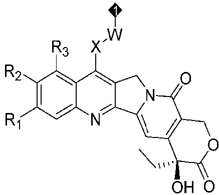 is selected from the following structural fragments: 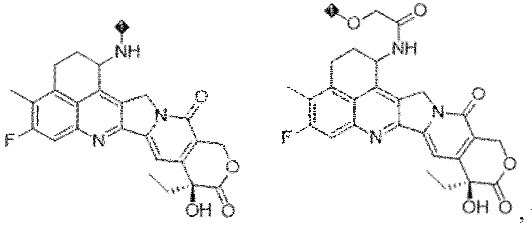 wherein, position 1 is attached to L4.

21. The antibody-drug conjugate of claim 17 or 18, a stereoisomer thereof, a prodrug thereof, a pharmaceutically acceptable salt thereof, or a pharmaceutically acceptable solvate thereof, **characterized in that** one or more of the following conditions are met:
(1) the antibody-drug conjugate has the structure represented by formula I-1, I-2, I-3: 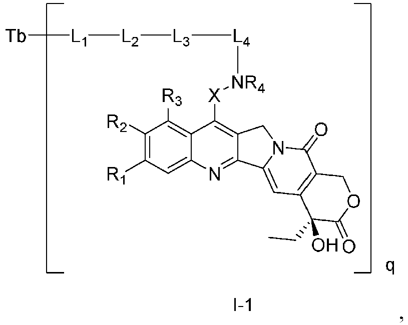 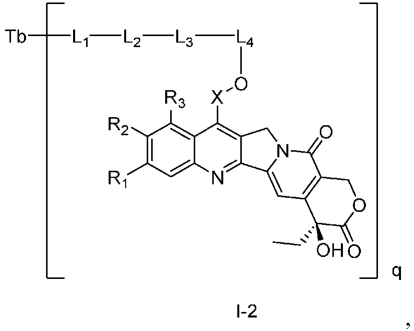 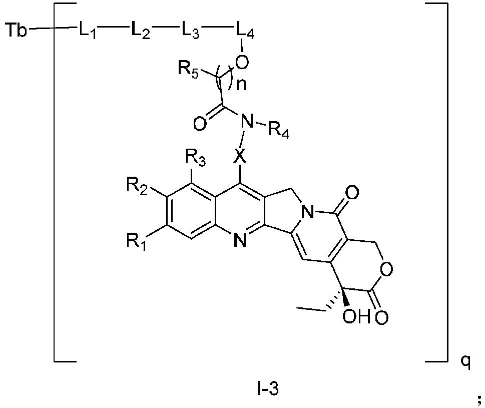
(2) the antibody-drug conjugate has the structure represented by formula I-1A, I-1B, I-2A, 1-2B, I-3A or I-3B: 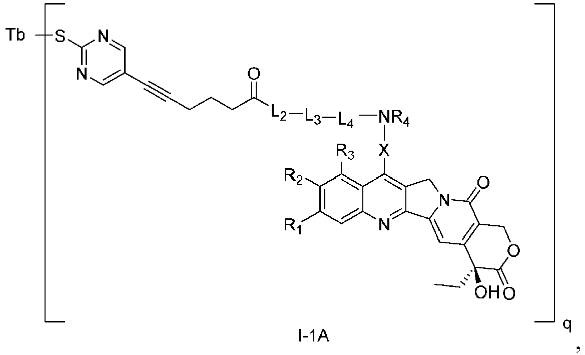 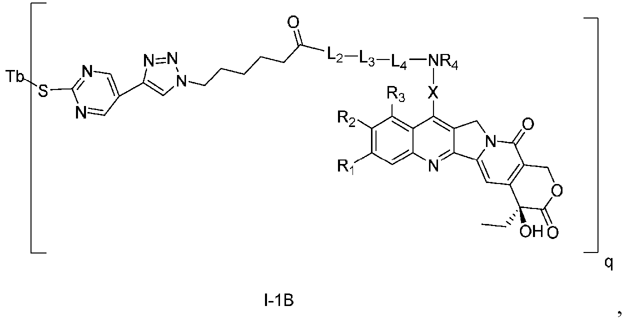 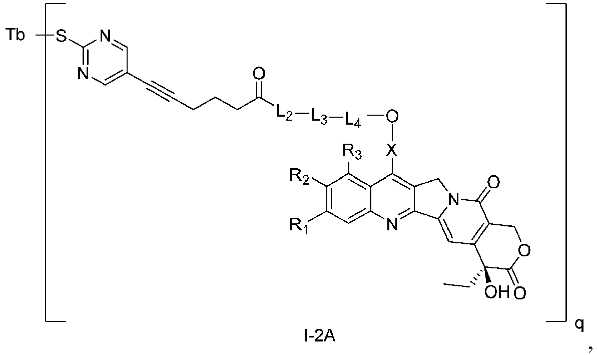 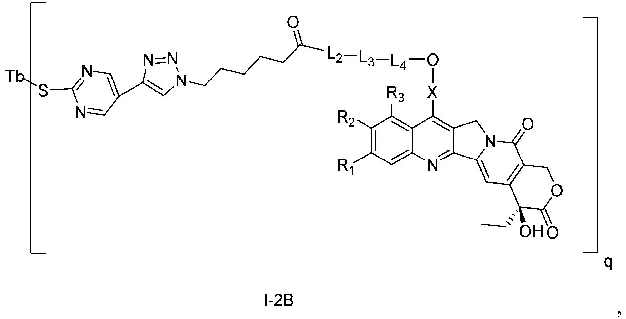 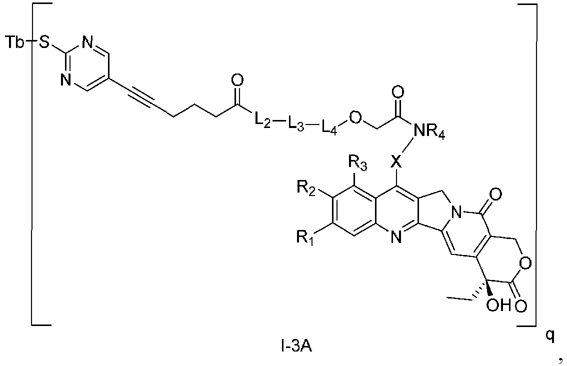 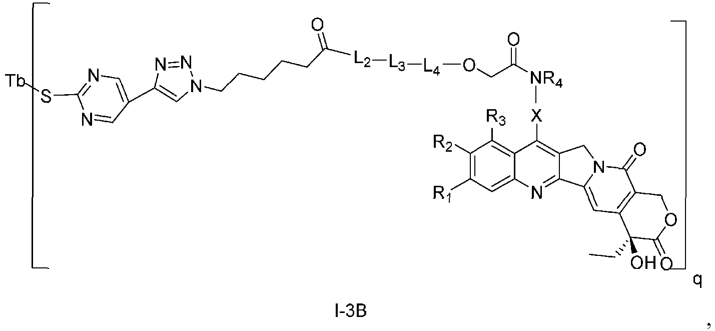
(3) the antibody-drug conjugate has the structure represented by formula I-A, I-B: 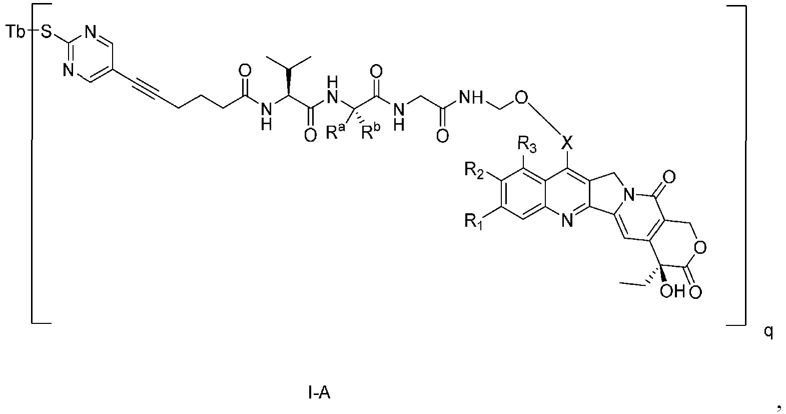 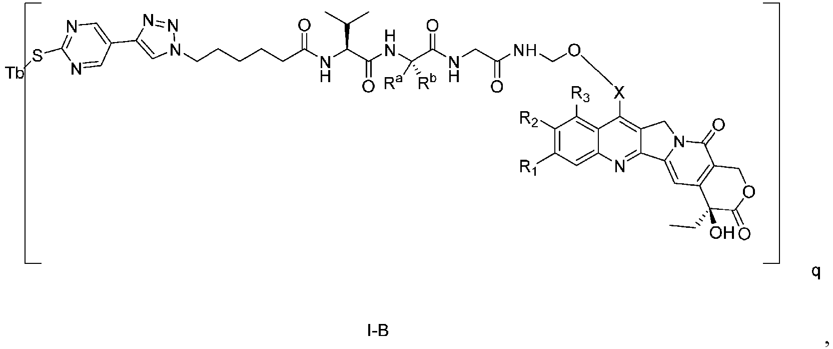 wherein Tb, L₁, L₂, L₃, L₄, X, R₁, R₂, R₃, R₄, R₅, R^{a}, R^{b}, n, and q are as defined in any one of claims 16 to 20.

22. The antibody-drug conjugate of any one of claims 15 to 21, a stereoisomer thereof, a prodrug thereof, a pharmaceutically acceptable salt thereof, or a pharmaceutically acceptable solvate thereof, **characterized in that** the antibody-drug conjugate is selected from:
| the structure of the antibody-drug conjugate |
|---|
| 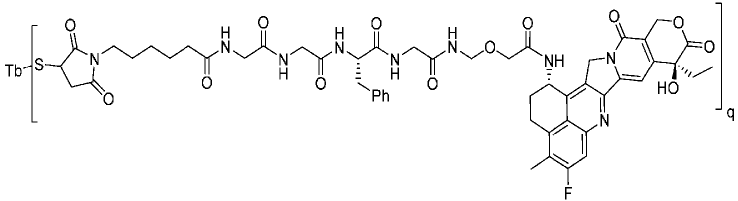 |
| 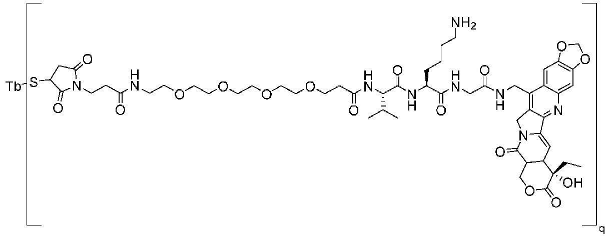 |
| 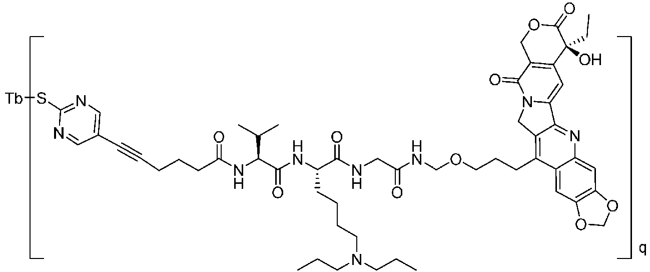 |
| 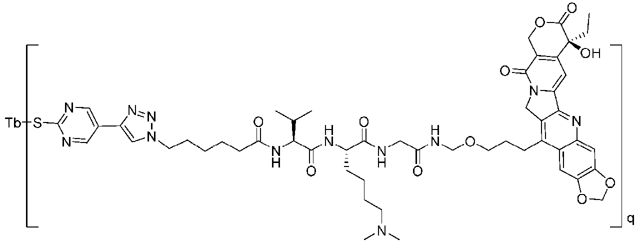 |
| 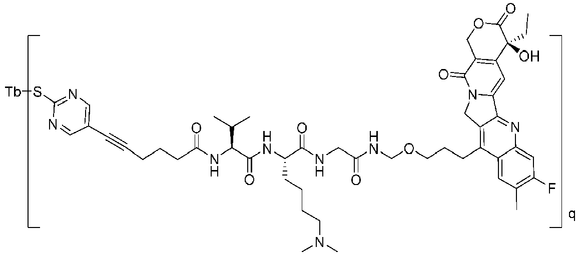 |
| 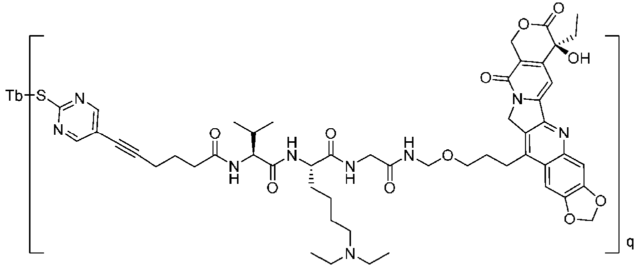 |
| 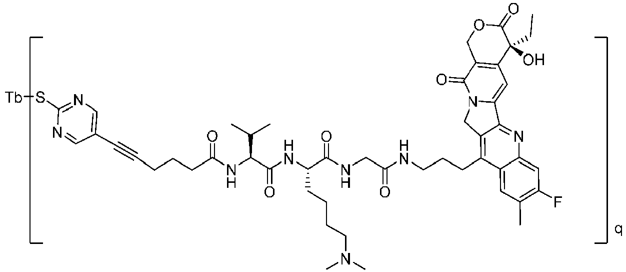 |
| 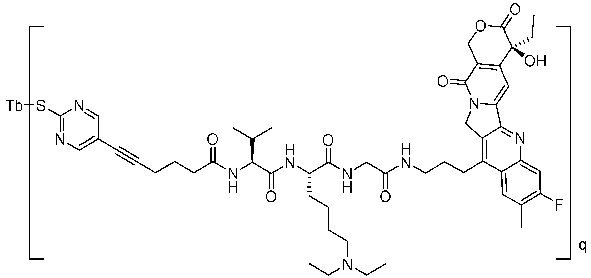 |
| 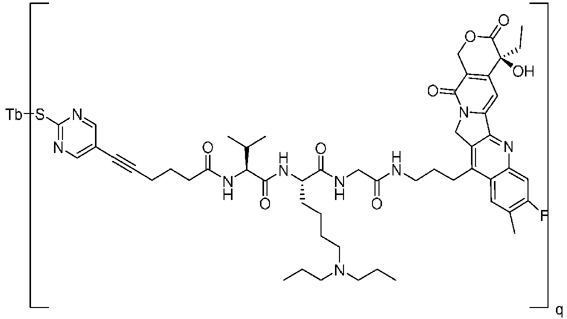 |
| 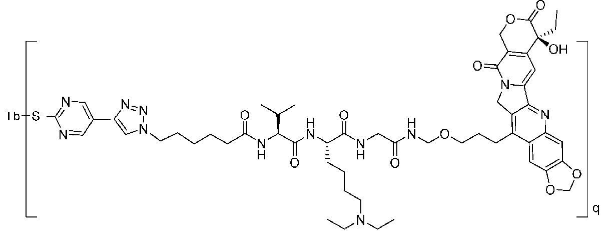 |
| 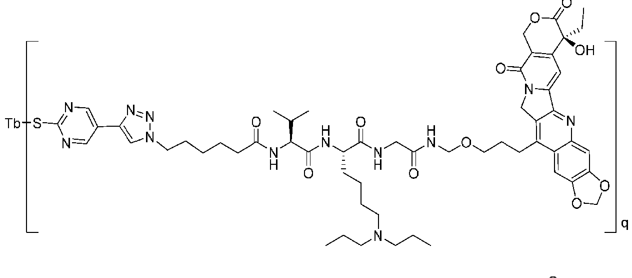 |
| 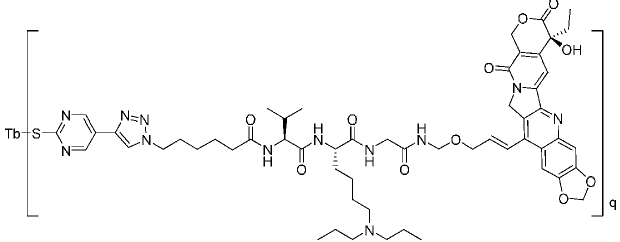 |
| 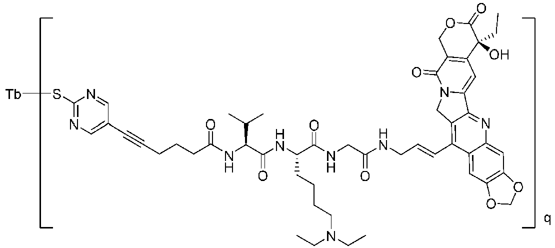 |
| 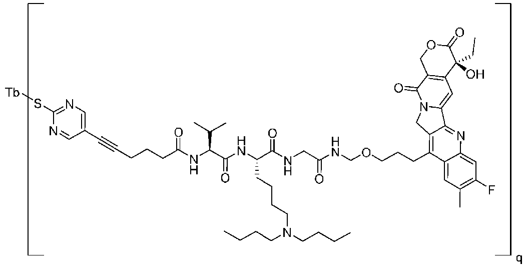 |
| 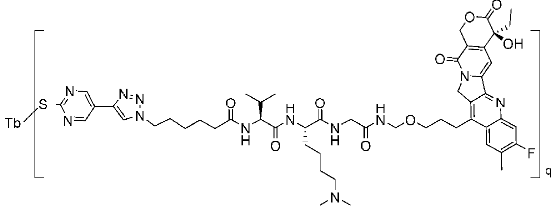 |
| 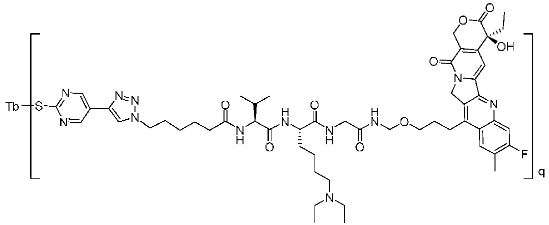 |
| 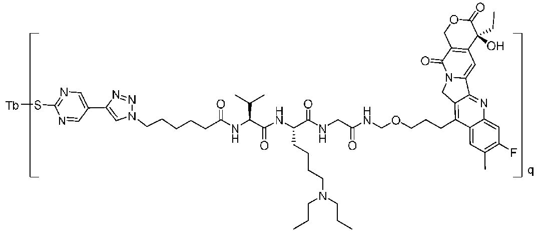 |
| 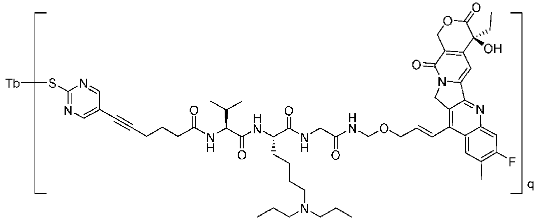 |
| 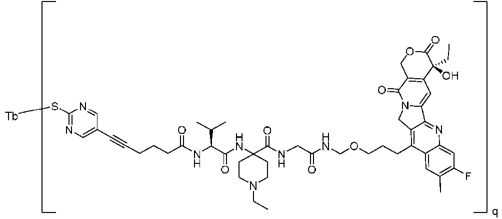 |
| 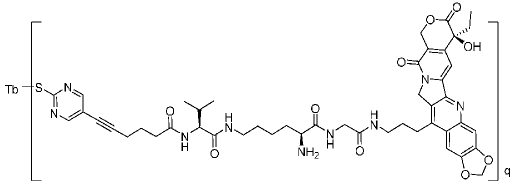 |
wherein,
Tb is the anti-Her3 antibody of any one of preceding claims;
q is 1, 2, 3, 4, 5, 6, 7, 8, 9, 10, 11, 12, 13, 14, 15, 16; and q is preferably 1, 2, 3, 4, 5, 6, 7, 8; for example, 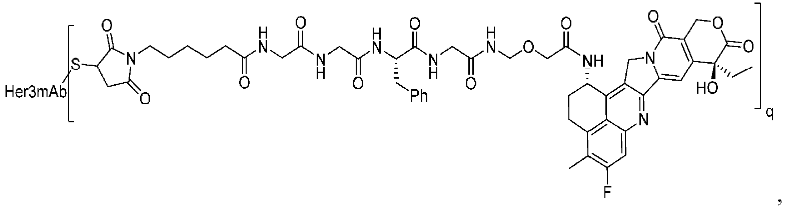 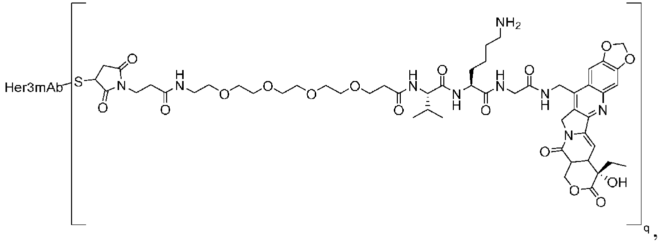 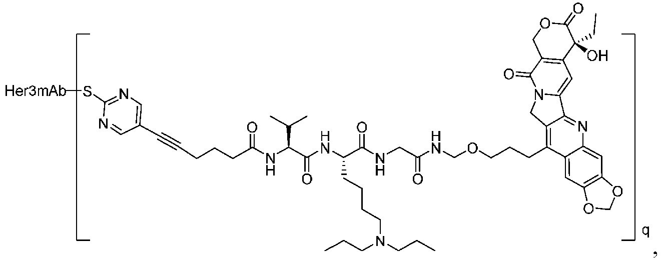 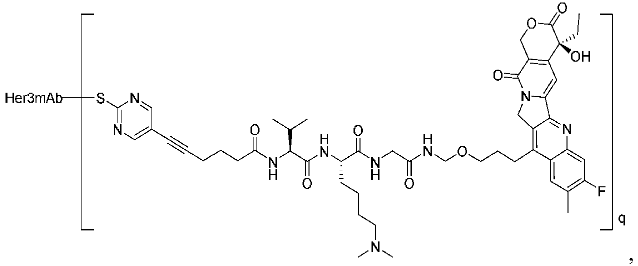 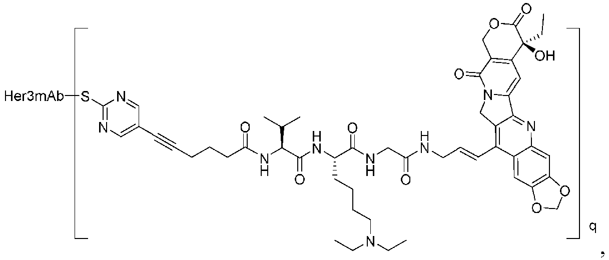 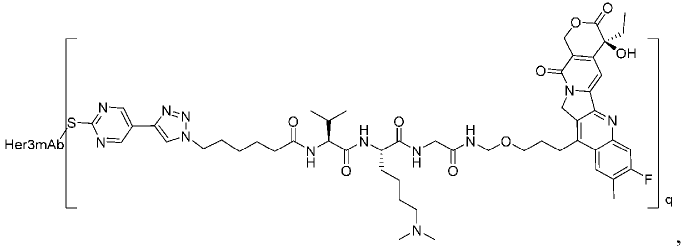
wherein, Her3mAb is antibody 202-2-1.

23. The antibody-drug conjugate of any one of claims 15 to 21, a stereoisomer thereof, a prodrug thereof, a pharmaceutically acceptable salt thereof, or a pharmaceutically acceptable solvate thereof, **characterized in that** the antibody-drug conjugate is selected from: 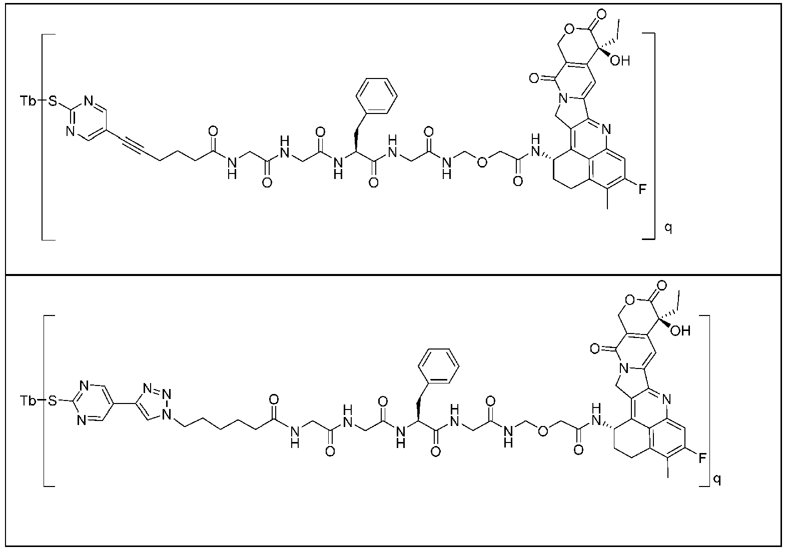 wherein, Tb is the anti-Her3 antibody of any one of preceding claims,
q is 1, 2, 3, 4, 5, 6, 7, 8, 9, 10, 11, 12, 13, 14, 15, 16; q is preferably 1, 2, 3, 4, 5, 6, 7, 8;
for example, 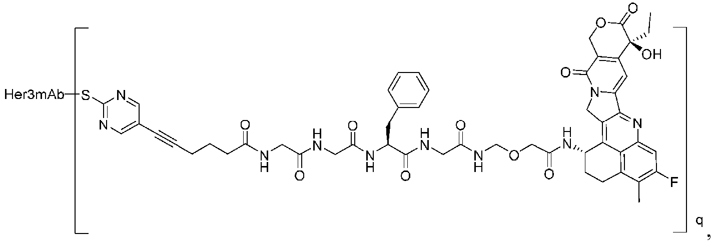 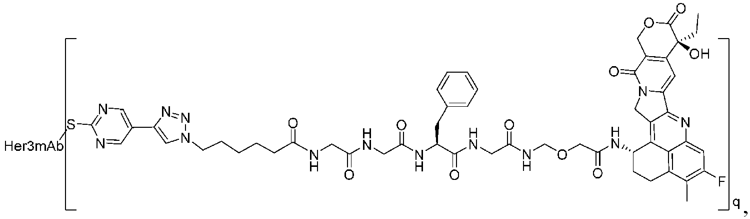 wherein, Her3mAb is antibody 202-2-1.

24. Antibody-drug conjugates, comprising two or more antibody-drug conjugates of any one of claims 16 to 23, stereoisomers thereof, prodrugs thereof, pharmaceutically acceptable salts thereof or pharmaceutically acceptable solvates thereof, and the antibody-drug conjugate in the antibody-drug conjugates has one, two or more q values.

25. The antibody-drug conjugates of claim 24, **characterized in that**,
the ratio of drug to antibody (DAR) in the antibody-drug conjugates is selected from an integer or decimal between 1 and 10.
for example, the DAR is selected from 2, 2.5, 3, 3.5, 4, 4.5, 5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9 and 9.

26. A pharmaceutical composition comprising the antibody or antigen-binding fragment thereof of any one of claims 1 to 10, the nucleic acid molecule of claim 11, the vector of claim 12, the host cell of claim 13, the antibody-drug conjugate of claims 15 to 23, a stereoisomer thereof, a prodrug thereof, a pharmaceutically acceptable salt thereof or a pharmaceutically acceptable solvate thereof, and/or the antibody-drug conjugates of claims 24 to 25, as well as a pharmaceutically acceptable carrier and/or excipient.

27. The use of the antibody or antigen-binding fragment thereof of any one of claims 1 to 10, the nucleic acid molecule of claim 11, the vector of claim 12, the host cell of claim 13, the antibody-drug conjugate of claims 15 to 23, a stereoisomer thereof, a prodrug thereof, a pharmaceutically acceptable salt thereof or a pharmaceutically acceptable solvate thereof, the antibody-drug conjugates of claims 24 to 25, and/or the pharmaceutical composition of claim 26 in the manufacture of a medicament for the prevention and/or treatment of tumors and/or infectious diseases;
optionally, the tumor is a solid tumor;
optionally,, the tumor is selected from esophageal cancer (e.g., esophageal adenocarcinoma and esophageal squamous cell carcinoma), brain tumor, lung cancer (e.g., small cell lung cancer and non-small cell lung cancer), squamous cell carcinoma, bladder cancer, stomach cancer, ovarian cancer, peritoneal cancer, pancreatic cancer, breast cancer, head and neck cancer, cervical cancer, endometrial cancer, colorectal cancer, liver cancer, kidney cancer, urothelial cancer, solpid tumor, non-Hodgkin lymphoma, central nervous system tumor (e.g., neuroglioma, glioblastoma multiforme, glioma or sarcoma), prostate cancer or thyroid cancer;
optionally, the tumor is selected from colorectal cancer (e.g., colon cancer), lung cancer (e.g., non-small cell lung cancer), breast cancer, and prostate cancer.

28. A method for preventing and/or treating tumors and/or infectious diseases in a subject, which comprises administering an effective amount of the antibody or antigen-binding fragment thereof of any one of claims 1 to 10, the nucleic acid molecule of claim 11, the vector of claim 12, the host cell of claim 13, the antibody-drug conjugate of claims 15 to 23, a stereoisomer thereof, a prodrug thereof, a pharmaceutically acceptable salt thereof or a pharmaceutically acceptable solvate thereof, the antibody-drug conjugates of claims 24 to 25 and/or the pharmaceutical composition of claim 26 to a subject in need thereof, wherein the subject is a mammal;
preferably, the subject is human.
